(19) Europäisches Patentamt
European Patent Office
Office européen des brevets

(11) Veröffentlichungsnummer: **0 373 549 B1**

(12) **EUROPÄISCHE PATENTSCHRIFT**

(45) Veröffentlichungstag der Patentschrift: **01.09.93**

(51) Int. Cl.[5]: **C07K 5/02**, C07K 5/06, A61K 37/64

(21) Anmeldenummer: **89122794.4**

(22) Anmeldetag: **11.12.89**

# (54) Enzym-hemmende Aminosäurederivate, Verfahren zu ihrer Herstellung, diese enthaltende Mittel und ihre Verwendung.

(30) Priorität: **14.12.88 DE 3842067**

(43) Veröffentlichungstag der Anmeldung:
**20.06.90 Patentblatt 90/25**

(45) Bekanntmachung des Hinweises auf die Patenterteilung:
**01.09.93 Patentblatt 93/35**

(84) Benannte Vertragsstaaten:
**AT BE CH DE ES FR GB GR IT LI LU NL SE**

(56) Entgegenhaltungen:
**WO-A-87/04349**

(73) Patentinhaber: **HOECHST AKTIENGESELLSCHAFT**

**D-65926 Frankfurt(DE)**

(72) Erfinder: **Rüger, Wolfgang, Dr.**
**Parkstrasse 10**
**D-6233 Kelkheim (Taunus)(DE)**
Erfinder: **Urbach, Hansjörg, Dr.**
**Le Lavandoustrasse 41**
**D-6242 Kronberg/Taunus(DE)**
Erfinder: **Ruppert, Dieter, Dr.**
**Schreyerstrasse 30**
**D-6242 Kronberg/Taunus(DE)**
Erfinder: **Schölkens, Bernward, Dr.**
**Hölderlinstrasse 62**
**D-6233 Kelkheim (Taunus)(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.6/3.3.1)

## Beschreibung

Die Erfindung betrifft Enzym-hemmende Aminosäurederivate, die ein Nitron-Strukturelement in der "$P_1$-$P_1$'-Position" enthalten, Verfahren zu ihrer Herstellung, diese enthaltende Mittel und ihre Verwendung.

Es sind zahlreiche Derivate von Aminosäuren, Dipeptiden und Oligopeptiden bekannt, die in der Lage sind, Aspartylproteasen wie z. B. das Enzym Renin zu inhibieren. Eine Übersicht darüber gibt z.B. W. Greenlee, Pharmac. Res. 4 (1987) 364. Die wirksamsten dieser Verbindungen beruhen auf dem Prinzip der Übergangszustands-Analoga und enthalten in der entsprechenden Position ($P_1$-$P_{1'}$ in der Notation von Schechter und Berger, Biochem. Biophys. Res. Commun. 27 (1967) 157) ein Hydroxy-Isosteres, z.B. in Form eines Statin- oder Norstatinderivates, oder ein Dihydroxy-Isosteres, z.B. in Form eines Aminodiolderivates.

Der Erfindung liegt die Aufgabe zugrunde, neue Verbindungen zu suchen, die sich von den bekannten Strukturen unterscheiden, d.h. nicht die als essentiell angesehene Hydroxy-Funktion der bekannten Übergangszustands-Analoga aufweisen, und dennoch hochwirksame Inhibitoren von Aspartylproteasen wie z. B. Renin und retroviralen Proteasen wie z. B. der HIV-Protease in vitro und in vivo sind.

Diese Aufgabe wird erfindungsgemäß durch die Verbindungen der allgemeinen Formel I gelöst,

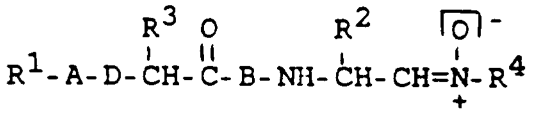

(I)

in welcher

$R^1$ Wasserstoff, ($C_1$-$C_{18}$)-Alkyl, ($C_3$-$C_7$)-Cycloalkyl, die jeweils durch Amino, Hydroxy, Mercapto, Halogen, ($C_1$-$C_4$)-Alkoxy, Mono- oder Di-($C_1$-$C_4$)-alkylamino, Carboxy, ($C_1$-$C_4$)-Alkoxycarbonyl, Phenoxy, Phenyl-($C_1$-$C_4$)-alkoxy, Phenyl-($C_1$-$C_4$)-alkoxycarbonyl oder einen Rest -$CONR^5R^6$ substituiert sein können; ($C_1$-$C_4$)-Alkoxy, ($C_6$-$C_{14}$)-Aryl, ($C_6$-$C_{14}$)-Aryl-($C_1$-$C_4$)alkyl, ($C_6$-$C_{14}$)-Aryl-($C_1$-$C_4$)-alkoxy, wobei der Arylrest jeweils durch ein, zwei oder drei Reste der Gruppe ($C_1$-$C_6$)-Alkyl, Amino, Mono- oder Di-($C_1$-$C_4$)-alkylamino, Amino-($C_1$-$C_4$)-alkyl, Hydroxy-($C_1$-$C_4$)-alkyl, Mono- oder Di-($C_1$-$C_4$)-alkylamino-($C_1$-$C_4$)-alkyl, Hydroxy, ($C_1$-$C_4$)-Alkoxy, Halogen, Formyl, ($C_1$-$C_4$)-Alkoxycarbonyl, Carboxamido, Mono- oder Di-($C_1$-$C_4$)-alkylaminocarbonyl oder Nitro oder durch einen Methylendioxyrest substituiert sein kann; Het oder Het-($C_1$-$C_4$)-alkyl, wobei Het einen 5-, 6- oder 7-gliedrigen heterocyclischen Ring darstellt, der benzanneliert und entweder aromatisch, teilhydriert oder vollständig hydriert sein kann und der als Heteroelemente ein oder zwei Reste der Gruppe N, O, S, NO, SO oder $SO_2$ enthalten und durch ein oder zwei Reste der Gruppe ($C_1$-$C_4$)-Alkyl, ($C_1$-$C_4$)-Alkoxy, ($C_1$-$C_4$)-Alkoxycarbonyl, Hydroxy, Halogen, Amino, Mono- oder Di-($C_1$-$C_4$)-alkylamino substituiert sein kann oder einen Rest -$NR^5R^6$ bedeutet, wobei

$R^5$ und $R^6$ gleich oder verschieden und voneinander unabhängig Wasserstoff, ($C_1$-$C_8$)-Alkyl, das durch Amino, ($C_1$-$C_4$)-Alkylamino, Di-($C_1$-$C_4$)-alkylamino, Hydroxy oder ($C_1$-$C_4$)-Alkoxy substituiert sein kann, ($C_3$-$C_7$)-Cycloalkyl, Mercapto, ($C_1$-$C_4$)-Alkylthio, Phenylthio, ($C_1$-$C_4$)-Alkoxycarbonyl, Carboxy, ($C_6$-$C_{14}$)-Aryl, das im Arylrest wie bei $R^1$ beschrieben, substituiert sein kann, Het oder Het-($C_1$-$C_4$)-alkyl, wobei Het wie bei $R^1$ beschrieben definiert ist, bedeutet oder wobei

$R^5$ und $R^6$ zusammen mit dem sie tragenden Stickstoffatom einen 5- bis 12-gliedrigen Ring bilden, der mono-oder bicyclisch sein kann und als weitere Ringglieder noch 1 oder 2 Stickstoffatome, 1 Schwefelatom oder Sauerstoffatom enthalten und durch ($C_1$-$C_4$)-Alkyl substituiert sein kann, bilden:

A einen Rest der Gruppe S, SO, $SO_2$, O, CO, CS oder eine direkte Bindung bedeutet;

D eine $CH_2$-Gruppe oder einen Rest -$NR^7$-, wobei $R^7$ Wasserstoff oder ein ($C_1$-$C_4$)-Alkylrest sein kann, bedeutet;

$R^3$ ($C_6$-$C_{14}$)-Aryl, ($C_6$-$C_{14}$)-Aryl-($C_1$-$C_4$)-alkyl, wobei der Arylrest jeweils durch ein, zwei oder drei Reste wie unter $R^1$ beschrieben, substituiert sein kann, Thienyl oder Thienyl-($C_1$-$C_4$)-alkyl, wobei der Thiophenrest jeweils durch ein oder zwei Reste der Gruppe ($C_1$-$C_4$)-Alkyl, ($C_1$-$C_4$)-Alkoxy oder Halogen substituiert sein kann, 2-, 3- oder 4-Pyridyl oder 2-, 3- oder 4-Pyridyl-($C_1$-$C_4$)-alkyl, wobei der Pyridinrest durch ein oder zwei Reste der Gruppe ($C_1$-$C_4$)-Alkyl, ($C_1$-$C_4$)-Alkoxy oder Halogen substituiert sein kann, bedeutet;

B einen N-terminal über $-NR^8-$ mit $R^1$-A-D-$CHR^3$-CO- und C-terminal über -CO- mit

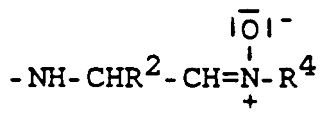

verknüpften Rest einer Aminosäure aus der Reihe Phenylalanin, Histidin, Tyrosin, Tryptophan, Methionin, Leucin, Isoleucin, Asparagin, Asparaginsäure, β-2-Thienylalanin, β-3-Thienylalanin, β-2-Furylalanin, β-3-Furylalanin, Lysin, Ornithin, Valin, Alanin, 2,4-Diaminobuttersäure, Arginin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, Norvalin, β-2-Benzo[b]thienylalanin, β-3-Benzo[b]thienylalanin, 2-Fluorphenylalanin, 3-Fluorphenylalanin, 4 Fluorphenylalanin, Norleucin, Cystein, S-Methyl-Cystein, 1,2,3,4-Tetrahydroisochinolin-3-carbonsäure, Homophenylalanin, DOPA, O-Dimethyl-DOPA, 2-Amino-4-(2-thienyl)-buttersäure, Benzodioxol-5-yl-alanin, N-Methyl-histidin, 2-Amino-4-(3-thienyl)-buttersäure, 3-(2-Thienyl)-serin, (Z)-Dehydrophenylalanin, (E)-Dehydrophenylalanin, (1,3-Dioxolan-2-yl)alanin, N-Pyrrolylalanin, (1-, 3-oder 4-Pyrazolyl)-alanin, (4-Thiazolyl)alanin, (2-, 4- oder 5-Pyrimidyl)alanin, Cyclopentylglycin, tert.Butylglycin oder Phenylserin, bedeutet und $R^8$ Wasserstoff, $(C_1$-$C_6)$-Alkyl, Formyl, $(C_1$-$C_6)$-Alkoxycarbonyl oder Benzyloxycarbonyl bedeutet;

$R^2$ Wasserstoff, $(C_1$-$C_{10})$-Alkyl, $(C_4$-$C_7)$-Cycloalkyl, $(C_4$-$C_7)$-Cycloalkyl-$(C_1$-$C_4)$-alkyl, $(C_6$-$C_{14})$-Aryl, $(C_6$-$C_{14})$-Aryl$(C_1$-$C_4)$-alkyl oder (Heterocyclyl)-$(C_1$-$C_4)$-alkyl bedeutet, wobei der Heterocyclus 4-7 Ringglieder, davon 1 oder 2 Schwefel- und/oder Sauerstoffatome besitzt, bedeutet; und

$R^4$ $(C_1$-$C_8)$-Alkyl, $(C_3$-$C_8)$-Cycloalkyl, $(C_3$-$C_8)$-Cycloalkyl-$(C_1$-$C_4)$-alkyl, die jeweils durch Amino, Hydroxy, Mercapto, Halogen, $(C_1$-$C_4)$-Alkoxy, Mono- oder Di-$(C_1$-$C_4)$-alkylamino, Carboxy, Guanidino, $(C_1$-$C_4)$-Alkoxycarbonyl oder einen Rest $-CONR^5R^6$, worin $R^5$ und $R^6$ wie oben definiert sind, einfach, zweifach oder dreifach substituiert sein können; $(C_6$-$C_{14})$-Aryl, $(C_6$-$C_{14})$-Aryl$(C_1$-$C_4)$-alkyl, wobei der Arylrest wie unter $R^1$ beschrieben und die Alkylkette wie oben für $R^4$ beschrieben, substituiert sein kann; Het oder Het-$(C_1$-$C_4)$-alkyl, wobei Het wie unter $R^1$ beschrieben, definiert ist und die Alkylkette wie oben für $R^4$ beschrieben, substituiert sein kann;

sowie deren physiologisch verträgliche Salze.

Die Chiralitätszentren in den Verbindungen der Formel I können die R-, S- oder R,S-Konfiguration aufweisen.

Alkyl kann geradkettig oder verzweigt sein. Entsprechendes gilt für davon abgeleitete Reste, wie z.B. Alkoxy, Alkylthio, Alkylamino, Dialkylamino und Alkanoyl.

Unter Cycloalkyl werden auch alkylsubstituierte Reste, wie z.B. 4-Methylcyclohexyl oder 2,3-Dimethylcyclopentyl verstanden.

Halogen steht für Fluor, Chlor, Brom oder Jod, bevorzugt für Fluor oder Chlor.

$(C_6$-$C_{14})$-Aryl ist beispielsweise Phenyl, Naphthyl, Biphenylyl oder Fluorenyl; bevorzugt ist Phenyl. Entsprechendes gilt für $(C_6$-$C_{14})$-Aryl-$(C_1$-$C_4)$-alkyl. Bevorzugte Reste dieser Art sind z.B. Benzyl, α- und β-Naphthylmethyl, Halobenzyl und Alkoxybenzyl.

Ein Rest Het im Sinne vorstehender Definition ist beispielsweise Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl oder ein benzannelliertes, cyclopenta-, cyclohexa- oder cyclohepta-annelliertes Derivat dieser Reste.

Bevorzugte Reste Het sind 2- oder 3-Pyrrolyl, Phenyl-pyrrolyl, z.B. 4- oder 5-Phenyl-2-pyrrolyl, 2-Furyl, 2-Thienyl, 4-Imidazolyl, Methyl-imidazolyl, z.B. 1-Methyl-2-, 4- oder 5-imidazolyl, 1,3-Thiazol-2-yl, 2-, 3- oder 4-pyridyl, 1-Oxido-2-, 3- oder 4-pyridinio, 1- oder 2-Pyrazinyl, 2-, 4- oder 5-Pyrimidinyl, 4-Morpholinyl, 2-, 3- oder 5-Indolyl, substituiertes 2-Indolyl, z.B. 1-Methyl-, 5-Methyl-, 5-Methoxy, 5-Benzyloxy-, 5-Chlor- oder 4,5-Dimethyl-2-indolyl, 1-Benzyl-2 oder 3-indolyl, 4,5,6,7-Tetrahydro-2-indolyl, Cyclohepta[b]-5-pyrrolyl, 2-, 3- oder 4-Chinolyl, 4-Hydroxy-2-chinolyl, 1-, 3- oder 4-Isochinolyl, 1-Oxo-1,2-dihydro-3-isochinolyl, 2-Chinoxalinyl, 2-Benzofuranyl, 2-Benzoxazolyl, 2-Benzthiazolyl, Benz[e]indol-2-yl, β-Carbolin-3-yl, 2-Oxazolinyl, 4-Alkyl-2-oxazolinyl oder 4,5-Dialkyloxazolinyl.

Unter Salzen von Verbindungen der Formel I sind insbesondere pharmazeutisch verwendbare oder nicht-toxische Salze zu verstehen.

Solche Salze werden beispielsweise von Verbindungen der Formel I, welche saure Gruppen, z.B. Carboxy, enthalten, mit Alkali- oder Erdalkalimetallen gebildet, wie Na, K, Mg und Ca, sowie mit physiologisch verträglichen organischen Aminen, wie z.B. Triethylamin und Tri-(2-hydroxy-ethyl)-amin.

Verbindungen der Formel I, welche basische Gruppen, z.B. eine Aminogruppe enthalten, bilden Salze mit anorganischen Säuren, wie z.B. Salzsäure, Schwefelsäure oder Phosphorsäure und mit organischen Carbon- oder Sulfonsäuren, wie z.B. Essigsäure, Citronensäure, Benzoesäure, Maleinsäure, Fumarsäure, Weinsäure und p-Toluolsulfonsäure.

Bevorzugt sind Verbindungen der Formel I, in welcher

$R^1$ Methyl, Ethyl, Isopropyl, tert.-Butyl, Isobutyl, 2-Hydroxyethyl, 2-Methoxyethyl, Carboxymethyl, 2-Carboxyethyl, Methoxycarbonylmethyl, 2-Methoxycarbonylethyl, Ethoxycarbonylmethyl, 2-Ethoxycarbonylethyl, Carbamoylmethyl, 2-Carbamoylethyl, 2-Aminoethyl, 2-Dimethylaminoethyl, 2-Morpholinoethyl, Aminoisobutyl, 2-Piperidinoethyl, Aminopropyl, Dimethylaminopropyl, Methylaminopropyl, Piperidinopropyl, Morpholinopropyl, Methylaminoisobutyl, Dimethylaminoisobutyl, Piperidinoisobutyl, Morpholinoisobutyl, Cyclopropyl, Cyclobutyl, Cyclopentyl oder Cyclohexyl, Phenyl, 1- oder 2-Naphthyl, o-, m- oder p-Methylphenyl, o-, m- oder p-Hydroxyphenyl oder o-, m-oder p-Aminophenyl, Benzyl, 2-Phenylethyl oder $\alpha$- oder $\beta$-Naphthylmethyl, unsubstituiertes oder substituiertes Heteroaryl, z.B. 2- oder 3-Pyrrolyl, 2-Furyl, 2-Thienyl, 2- oder 4-Imidazolyl, 1-Methyl-2-, -4- oder -5-Imidazolyl, 1,3-Thiazol-2-yl, 2-, 3- oder 4-Pyridyl, 1-Oxido-2-, 3- oder 4-pyridinio, 2-Pyrazinyl, 2-, 4- oder 5-Pyrimidinyl, 2-, 3- oder 4-Chinolyl, 1-, 3- oder 4-Isochinolyl oder 2-Benzoxazolyl, Methoxy, Ethoxy oder n-Butoxy oder einen Rest $-NR^5R^6$ bedeutet, worin

$R^5$ und $R^6$ gleich oder verschieden und voneinander unabhängig Wasserstoff, Methyl, Ethyl, Propyl, Butyl, Isobutyl, sec.-Butyl, tert.-Butyl, Aminoethyl, Aminopropyl, Methylaminoethyl, Dimethylaminoethyl, Methylaminopropyl, Dimethylaminopropyl, Hydroxyethyl, Methoxyethyl, Cyclohexylmethyl, Mercaptoethyl, Methylthioethyl, Benzyl, 1-Phenethyl, 2-Phenethyl, 2-(3,4-Dimethoxy)-phenethyl, 2-Pyridylmethyl, 3-Pyridylmethyl bedeuten oder worin

$R^5$ und $R^6$ zusammen mit dem sie tragenden Stickstoffatom einen Pyrrolidin-, Piperidin-, Azepin, Azocin-, Morpholin-, Piperazin-, 4-Methyl-piperazin-, 4-Ethyl-piperazin-, Homopiperazin- oder Thiomorpholin-Ring bilden;

A einen Rest der Gruppe S, SO, $SO_2$, O, CO oder CS bedeutet;

D eine $CH_2$-Gruppe, eine NH-Gruppe oder eine $N(CH_3)$-Gruppe bedeutet;

$R^3$ Phenyl, 2-Thienyl, 2-Pyridyl, 1-Naphthyl, Phenyl-($C_1$-$C_4$)-alkyl, 2-Thienyl-($C_1$-$C_4$)-alkyl, 2-Pyridyl-($C_1$-$C_4$)-alkyl, 1-Naphthyl-($C_1$-$C_4$)-alkyl, die jeweils gegebenenfalls durch einen, zwei oder drei Reste aus der Gruppe Methyl, Ethyl, Isopropyl, tert.-Butyl, Methoxy, Hydroxy, Fluor, Chlor oder Nitro oder einen Methylendioxyrest substituiert sind, bedeutet;

B einen N-terminal über -NH- mit $R^1$-A-D-$CHR^3$-CO- und C-terminal über -CO- mit

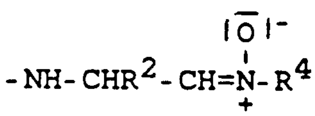

verknüpften Rest einer Aminosäure aus der Reihe Phenylalanin, Histidin, Tyrosin, Tryptophan, Methionin, Leucin, Isoleucin, Asparagin, Asparaginsäure, $\beta$-2-Thienylalanin, $\beta$-3-Thienylalanin, $\beta$-2-Furylalanin, $\beta$-3-Furylalanin, Lysin, Ornithin, 2,4-Diaminobuttersäure, Arginin, Norvalin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, Norleucin, Valin, Alanin, Cystein, S-Methylcystein, N-Methyl-Histidin, Benzodioxol-5-yl-alanin, 1,2,3,4-Tetrahydroisochinolin-3-carbonsäure, Homophenylalanin, 2-Amino-4-(3-thienyl)buttersäure, (Z)-Dehydrophenylalanin, (E)-Dehydrophenylalanin, (1,3-Dioxolan-2-yl)alanin, (4-Thiazolyl)alanin, (2-, 4- oder 5-Pyrimidyl)alanin, (1-,3- oder 4-Pyrazolyl)alanin, (2-,3- oder 4-Fluorphenyl)alanin, Cyclopentylglycin. tert.-Butylglycin oder Phenylserin bedeutet;

$R^2$ wie auf Seite 4 definiert ist, und

$R^4$ Methyl, Ethyl, Propyl, Isopropyl, Butyl, Isobutyl, sec.-Butyl, tert.-Butyl, Pentyl, Isopentyl,

Neopentyl, sec.-Pentyl, tert.-Pentyl, Hexyl, Cyclopentyl, Cyclohexyl, Cycloheptyl, Cyclopentylmethyl, Cyclohexylmethyl, Cycloheptylmethyl, Phenyl, Benzyl, Phenylethyl, Phenylpropyl, 2-, 3- oder 4-Pyridyl, 2-, 4- oder 5-Imidazolyl, (2-, 3- oder 4-Pyridyl)methyl, (2-, 4- oder 5-Imidazolyl)methyl, wobei die genannten Alkyl-und Cycloalkylreste jeweils durch Amino, Halogen, Mono-oder Di-($C_1$-$C_4$)-alkylamino, Carboxy, Guanidino, ($C_1$-$C_4$)-Alkoxycarbonyl oder einen Rest -CONR$^5$R$^6$-, worin $R^5$ und $R^6$ wie auf Seite 7 definiert sind, einfach oder zweifach substituiert sein können; die genannten Phenylreste einfach oder zweifach durch ($C_1$-$C_4$)-Alkyl, Methoxy, Hydroxy, Amino, Aminomethyl, Fluor, Chlor oder Trifluormethyl substituiert sein können und die genannten heteroaromatischen Reste jeweils durch ein oder zwei Reste aus der Reihe ($C_1$-$C_4$)-Alkyl, Methoxy, Fluor, Chlor, Brom oder Trifluormethyl substituiert sein können, bedeutet

sowie deren physiologisch verträgliche Salze.

Besonders bevorzugt sind Verbindungen der Formel I, in welcher

$R^1$ Methyl, Ethyl, Isopropyl, tert.-Butyl, Isobutyl, 2-Hydroxyethyl, 2-Methoxyethyl, Carboxymethyl, 2-Carboxyethyl, Methoxycarbonylmethyl, 2-Methoxycarbonylethyl, Ethoxycarbonylmethyl, 2-Ethoxycarbonylethyl, Carbamoylmethyl, 2-Carbamoylethyl, 2-Aminoethyl, 2-Dimethylaminoethyl, 2-Morpholinoethyl, Aminopropyl, Aminoisobutyl, Methylaminosobutyl, Dimethylaminoisobutyl, 2-Piperidinoethyl, Cyclopropyl, Cyclobutyl, Cyclopentyl oder Cyclohexyl, Phenyl, 1- oder 2-Naphthyl, o-, m- oder p-Methylphenyl, o-, m- oder p-Hydroxyphenyl oder o-, m- oder p-Aminophenyl, Benzyl, 2-Phenylethyl oder $\alpha$- oder $\beta$-Naphthylmethyl, unsubstituiertes oder substituiertes Heteroaryl, z.B. 2- oder 3-Pyrrolyl, 2-Furyl, 2-Thienyl, 2- oder 4-Imidazolyl, 1-Methyl-2-, -4- oder -5-Imidazolyl, 1,3-Thiazol-2-yl, 2-, 3- oder 4-Pyridyl, 1-Oxido-2-, 3- oder 4-pyridinio, 2-Pyrazinyl, 2-, 4- oder 5-Pyrimidinyl, 2-, 3- oder 4-Chinolyl, 1-, 3- oder 4-Isochinolyl oder 2-Benzoxazolyl, Methoxy, Ethoxy oder n-Butoxy oder einen Rest -NR$^5$R$^6$ bedeuten, worin

$R^5$ und $R^6$ gleich oder verschieden und voneinander unabhängig Wasserstoff, Methyl, Ethyl, Propyl, Butyl, Isobutyl, sec.-Butyl, tert.-Butyl, Hydroxyethyl, Methoxyethyl, Aminoethyl, Aminopropyl, Benzyl- oder Pyridylmethyl bedeuten oder worin

$R^5$ und $R^6$ zusammen mit dem sie tragenden Stickstoffatom einen Pyrrolidin-, Piperidin-, Morpholin- oder Piperazinring bilden;

A einen Rest der Gruppe S, SO, $SO_2$, O, CO oder CS bedeutet;

D eine $CH_2$-Gruppe, eine NH-Gruppe oder eine N($CH_3$)-Gruppe bedeutet;

$R^3$ Aryl oder Arylmethyl bedeutet, wobei Aryl, Phenyl, 2-Thienyl, 2-Pyridyl, 1-Naphthyl bedeutet, die jeweils gegebenenfalls durch Hydroxy, Dihydroxy, Methoxy, Dimethoxy, Fluor, Chlor oder Methylendioxy substituiert sind;

B einen N-terminal über -NH- mit R$^1$-A-D-CHR$^3$-CO- und C-terminal über -CO- mit

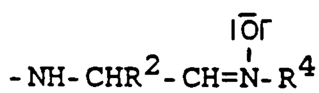

verknüpften Rest einer Aminosäure aus der Reihe Phenylalanin, Histidin, Leucin, $\beta$-2-Thienylalanin, $\beta$-3-Thienylalanin, Lysin, Norvalin, 2-Fluorphenylalanin, 3-Fluorphenylalanin, 4-Fluorphenylalanin, Norleucin, S-Methylcystein, (1,3-Dioxolan-2-yl)alanin, (1-,3- oder 4-Pyrazolyl)alanin, 4-Thiazolylalanin, (2-,4- oder 5-Pyrimidyl)alanin, bedeutet;

$R^2$ Isobutyl, Cyclohexylmethyl, Benzyl oder (1,3-Dithiolan-2-yl)methyl bedeutet; und

$R^4$ Methyl, Ethyl, Propyl, Isopropyl, Butyl, Isobutyl, sec.-Butyl, tert. Butyl, Pentyl, Isopentyl, Neopentyl, sec.-Pentyl, tert. Pentyl, Hexyl, Cyclopentyl, Cyclohexyl, Cyclopentylmethyl, Cyclohexylmethyl, Phenyl, Benzyl, Phenylethyl, 2-,3- oder 4-Pyridyl, 2-Imidazolyl, (2-,3- oder 4-Pyridyl)methyl, 2-Imidazolylmethyl, wobei die genannten Alkyl- und Cycloalkylreste jeweils durch Amino, Fluor, Chlor, Methylamino, Ethylamino, Dimethylamino, Diethylamino, Carboxy, Guanidino, Methoxycarbonyl, Ethoxycarbonyl, tert. Butyloxycarbonyl, oder einen Rest -CONR$^5$R$^6$, worin $R^5$ und $R^6$ wie auf Seite 10 definiert sind, einfach oder zweifach, substituiert sein können, die genannten Phenylreste ein- oder zweifach durch Methyl, Methoxy, Hydroxy, Amino, Aminomethyl, Fluor, Chlor oder Trifluormethyl substituiert sein können und die genannten heteroaromatischen Reste mit jeweils ein oder zwei Resten aus der Reihe Methyl, Methoxy, Fluor, Chlor oder Trifluormethyl substituiert sein können, bedeutet, sowie deren physiologisch vertäglichen Salze.

Die Erfindung betrifft weiterhin ein Verfahren zur Herstellung von Verbindungen der Formel I, das dadurch gekennzeichnet ist, daß man

a) eine Verbindung der allgemeinen Formel II

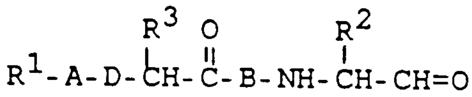

(II)

in welcher $R^1$, $R^2$, $R^3$, A, B und D die gleiche Bedeutung wie in Formel I besitzen, mit einem Hydroxylaminderivat der allgemeinen Formel III

$HO-NH-R^4$ (III)

worin $R^4$ die gleiche Bedeutung wie in Formel I besitzt, in einem organischen Lösungsmittel wie z.B. Diethylether, Ethanol, Benzol, Toluol oder in Wasser oder ohne jedes Lösungsmittel bei einer Temperatur zwischen -20 °C und dem Siedepunkt des Lösungsmittels, vorzugsweise zwischen -20 °C und +60 °C, mit oder ohne Gegenwart einer Säure, z.B. Chlorwasserstoff oder Essigsäure, die z.B. in Form der Salze der Hydroxylamine der Formel III eingebracht werden kann, mit oder ohne Gegenwart einer Hilfsbase wie z.B. Natriumhydrogencarbonat, Natriumacetat, Natriumcarbonat, Natriumethanolat oder Kaliumhydroxid sowie mit oder ohne Gegenwart eines wasserentziehenden Mittels wie z.B. Calciumchlorid oder Molekularsieb, umsetzt und gegebenenfalls zum Schutz weiterer funktioneller Gruppen (eine) temporär eingeführte Schutzgruppe(n) abspaltet

oder

b) ein Fragment mit endständiger Carboxylgruppe oder dessen reaktives Derivat mit einem entsprechenden Fragment mit freier Aminogruppe kuppelt, gegebenenfalls zum Schutz weiterer funktioneller Gruppen (eine) temporär eingeführte Schutzgruppe(n) abspaltet und die so erhaltene Verbindung gegebenenfalls in ihr physiologisch verträgliches Salz überführt.

Die Verbindung der allgemeinen Formel II lassen sich beispielsweise herstellen aus Aminoalkoholen der Formel IV

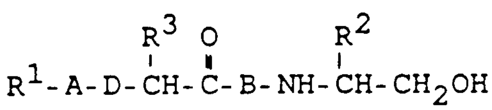

(IV)

in welchen $R^1$, $R^2$, $R^3$, A, B, D die gleiche Bedeutung wie in der Formel I besitzen, durch Oxidation unter Bedingungen, wie sie für die Überführung N-geschützter 2-Aminoalkohole in N-geschützte 2-Aminoaldehyde literaturbekannt sind

oder

aus Aminosäurederivaten der Formel V

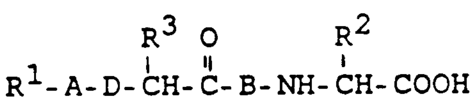

(V)

in welchen $R^1$, $R^2$, $R^3$, A B, D die gleiche Bedeutung wie in der Formel I besitzen, durch ein- oder zweistufige Reduktion unter Bedingungen, wie sie für die Überführung N-geschützter Aminosäuren in N-geschützte 2-Aminoaldehyde literaturbekannt sind.

Besonders vorteilhaft ist die Herstellung der Aldehyde der Formel II aus den Carbonsäuren der Formel V durch sukzessive Reaktion mit N,O-Dimethylhydroxylamin und anschließende Reduktion des resultierenden N,O-Dimethylhydroxylamids mit Lithiumaluminiumhydrid oder Natrium-bis-(2-methoxyethoxy)-dihydroaluminat nach der Methode von Castro et al., Synthesis 1983, 676.

Die Herstellung der Carbonsäuren der allgemeinen Formel V kann ihrerseits vorteilhaft durch Kupplung eines Fragments mit endständiger Carboxylgruppe der Formel VIa oder VIb

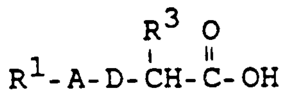 (VIa)

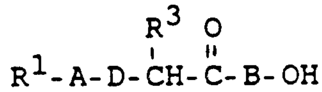 (VIb),

worin $R^1$, $R^3$, A, B und D die gleiche Bedeutung wie in Formel I besitzen oder eines reaktiven Derivats dieses Fragments mit einem entsprechenden Fragment mit freier Aminogruppe der Formel VIIa oder VIIb

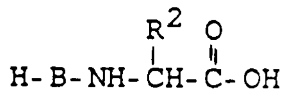 (VIIa)

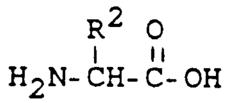 (VIIb)

worin $R^2$ und B die gleiche Bedeutung wie in Formel I besitzen, erfolgen, wobei gegebenenfalls zum Schutz der Carboxylgruppe in VIIa und VIIb temporär eine Schutzgruppe eingeführt und nach Durchführung der Kupplung wieder abgespalten wird, in Analogie zu den nachstehend für die Verfahrensvariante b) erläuterten Fragment-Kupplungen.

Die unter der Verfahrensvariante b) erwähnten Fragmente einer Verbindung der Formel I mit einer endständigen Carboxylgruppe besitzen die Formeln VIa und VIb. Fragmente einer Verbindung der Formel I mit einer endständigen Aminogruppe besitzen die nachstehenden Formeln VIIIa und VIIIb.

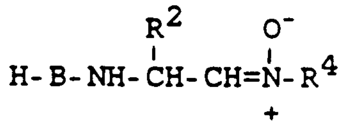 (VIIIa)

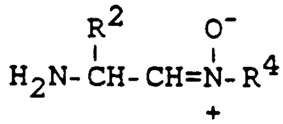 (VIIIb)

Methoden, die zur Herstellung einer Amidbindung geeignet sind, werden z.B. in Houben-Weyl, Methoden der organischen Chemie, Band 15/2; Bodanszky et al., Peptide Synthesis, 2nd ed. (Wiley & Sons, New York 1976) oder Gross, Meienhofer, The Peptides. Analysis, synthesis, biology (Academic Press, New York 1979) beschrieben. Vorzugsweise werden die folgenden Methoden herangezogen Aktivestermethode mit N-Hydroxy-succinimid als Esterkomponente, Kupplung mit einem Carbodiimid wie Dicyclohexylcarbodiimid oder mit Propanphosphonsäureanhydrid und die Gemischt-Anhydrid-Methode mit Pivaloylchlorid.

Fragmente der Formel VIIIa und VIIIb lassen sich herstellen aus geschützten Aminoaldehyden durch Reaktion mit Hydroxylaminen der Formel III unter analogen Bedingungen, wie sie unter der Verfahrensvariante a) beschrieben sind, gefolgt von einer Abspaltung der Schutzgruppe.

Die Hydroxylaminderivate der Formel III sind großenteils literaturbekannt bzw. auf analogem Wege wie die bekannten Verbindungen zugänglich, z.B. durch Reduktion der entsprechenden Oximderivate mit Borwasserstoff/Tetrahydrofuran. Hydroxylaminderivate der Formel III, in welchen $R^4$ einen Carboxy-($C_1$-$C_4$)-alkoxycarbonyl- oder -$CONR^5R^6$-Substituenten in -Position trägt, sind N-Hydroxyaminosäurederivate, deren Herstellung ebenfalls literaturbekannt ist (z.B. HCJ Ottenheijm et al., Chem. Rev. 86, 697 (1986)) bzw. auf analogen Wege erfolgen kann.

Die zur Herstellung von Verbindungen der Formel I erforderlichen Vor- und Nachoperationen wie Einführung und Abspaltung von Schutzgruppen sind literaturbekannt und sind z.B. in T.W. Greene, "Protective Groups in Organic Synthesis" beschrieben. Salze von Verbindungen der Formel I mit salzbildenden Gruppen werden in an sich bekannter Weise hergestellt, indem man z.B. eine Verbindung der Formel I mit einer basischen Gruppe mit einer stöchiometrischen Menge einer geeigneten Säure umsetzt. Stereoisomerengemische, insbesondere Diastereomerengemische, können in an sich bekannter Weise durch fraktionierte Kristallisation oder durch Chromatographie getrennt werden.

In einer erhältlichen Verbindung der Formel I kann man eine Thiogruppe zu einer Sulfinyl- oder Sulfonylgruppe oder eine Sulfinylgruppe zu einer Sulfonylgruppe oxidieren.

Die Oxidation zur Sulfonylgruppe kann mit den meisten der üblichen Oxidationsmittel durchgeführt werden. Bevorzugt verwendet man solche Oxidationsmittel, die die Thiogruppe oder Sulfinylgruppe selektiv in Gegenwart anderer funktioneller Gruppen der Verbindung der Formel I, z.B. der Amidfunktion und der Hydroxygruppe, oxidieren, beispielsweise aromatische oder aliphatische Peroxycarbonsäuren, z.B. Perbenzoesäure, Monoperphthalsäure, m-Chlorperbenzoesäure, Peressigsäure, Perameisensäure oder Trifluorperessigsäure.

Die erfindungsgemäßen Verbindungen der Formel I weisen enzymhemmende Eigenschaften auf; insbesondere hemmen sie die Wirkung des natürlichen Enzyms Renin und retrovirale Proteasen wie z. B. die HIV-Protease. Renin ist ein proteolytisches Enzym aus der Klasse der Aspartyl-Proteasen, welches als Folge verschiedener Stimuli (Volumendepletion, Natriummangel, $\beta$-Rezeptorenstimulation) von den juxtaglomerulären Zellen der Niere in den Blutkreislauf sezerniert wird. Dort spaltet es von dem aus der Leber ausgeschiedenen Angiotensinogen das Decapeptid Angiotensin I ab. Dieses wird durch das "angiotensin converting enzyme" (ACE) in Angiotensin II überführt. Angiotensin II spielt eine wesentliche Rolle bei der Blutdruckregulation, da es direkt den Blutdruck durch Gefäßkontraktion steigert. Zusätzlich stimuliert es die Sekretion von Aldosteron aus der Nebenniere und erhöht auf diese Weise über die Hemmung der Natrium-Ausscheidung das extrazelluläre Flüssigkeitsvolumen, was seinerseits zu einer Blutdrucksteigerung beiträgt. Hemmer der enzymatischen Aktivität des Renins bewirken eine verminderte Bildung von Angiotensin I, was eine verminderte Bildung von Angiotensin II zur Folge hat. Die Erniedrigung der Konzentration dieses aktiven Peptidhormons ist die direkte Ursache für die blutdrucksenkende Wirkung von Renin-Hemmern.

Die Wirksamkeit von Renin-Hemmern kann durch in-vitro-Tests überprüft werden. Hierbei wird die Verminderung der Bildung von Angiotensin I in verschiedenen Systemen (Humanplasma, gereinigtes Humanrenin) gemessen.

**1. Testprinzip**

Z.B. Humanplasma, welches sowohl Renin als auch Angiotensinogen enthält, wird bei 37 °C mit der zu testenden Verbindung inkubiert. Dabei wird aus Angiotensinogen unter der Einwirkung von Renin Angiotensin I freigesetzt, das anschließend mit einem handelsüblichen Radioimmunoassay gemessen werden kann. Diese Angiotensin-Freisetzung wird durch Renin-Inhibitoren gehemmt.

**2. Gewinnung des Plasmas**

Das Blut wird von freiwilligen Probanden gewonnen (ca. 0,5 l pro Person; Bluko-Entnahmegerät der Fa. ASID Bonz und Sohn, Unterschleißheim) und in teilweise evakuierten Flaschen unter Eiskühlung aufgefangen. Die Gerinnung wird durch Zugabe von EDTA (Endkonzentration 10 mM) verhindert. Nach dem Zentrifugieren (Rotor HS 4 (Sorvall), 3 500 Upm, 0-4 °C, 15 min; wiederholen, falls erforderlich) wird das Plasma vorsichtig abpipettiert und in geeigneten Portionen bei -30 °C eingefroren. Für den Test werden nur Plasmen mit ausreichend hoher Reninaktivität verwendet. Plasmen mit niedriger Reninaktivität werden durch eine Kältebehandlung (-4 °C, 3 Tage) aktiviert (Prorenin → Renin).

**3. Durchführung des Tests**

Angiotensin I wird mit dem Renin-Maia®-Kit (Serono Diagnostics S.A., Coinsins, Schweiz) bestimmt. Die Inkubation des Plasmas wird nach der dort angegebenen Anleitung durchgeführt:
Inkubationsansatz:
1000 µl Plasma (bei 0-4 °C aufgetaut)
100 µl Phosphatpuffer (pH 7,4) Zusatz von $10^{-4}$ M Ramiprilat)
10 µl PMSF-Lösung
10 µl 0,1 % Genapol PFIC
12 µl DMSO bzw. Testpräparat

Die Testpräparate werden i.a. $10^{-2}$ M in 100 % Dimethylsulfoxid (DMSO) gelöst und mit DMSO entsprechend verdünnt; der Inkubationsansatz enthält max. 1 % DMSO.

Die Ansätze werden in Eis gemischt und für 1 Stunde zur Inkubation in ein Wasserbad (37 °C) gestellt. Aus einem zusätzlichen Ansatz ohne Inhibitor werden ohne weitere Inkubation insgesamt 6 Proben (jeweils 100 µl) zur Bestimmung des Ausgangs-Angiotensin I-Gehaltes des verwendeten Plasmas entnommen.

Die Konzentrationen der Testpräparate werden so gewählt, daß etwa der Bereich von 10-90 % Enzymhemmung abgedeckt ist (mindestens fünf Konzentrationen). Am Ende der Inkubationszeit werden aus jedem Ansatz drei 100 µl-Proben in vorgekühlten Eppendorf-Gefäßen auf Trockeneis eingefroren und bei ca. -25 °C für die Angiotensin I-Bestimmung aufbewahrt (Mittelwert aus drei Einzelproben).

**Angiotensin I-Radioimmunoassay (RIA)**

Es wird exakt die Gebrauchsanweisung des RIA-Kits (Renin-Maia®-Kit, Serono Diagnostics S.A., Coinsins, Schweiz) befolgt.

Die Eichkurve umfaßt den Bereich von 0,2 bis 25,0 ng Angiotensin I pro ml. Der Basis-Angiotensin I-Gehalt des Plasmas wird von allen Meßwerten abgezogen. Die Plasma-Renin-Aktivität (PRA) wird als ng Ang I/ml x Stunde angegeben. PRA-Werte in Gegenwart der Testsubstanzen werden auf einen Ansatz ohne Inhibitor (= 100 %) bezogen und als % Restaktivität angegeben. Aus der Auftragung von % Restaktivität gegen die Konzentration (M) des Testpräparates (logarithmische Skala) wird der $IC_{50}$-Wert abgelesen.

Die in der vorliegenden Erfindung beschriebenen Verbindungen der allgemeinen Formel I zeigen in dem in-vitro-Test Hemmwirkungen bei Konzentrationen von etwa $10^{-5}$ bis $10^{-10}$ Mol/l.

Renin-Hemmer bewirken an salzverarmten Tieren eine Blutdrucksenkung. Da sich menschliches Renin von dem Renin anderer Spezies unterscheidet, werden zum in-vivo-Test von Renin-Hemmern Primaten (Marmosets, Rhesus-Affen) herangezogen. Primaten-Renin und Human-Renin sind in ihrer Sequenz weitgehend homolog. Durch i.v. Injektion von Furosemid wird eine endogene Renin-Ausschüttung angeregt. Anschließend werden die Testverbindungen durch kontinuierliche Infusion verabreicht und ihre Wirkung auf Blutdruck und Herzfrequenz wird gemessen. Die Verbindungen der vorliegenden Erfindung sind hierbei in einem Dosisbereich von etwa 0,1 - 5 mg/kg i.v. wirksam, bei intraduodenaler Applikation per Gastroskop im Dosisbereich von etwa 1-50 mg/kg. Die in der vorliegenden Erfindung beschriebenen Verbindungen der allgemeinen Formel I können als Antihypertensiva, sowie zur Behandlung der Herzinsuffizienz verwendet werden.

Neben der Renin-Inhibitorischen Wirkung zeigen die Verbindungen der Formel I auch eine Hemmwirkung auf andere Aspartylproteasen wie z. B. retrovirale Proteasen, speziell auf die HIV-Protease.

Die HIV-Protease schneidet sich autokatalytisch aus dem GAG-POL-Polypeptid heraus und spaltet anschließend das Vorläuferpeptid p55 in die Core-Antigene p17, p24 und p14. Sie ist damit ein essentielles Enzym, dessen Inhibition den Lebenszyklus des Virus unterbricht und seine Vermehrung unterbindet.

Besondere Bedeutung hat die HIV-Protease inhibierende Wirkung, die die erfindungsgemäßen Verbindungen insbesondere zur Therapie und Prophylaxe von durch Infektion mit HIV bedingten Erkrankungen qualifiziert. Die erfindungsgemäßen Verbindungen der allgemeinen Formel I zeigen in verwendeten in-vitro-Tests Hemmwirkungen bei Konzentrationen von etwa $10^{-4}$ bis $10^{-8}$ Mol/l.

Zum Gegenstand der Erfindung gehören weiterhin die Verwendung von Verbindungen der Formel I zur Herstellung von Arzneimittel zur Bluthochdrucktherapie und der Behandlung der Herzinsuffizienz sowie zur Therapie und Prophylaxe von Viruserkrankungen, insbesondere von Erkrankungen, die durch das HIV verursacht werden sowie die genannten Arzneimittel.

Pharmazeutische Präparate enthalten eine wirksame Menge des Wirkstoffs der Formel I zusammen mit einem anorganischem oder organischen pharmazeutisch verwendbaren Trägerstoff. Die Anwendung kann intranasal, intravenös, subkutan oder peroral erfolgen. Die Dosierung des Wirkstoffs hängt von der Warmblüter-Spezies, dem Körpergewicht, Alter und von der Applikationsart ab.

Die pharmazeutischen Präparate der vorliegenden Erfindung werden in an sich bekannten Lösungs-, Misch-, Granulier-oder Dragierverfahren hergestellt.

Für eine orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wäßrige, alkoholische oder ölige Suspensionen oder wäßrige, alkoholische oder ölige Lösungen. Als inerte Träger können z.B. Gummi arabicum, Magnesia, Magnesiumcarbonat, Kaliumphosphat, Milchzucker, Glucose, Magnesiumstearylfumarat oder Stärke, insbesondere Maisstärke verwendet werden. Dabei kann die Zübereitung sowohl als Trocken- und Feuchtgranulat erfolgen. Als ölige Trägerstoffe oder Lösungsmittel kommen beispielsweise pflanzliche oder tierische Öle in Betracht, wie Sonnenblumenöl und Lebertran.

Zur subkutanen oder intravenösen Applikation werden die aktiven Verbindungen oder deren physiologisch verträgliche Salze, gewünschtenfalls mit den dafür üblichen Substanzen wie Lösungsvermittler,

Emulgatoren oder weitere Hilfsstoffe in Lösungen, Suspensionen oder Emulsionen gebracht. Als Lösungsmittel kommen z.B. in Frage Wasser, physiologische Kochsalzlösungen oder Alkohole, z.B. Ethanol, Propandiol oder Glycerin, daneben auch Zuckerlösungen wie Glucose-oder Mannitlösungen, oder auch eine Mischung aus den verschiedenen genannten Lösungsmitteln.

**Verzeichnis der verwendeten Abkürzungen:**

| | |
|---|---|
| Boc | tert.-Butoxycarbonyl |
| Cha | L-Cyclohexylalanin |
| DC | Dünnschichtchromatographie |
| DCC | Dicyclohexylcarbodiimid |
| DCI | Desorption Chemical Ionisation |
| DME | 1,2-Dimethoxyethan |
| DMF | Dimethylformamid |
| DNP | 2,4-Dinitrophenyl |
| EE | Essigester |
| EI | Electron Impact |
| EtOH | Ethanol |
| FAB | Fast atom bombardment |
| HOBt | 1-Hydroxybenzotriazol |
| Iva | Isovaleroyl |
| LAH | Lithiumaluminiumhydrid |
| M | Molekularpeak |
| MCPBA | 3-Chlorperbenzoesäure |
| MeOH | Methanol |
| MS | Massenspektrum |
| NEM | N-Ethylmorpholin |
| R.T | . Raumtemperatur |
| SC | Säulenchromatographie |
| Schmp. | Schmelzpunkt |
| Thi | β-2-Thienylalanin |
| THF | Tetrahydrofuran |

Die sonstigen für Aminosäuren verwendeten Abkürzungen entsprechen dem in der Peptidchemie üblichen Drei-Buchstaben-Code, wie er z.B. in Eur. J. Biochem. 138, 9-37 (1984) beschrieben ist. Falls nicht ausdrücklich anders angegeben, handelt es sich immer um Aminosäuren der L-Konfiguration.

Die nachstehenden Beispiele dienen zur Erläuterung der vorliegenden Erfindung, ohne diese darauf zu beschränken.

**Beispiel 1**

N-[3-Cyclohexyl-2S-[N-(isovaleroyl-L-phenylalanyl-L-norvalyl)amino]propyliden]-N-(1S-ethoxycarbonyl-2-methyl-1-propyl)amin-N-oxid

**1a) Boc-Cha-(N,O-dimethylhydroxylamid)**

100g (0,37 mol) Boc-Cha-OH und 38 g (0,39 mol) N,O-Dimethylhydroxylamin werden in 500 ml absolutem Methylenchlorid gelöst und unter leichter Kühlung 232 ml (1,68 mol) Triethylamin zugetropft. Anschließend wird bei 0 °C innerhalb von 90 Minuten, 240 ml einer Lösung von Propanphosphonsäureanhydrid in Methylenchlorid (50 %) zugetropft und 4 Stunden bei R.T. gerührt. Dann werden 500 ml Wasser zugesetzt, die organische Phase abgetrennt, je dreimal mit gesättigter Natriumbicarbonatlösung, gesättigter Natriumhydrogencarbonatlösung und gesättigter Natriumchloridlösung gewaschen, getrocknet und eingeengt. Man erhält 95,9 g der Titelverbindung als gelbes Öl.

$[\alpha]_D^{25}$ = -11,8 ° (c=1, Methanol)

**1b) H-Cha-(N,O-dimethylhydroxylamid)**

Zu einer Lösung von 5,5 g (17,5 mmol) Boc-Cha-(N,O-dimethylhydroxylamid) in 50 ml DME gibt man 50 ml HCl-gesättigtes DME und rührt 90 Minuten bei R.T. Das Lösungsmittel wird abgedampft und der Rückstand dreimal mit Toluol abgeraucht. Man erhält 4,35 g der Titelverbindung als Hydrochlorid.

Schmp. 145 - 152 °C.

**1c) Iva-Phe-Nva-Cha-(N,O-dimethylhydroxylamid)**

Zu einer Lösung von 5,9 g (17 mmol) Iva-Phe-Nva-OH, 4,25 g (17 mmol) H-Cha-(N,O-dimethylhydroxylamid)hydrochlorid, 2,75 g (20,4 mmol) HOBt und 4,2 g (20,4 mmol) DCC in 40 ml absolutem DMF werden 6,48 ml (51 mmol) NEM zugegeben und zwei Tage bei R.T. gerührt. Der Niederschlag wird abfiltriet, das Filtrat eingedampft, der Rückstand in EE aufgenommen, mit 10 %iger Citronensäurelösung, gesättigter Natriumbicarbonatlösung und gesättigter Natriumchloridlösung gewaschen, getrocknet, eingeengt und das verbleibende Rohprodukt (10,9 g) durch SC an Kieselgel (Methylenchlorid/EE 1:1) gereinigt. Man erhält 7,0 g der Tietelverbindung.

MS (FAB) = 545 (M + 1).

**1d) Iva-Phe-Nva-Cha-H**

Zu einer Suspension von 60,8 mg (1,59 mmol) LAH in 1,6 ml absolutem THF wird bei -5 °C eine Lösung von 640 mg (1,18 mmol) Iva-Phe-Nva Cha-(N,O-dimethylhydroxylamid) in 3,2 ml absolutem THF getropft; nach 2,5 Stunden bei 0 °C werden erneut 30,5 mg (0,8 mmol) LAH zugegeben, weitere 30 Minuten bei 0 °C gerührt, auf -10 °C abgekühlt, 1,08 ml gesättigte Seignettesalzlösung langsam zugetropft, 30 Minuten nachgerührt und anschließend 2,46 ml 2 N Schwefelsäure zugetropft. Das Reaktionsgemisch wird mit EE überschichtet, 30 Minuten gerührt, vom Niederschlag abdekantiert und der Rückstand zweimal mit EE extrahiert. Die vereinigten organischen Phasen werden mit Wasser, gesättigter Natriumbicarbonatlösung und gesättigter Natriumchloridlösung gewaschen, getrocknet und bei R.T. eingeengt. Man erhält 470 mg der Titelverbindung, die rasch weiter umgesetzt wird.

MS (FAB) = 486 (M + 1)

**1e) N-(4-Methoxybenzyliden)Val-OEt**

Zu einer Lösung von 6,51 g (30 mmol) H-Val-OEt-hydrochlorid und 4,08 g (30 mmol) 4-Methoxybenzaldehyd in 30 ml absolutem Methylenchlorid gibt man 4,2 ml (30 mmol) Triethylamin und rührt über Nacht bei R.T. Die Reaktionslösung wird zweimal mit Wasser ausgeschüttelt, getrocknet und eingeengt. Man erhält 7,0 g der Titelverbindung die noch mit ca. 15 % Aldehyd verunreinigt ist.

MS (EI) = 263 ($M^+$).

**1f) 2-(1S-Ethoxycarbonyl-2-methylpropyl)-3-(4-methoxybenzyl)-oxaziridin**

Zu einer Lösung von 6,9 g (33,8 mmol) MCPBA (85 %) in 40 ml absolutem Methylenchlorid gibt man langsam bei 0 °C 8,9 g (33,8 mmol) N-(4-Methoxybenzyliden)-Val-OEt und rührt 2 Stunden bei 0 °C nach. Man saugt vom Niederschlag ab, wäscht zweimal mit gesättigter Natriumhydrogencarbonatlösung, einmal mit Wasser, trocknet und engt ein. Man erhält 8,9 g der Titelverbindung.

MS (EI) = 279 ($M^+$).

**1g) HO-Val-OEt**

Zu einer Lösung von 8,9 g (32 mmol) 2-(1S-Ethoxycarbonyl-2-methylpropyl)-3-(4-methoxybenzyl)-oxaziridin in 32 ml Ethanol gibt man unter Eiskühlung 2,93 g (43 mmol) Hydroxylamin-hydrochlorid und rührt über Nacht bei R.T. Das Lösungsmittel wird abgedampft, der Rückstand in 40 ml Methylenchlorid gelöst und der verbleibende Niederschlag abgesaugt. Das Filtrat wird eingeengt, der Rückstand in Ether aufgenommen, der nach einiger Zeit ausgefallene Niederschlag abgesaugt und getrocknet. Zur Entfernung von 4-Methoxybenzaldoxim wird erneut in 15 ml EE 2 Stunden bei R.T. gerührt und abgesaugt. Man erhält 2,45 g der Titelverbindung als Hydrochlorid von ausreichender Reinheit für die folgenden Umsetzungen.

Schmp. 127 - 129 °C.

**1h) N-[3-Cyclohexyl-2S(Iva-Phe-Nva-amino)propyliden]-N-(1S-ethoxycarbonyl-2-methyl-1-propyl)-amin-N-oxid**

500 mg (1,03 mmol) Iva-Ihe-Nva-Cha-H werden in 5 ml absolutem DMF gelöst, 222 mg (1,11 mmol) HO-Val-OEt-hydrochlorid zugegeben und 20 Stunden bei R.T. gerührt. Das Lösungsmittel wird abgedampft, der Rückstard in EE aufgenommen, mit gesättigter Natriumhydrogencarbonatlösung, Wasser und gesättigter Natriumchloridlösung gewaschen, getrocknet, eingeengt und das Rohprodukt (402 mg) durch SC an Kieselgel (Methylenchlorid/MeOH 98/2) gereinigt. Man erhält 39 mg der Titelverbindung.

MS (FAB) = 629 (M + 1)

**Beispiel 2**

**N-[3-Cyclohexyl-2S-[N-(isovaleroyl-L-phenylalanyl-L-norvalyl)amino]propyliden]-cyclohexylmethylamin-N-oxid**

**2a) Cyclohexancarbaldoxim**

11,2 g (0,1 mol) frisch destillierter Cyclohexancarbaldehyd werden in 25 ml MeOH gelöst und bei R.T.

13,8 g (0,2 mol) Hydroxylamin-hydrochlorid und 14,35 g (0,175 mol) Natriumacetat, gemeinsam gelöst in 40 ml Wasser, zugegeben. Nach 4½ Stunden bei R.T. wird das abgeschiedene Öl abgetrennt, die wäßrige Phase zweimal mit Ether extrahiert und die vereinigten organischen Phasen mit Wasser und gesättigter Natriumchloridlösung gewaschen, getrocknet und eingeengt. Man erhält 12,3 g der Titelverbindung.
MS (DCI) = 128 (M + 1).

**2b) N-(Cyclohexylmethyl)hydroxylamin**

Zu 7,15 g (56,5 mmol) Cyclohexancarbaldoxim tropft man bei 0 °C innerhalb von 30 Minuten 96 ml (96 mmol) Boranlösung (1M in THF) und rührt anschließend 4 Stunden bei R.T. Das THF wird am Rotationsverdampfer entfernt, der Rückstand auf 0 °C abgekühlt und langsam 40 ml 2N Natronlauge zugetropft. Anschließend wird 1 Stunde zum Rückfluß erhitzt, die wäßrige Phase drei Tage lang kontinuierlich mit n-Pentan extrahiert, der Extrakt eingedampft und das Rohprodukt (6,1 g) durch SC an Kieselgel (Toluol/EtOH 95:5) gereinigt. Man erhält 3,1 g der Titelverbindung.
Schmp. 61 - 63 °C.

**2c) N-[3-Cyclohexyl-2S-Iva-Phe-Nva)amino-propyliden]cyclohexylmethylamin-N-oxid**

Zu einer Lösung von 460 mg (0,95 mmol) Iva-Phe-Nva-Cha-H in 5 nl absolutem DMF gibt man 200 mg Molekularsieb und 122 mg (0,95 mmol) n-(Cyclohexylmethyl)hydroxylamin und rührt 20 Stunden bei R.T. Die Reaktionslösung wird filtriert, das Lösungmittel abgedampft und der Rückstand in EE aufgenommen, wobei ein Niederschlag ausfällt. Dieser wird abgesaugt (103 mg) und durch SC an Kieselgel (Methylenchlorid/MeOH 9:1) gereinigt. Man erhält 94 mg der Titelverbindung.
MS (FAB) = 597 (M + 1).

**Beispiel 3**

**N-[3-Cyclohexyl-2S-[N-(isovaleroyl-L-phenylalanyl-L-norvalyl)amino]propyliden]-methylamin-N-oxid**

Zu einer Lösung von 460 mg (0.95 mmol) Iva-Phe-Nva-Cha-H in 10 ml Ether und 1 ml absolutem DMF gibt man 89 mg (1,9 mmol) N-methylhydroxylamin und 300 mg wasserfreies Calciumchlorid und rührt 20 Stunden bei R.T. Man filtriert ab, engt ein und reinigt das Rohprodukt (265 mg) durch SC an Kieselgel (Toluol/EtOH 95:5). Man erhält 113 mg der Titelverbindung.
MS (FAB) = 515 (M + 1).

**Beispiel 4**

**N-[3-Cyclohexyl-2S-[N-(isovaleroyl-L-phenylalanyl-L-norvalyl)-amino]propyliden]-isopropylamin-N-oxid**

Zu einer Lösung von 438 mg (0.9 mmol) Iva-Phe-Nva-Cha-H in 20 ml absolutem EtOH werden 200 mg (1,8 mmol) N-Isopropylhydroxylamin-hydrochlorid hinzugegeben und 18 Stunden bei R.T. gerührt. Das Lösungsmittel wird abgedampft, der Rückstand zwischen gesättigter Natriumbicarbonatlösung und EE verteilt, die wäßrige Phase noch einmal mit EE extrahiert, die vereinigten organischen Phasen einmal mit Wasser gewaschen, getrocknet, eingeengt und das Rohprodukt (420 mg) an Kieselgel (Methylenchlorid/EE 7:3, dann Toluol/EtOH 9:1) gereinigt. Man erhält 223 mg der Titelverbindung.
MS (FAB) = 543 (M + 1)

**Beispiel 5**

**N-[3-Cyclohexyl-2S-[N-(isovaleroyl-L-phenylalanyl-L-norvalyl)amino]propyliden]-N-(1S-ethoxycarbonyl-2-methyl-1-propyl)amin-N-oxid**

**5a) Boc-Cha-H**

Zu einer Suspension von 3,42 g (0,09 mol) LAH in 60 ml absolutem THF wird bei -5 °C eine Lösung von 21,0 g (0,067 mol) Boc-Cha-(N,O-dimethylhydroxylamid) (Beispiel 1a) in 42 ml absolutem THF zugetropft und 90 Minuten bei 0 °C nachgerührt. Bei dieser Temperatur werden langsam 270 ml halbkonzentrierte Natriumhydrogensulfatlösung zugetropft und dreimal mit Methylenchlorid extrahiert. Die vereinigten organischen Phasen werden mit Wasser gewaschen, getrocknet und bei R.T. eingeengt. Man erhält 16,9 g der Titelverbindung, die rasch weiter umgesetzt wird.
MS (DCI) = 254 (M + 1)

**5b) N-(2S-Boc-amino-3-cyclohexyl-propyliden)-N-(1S-ethoxycarbonyl-2-methyl-1-propyl)amin-N-oxid**

Aus 2,0 g HO-Val-OEt-hydrochlorid (Beispiel 1 g) erhält man durch Lösen in 10 ml Wasser, Sättigen mit festem Natriumhydrogencarbonat, dreimaliges Extrahieren mit Ether, Trocknen und Einengen der vereinigten etherischen Phasen 1,6 g HO-Val-OEt.

Zu einer Lösung von 2,52 g (10 mmol) Boc-Cha-H in 50 ml absolutem Ether bei 0 °C gibt man nacheinander 1,5 g wasserfreies gepulvertes Calciumchlorid und eine Lösung von 1,6 g (10 mmol) HO-Val-OEt in 50 ml absolutem Ether und rührt 35 Stunden bei R.T. Man saugt vom Festkörper ab, engt ein und verreibt den Rückstand mit 50 ml Petrolether. Das Rohprodukt wird abgesaugt, in 20 ml Ether 1 Stunde gerührt, mit 20 ml Petrolether versetzt, erneut abgesaugt und mit wenig kaltem Ether gewaschen. Man erhält 2,55 g der Titelverbindung.

MS (DCI) = 399 (M+1).

**5c) N-(2S-Amino-3-cyclohexyl-propyliden)-N-(1S-ethoxycarbonyl-2-methyl-1-propyl)amin-N-oxid**

100 mg (0,25 mmol) N-(2S-Boc-amino-3-cyclohexylpropyliden)-N-(1S-ethoxycarbonyl-2-methyl-1-propyl) amin-N-oxid und 58,2 µl (0,5 mmol) 2,6-Lutidin werden in 1 ml absolutem Methylenchlorid gelöst, und 68 µl (0,375 mmol) Trimethylsilyltriflat zugespritzt. Nach 100 Minuten Rühren bei R.T. werden weitere 68 µl Trimethylsilyltriflat zugegeben und 10 Minuten nachgerührt, wonach laut DC (Toluol/EtOH 8:2) das gesamte Ausgangsmaterial verbraucht ist. Nun werden 140 µl MeOH zugespritzt, 10 Minuten gerührt, die Reaktionslösung mit Methylenchlorid verdünnt, einmal mit Eiswasser gewaschen, getrocknet, auf ein Volumen von ca. 3 ml eingeengt und sofort weiter umgesetzt.

**5d) N-[3-Cyclohexyl-2S-(Iva-Phe-Nva-amino)propyliden]-N-(1S-ethoxycarbonyl-2-methyl-1-propyl)amin-N-oxid**

174 mg (0,5 mmol) Iva-Phe-Nva-OH, 40,3 µl (0,5 mmol) Pyridin und 69,4 µl (0,5 mmol) N-Ethylpiperidin werden in 14 ml absolutem Methylenchlorid gelöst und bei -15 °C innerhalb von 5 Minuten mit 63,4 µl (0,5 mmol) Pivaloylchlorid versetzt. Anschließend wird 10 Minuten bei R.T. gerührt und bei 0 °C eine frisch hergestellte Lösung von ca. 150 mg (0,5 mmol) N-(2S-Amino-3-cyclohexyl-propyliden)-N-(1S-ethoxycarbonyl-2-methyl-1-propyl)-amin-N-oxid in 10 ml Methylenchlorid zugesetzt. Nach Rühren über Nacht bei R.T. wird das Lösungsmittel abgedampft, der Rückstand in Essigester aufgenommen, je zweimal mit 5 %iger Natriumhydrogensulfatlösung, gesättigter Natriumhydrogencarbonatlösung und gesättigter Natriumchloridlösung gewaschen, getrocknet, eingeengt und das Rohprodukt (167 mg) an Kieselgel (Methylenchlorid/EE 9:1) gereinigt. Man erhält 14 mg der Titelverbindung, die mit dem unter 1h) erhaltenen Produkt identisch ist.

## Beispiel 6

**N-[3-Cyclohexyl-2S-[N-(tert.butyloxycarbonyl-L-phenylalanyl-L-histidyl)amino]propyliden]-N-(1S-ethoxycarbonyl-2-methyl-1-propyl)amin-N-oxid**

**6a) H-His(DNP)-OH**

Zu einer Lösung von 0,42 g (1 mmol) Boc-His(DNP)-OH in 5 ml Dimethoxyethan tropft man bei 0-5 °C 4 ml HCl-gesättigtes Dimethoxyethan und rührt 1 Stunde bei 0-5 °C und 3 Stunden bei Raumtemperatur nach. Die Reaktionslosung wird im Vakuum eingeengt und noch zweimal mit Toluol abgeraucht.

Ausbeute 0,5 g der Titelverbindung als Hydrochlorid.

Rf (Methylenchlorid/MeOH/AcOH/Wasser 70:30:1:1) = 0,16.

**6b) Boc-Phe-His(DNP)-OH**

Zu 0,5 g (1 mmol) H-His(DNP)-OH-hydrochlorid in 12,5 ml einer 0,25 N Natriumhydrogencarbonatlösung gibt man bei Raumtemperatur 0,362 g (1 mmol) Boc-Phe-hydroxysuccinimidester, gelöst in 5 ml Ethanol und 5 ml THF. Das Reaktionsgemisch wird drei Tage bei Raumtemperatur gerührt. Dann setzt man 0,83 g Citronensäure zu, wobei die Titelverbindung ölig ausfällt. Das Produkt wird mit Methylenchlorid extrahiert, die organische Phase über Natriumsulfat getrocknet, eingeengt und der Rückstand mit wenig Essigester versetzt, die Titelverbindung durch Zusatz von Diisopropylether ausgefällt und abgesaugt.

Ausbeute: 0,47 g (83 %).

Rf (Methylenchlorid/MeOH 7:3) = 0,43; MS (FAB) = 569 (M+1).

**6c) N-[3-Cyclohexyl-2S-(Boc-Phe-His(DNF)-amino)-propyliden]-N-(1S-ethoxycarbonyl-2-methyl-1-propyl)-amin-N-oxid**

Ausgehend von Boc-Phe-His(DNP)-OH und N-(2S-Amino-3-cyclohexylpropyliden)-N-(1S-ethoxycarbonyl-2-methyl-1-propyl)amin-N-oxid erhält man analog zu dem in Beispiel 5d) beschriebenen Verfahren die Titelverbindung.

MS (FAB) = 849 (M + 1).

**6d) N-[3-Cyclohexyl-2S-(Boc-Phe-His-amino)propyliden]-N-(1S-ethoxycarbonyl-2-methyl-2-propyl)amin-N-oxid**

53 mg (0,062 mmol) DNP-geschütztes Produkt aus Beispiel 6c) werden in 5 ml Actonitril mit 0,1 ml (1 mmol) Thiophenol versetzt und 5 Stunden bei R.T. gerührt. Die Reaktionslösung wird im Vakuum eingeengt und das Rohprodukt durch SC an Kieselgel (Methylenchlorid/MeOH 98:2, 95:5, 9:1) gereinigt. Man erhält 12 mg der Titelverbindung.

MS (FAB) = 683 (M + 1).

**Beispiel 7**

**N-[3-Cyclohexyl-2S-[N-(ethylaminocarbonyl-L-phenylalanyl L-norvalyl)amino]propyliden]-cyclohexylmethylamin-N-oxid**

**7a) N-(Ethylaminocarbonyl)-Phe-OH**

3,37 g (10 mmol) Phe-p-toluolsulfonat werden in 10 ml absolutem Tetrahydrofuran gelöst, bei Raumtemperatur 2,4 ml (12 mmol) frisch destilliertes Hexamethyldisilazan hinzugegeben und zwei Stunden nachgerührt. Zu der Suspension gibt man 0,97 ml (12 mmol) Ethylisocyanat und läßt über Nacht bei Raumtemperatur stehen. Man saugt von Niederschlag ab, kühlt das Filtrat im Eisbad ab, filtriert erneut, engt ein und verrührt den Rückstand mit Wasser. Der Niederschlag wird abgesaugt und über Phosphorpentoxid getrocknet.

Ausbeute 1,7 g (72 %).

Rf (Methylenchlorid/Methanol/Wasser/Eisessig 70:30:1:1) = 0,68; MS (DCI) = 237 (M + 1).

**7b) N-(Ethylcarbonylamino)-Phe-Nva-OMe**

Zu 1,2 g (5,08 mmol) N-(Ethylaminocarbonyl)-Phe-OH und 0,85 g (5,08 mmol) L-Nva-OMe-hydrochlorid in 30 ml absolutem Methylenchlorid gibt man unter Eiskühlung nacheinander 3,51 ml (25,4 mmol) absolutes Triethylamin und 3,30 ml Propanphosphonsäureanhydrid (50 % in Methylenchlorid), rührt 6 Stunden bei Raumtemperatur nach und läßt über Nacht stehen. Das Reaktionsgemisch wird zur Hydrolyse auf Eiswasser gegeben, die organische Phase abgetrennt und je dreimal mit jeweils 100 ml 10%-iger Citronensäurelösung, gesättigter Natriumhydrogencarbonatlösung und Wasser gewaschen, über Natriumsulfat getrocknet und eingeengt. Der Rückstand wird in wenig kaltem Diisopropylether verrührt, abgesaugt und im Vakuum getrocknet.

Ausbeute: 1,5 g (84 %).

Rf (Methylenchlorid/Methanol 9:1) = 0,49.

**7c) N-(Ethylaminocarbonyl)-Phe-Nva-OH)**

Zu 1,5 g (4,29 mmol) N-(Ethylaminocarbonyl)-Phe-Nva-OMe aus Beispiel Ib) in 8 ml Wasser und 8 ml Dioxan gibt man bei Raumtempertaur 0,2 g Lithiumhydroxid und rührt zwei Stunden nach. Die Reaktionslösung wird mit 10 %-iger Natriumhydrogensulfatlösung sauer gestellt, der Niederschlag abgesaugt, mit Diisopropylether verrührt und getrocknet.

Ausbeute: 1,3 g.

Rf (Toluol/Ethanol 8:2) = 0,02; MS (DCI) = 336 (M + 1).

**7d) N-(Ethylaminocarbonyl)-Phe-Nva-Cha-(N,O-dimethylhydroxylamid)**

Ausgehend von N-Ethylaminocarbonyl-Phe-Nva-OH und H-Cha-(N,O-dimethylhydroxylamid) erhält man analog dem in Beispiel 1c) beschriebenen Verfahren die Titelverbindung.

MS (FAB) = 566 (M + 1).

**7e) N-Ethylaminocarbonyl-Phe-Nva-Cha-H**

Durch Reduktion von N-Ethylaminocarbonyl-Phe-Nva-Cha-(N,O-dimethylhydroxylamid) mit LAH erhält man analog dem in Beispiel 1d) beschriebenen Verfahren die Titelverbindung.

MS (FAB) = 507 (M + 1).

**7f) N-[3-Cyclohexyl-2S-[N-(ethylaminocarbonyl-Phe-Nva)amino]propyliden]-cyclohexylmethylamin-N-oxid**

Ausgehend von N-Ethylaminocarbonyl-Phe-Nva-Cha-H und N-Cyclohexylmethylhydroxylamin erhält man analog dem in Beispiel 2c) beschriebenen Verfahren die Titelverbindung.

MS (FAB) = 618 (M + 1).

Ausgehend von den entsprechenden Ausgangsmaterialien und unter Anwendung der in den voranstehenden Beispielen beschriebenen Verfahren wurden außerdem folgende erfindungsgemäße Verbindungen hergestellt.

**Beispiel 8**

N-[3-Cyclohexyl-2S-[N-[N-(2S-benzyl-3-tert.butylsulfonylpropionyl)-L-histidyl]amino]-propyliden]-cyclohexylmethyl amin-N-oxid

MS (FAB) = 670 (M+1).

**Beispiel 9**

N-[3-Cyclohexyl-2S-[N-(morpholinocarbonyl-L-phenylalanyl-L-histidyl)amino]propyliden]cyclohexylmethyl-amin-N-oxid

MS (FAB) = 664 (M+1).

**Beispiel 10**

N-[3-Cyclohexyl-2S-[N-(tert.butylaminothiocarbonyl-L-phenylalanyl-L-norvalyl)amino]propyliden]-cyclohexylmethylamin-N-oxid

MS (FAB) = 628 (M+1).

**Beispiel 11**

N-[2S-[N-(Isovaleroyl-L-phenylalanyl-L-norvalyl)amino]-4-methylpentyliden]-cyclohexylmethylamin-N-oxid

MS (FAB) = 557 (M+1).

**Beispiel 12**

N-[2S-[N-(Isovaleroyl-L-phenylalanyl-L-norvalyl)-amino]-3-phenylpropyliden]-cyclohexylmethylamin-N-oxid

MS (FAB) = 591 (M+1).

**Beispiel 13**

N-[3-Cyclohexyl-2S-[N-(isovaleroyl-L-phenylalanyl-L-norvalyl)amino]-propyliden]-isobutylamin-N-oxid

MS (FAB) = 557 (M+1).

**Beispiel 14**

N-[3-Cyclohexyl-2S-[N-[N-[2S-(2-thienylmethyl)-3-tert.butylsulfonyl-propionyl]-L-norvalyl]amino]-propyliden]-cyclohexylmethylamin-N-oxid

MS (FAB) = 638 (M+1).

**Beispiel 15**

N-[3-Cyclohexyl-2RS[N-(isovaleroyl-L-phenylalanyl-L-norvalyl)amino]propyliden]-N-(1S-ethoxycarbonyl-2-methyl-1-propyl)amin-N-oxid

MS (FAB) = 629 (M+1).

**Beispiel 16**

N-[3-Cyclohexyl-2RS-[N-(isovaleroyl-L-phenylalanyl-L-norvalyl)amino]propyliden]-N-cyclohexylmethylamin-N-oxid

MS (FAB) = 597 (M + 1).

**Beispiel 17**

N-[3-Cyclohexyl-2RS-[isovaleroyl-L-phenylalanyl-L-norvalyl)amino]propyliden]-methylamin-N-oxid

MS (FAB) = 515 (M + 1).

**Beispiel 18**

N-[3-Cyclohexyl-2RS-[N-(isovaleroyl-L-phenylalanyl-L-norvalyl)amino]propyliden]-isopropylamin-N-oxid

MS (FAB) = 543 (M + 1).

**Beispiel 19**

N-[3-Cyclohexyl-2S-[-N-(isovaleroyl-L-phenylalanyl-L-histidyl)amino]-propyliden]-isobutylamin-N-oxid

MS (FAB) = 595 (M + 1).

**Beispiel 20**

N-[3-Cyclohexyl-2S-[N-[N-(2S-benzyl-3-tert.butylsulfonylpropionyl)-L-histidyl]amino]propyliden]isobutylamin-N-oxid

MS (FAB) = 630 (M + 1).

**Beispiel 21**

N-[3-Cyclohexyl-2S-[N-(isovaleroyl-L-phenylalanyl-L-histidyl)amino]propyliden]-N-[2-(2-pyridyl)ethyl]-amin-N-oxid

MS (FAB) = 644 (M + 1).

## Patentansprüche

**Patentansprüche für folgende Vertragsstaaten : AT, BE, CH, DE, FR, GB, IT, LI, LU, NL, SE**

1. Verbindungen der allgemeinen Formel I,

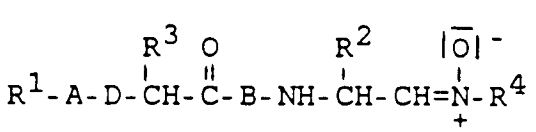

(I)

in welcher

$R^1$ Wasserstoff, $(C_1-C_{18})$-Alkyl, $(C_3-C_7)$-Cycloalkyl, die jeweils durch Amino, Hydroxy, Mercapto, Halogen, $(C_1-C_4)$-Alkoxy, Mono- oder Di-$(C_1-C_4)$-alkylamino, Carboxy, $(C_1-C_4)$-Alkoxycarbonyl, Phenoxy, Phenyl-$(C_1-C_4)$-alkoxy, Phenyl-$(C_1-C_4)$-alkoxycarbonyl oder einen Rest $-CONR^5R^6$ substituiert sein können; $(C_1-C_4)$-Alkoxy, $(C_6-C_{14})$-Aryl, $(C_6-C_{14})$-Aryl-$(C_1-C_4)$alkyl, $(C_6-C_{14})$-Aryl-$(C_1-C_4)$-alkoxy, wobei der Arylrest jeweils durch ein, zwei oder drei Reste der Gruppe $(C_1-C_6)$-Alkyl, Amino, Mono- oder Di-$(C_1-C_4)$-alkylamino, Amino-$(C_1-C_4)$-alkyl, Hydroxy-$(C_1-C_4)$-alkyl, Mono- oder Di-$(C_1-C_4)$-

alkylamino-($C_1$-$C_4$)-alkyl, Hydroxy, ($C_1$-$C_4$)-Alkoxy, Halogen, Formyl, ($C_1$-$C_4$)-Alkoxycarbonyl, Carboxamido, Mono- oder Di-($C_1$-$C_4$)-alkylaminocarbonyl oder Nitro oder durch einen Methyldioxyrest substituiert sein kann; Het oder Het-($C_1$-$C_4$)-alkyl, wobei Het einen 5-, 6- oder 7-gliedrigen heterocyclischen Ring darstellt. der benzanneliert und entweder aromatisch, teilhydriert oder vollständig hydriert sein kann und der als Heteroelemente ein oder zwei Reste der Gruppe N, O, S, NO, SO oder $SO_2$ enthalten und durch ein oder zwei Reste der Gruppe ($C_1$-$C_4$)-Alkyl, ($C_1$-$C_4$)-Alkoxy, ($C_1$-$C_4$)-Alkoxycarbonyl, Hydroxy, Halogen, Amino, Mono- oder Di-($C_1$-$C_4$)-alkylamino substituiert sein kann oder einen Rest $-NR^5R^6$ bedeutet, wobei

$R^5$ und $R^6$ gleich oder verschieden und voneinander unabhängig Wasserstoff, ($C_1$-$C_8$)-Alkyl, das durch Amino, ($C_1$-$C_4$)-Alkylamino, Di-($C_1$-$C_4$)-alkylamino, Hydroxy oder ($C_1$-$C_4$)-Alkoxy substituiert sein kann, ($C_3$-$C_7$)-Cycloalkyl, Mercapto, ($C_1$-$C_4$)-Alkylthio, Phenylthio, ($C_1$-$C_4$)-Alkoxycarbonyl, Carboxy, ($C_6$-$C_{14}$)-Aryl, das im Arylrest wie bei $R^1$ beschrieben substituiert sein kann, Het oder Het-($C_1$-$C_4$)-alkyl, wobei Het wie bei $R^1$ beschrieben definiert ist, bedeutet oder wobei

$R^5$ und $R^6$ zusammen mit dem sie tragenden Stickstoffatom einen 5- bis 12-gliedrigen Ring bilden, der mono-oder bicyclisch sein kann und als weitere Ringglieder noch 1 oder 2 Stickstoffatome, 1 Schwefelatom oder 1 Sauerstoffatom enthalten und durch ($C_1$-$C_4$)-Alkyl substituiert sein kann, bilden;

A einen Rest der Gruppe S, SO, $SO_2$, O, CO, CS oder eine direkte Bindung bedeutet;

D eine $CH_2$-Gruppe oder einen Rest $-NR^7-$, wobei $R^7$ Wasserstoff oder ein ($C_1$-$C_4$)-Alkylrest sein kann, bedeutet;

$R^3$ ($C_6$-$C_{14}$)-Aryl, ($C_6$-$C_{14}$)-Aryl-($C_1$-$C_4$)-alkyl, wobei der Arylrest jeweils durch ein, zwei oder drei Reste wie unter $R^1$ beschrieben, substituiert sein kann, Thienyl oder Thienyl-($C_1$-$C_4$)-alkyl, wobei der Thiophenrest jeweils durch ein oder zwei Reste der Gruppe ($C_1$-$C_4$)-Alkyl, ($C_1$-$C_4$)-Alkoxy oder Halogen substituiert sein kann, 2-, 3- oder 4-Pyridyl oder 2-, 3- oder 4-Pyridyl($C_1$-$C_4$)-alkyl, wobei der Pyridinrest durch ein oder zwei Reste der Gruppe ($C_1$-$C_4$)-Alkyl, ($C_1$-$C_4$)-Alkoxy oder Halogen substituiert sein kann, bedeutet;

B einen N-terminal über $-NR^8-$ mit $R^1$-A-D-$CHR^3$-CO- und C-terminal über -CO- mit

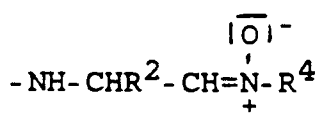

verknüpften Rest einer Aminosäure aus der Reihe Phenylalanin, Histidin, Tyrosin, Tryptophan, Methionin, Leucin, Isoleucin, Asparagin, Asparaginsäure, $\beta$-2-Thienylalanin, $\beta$-3-Thienylalanin, $\beta$-2-Furylalanin, $\beta$-3-Furylalanin, Lysin, Ornithin, Valin, Alanin, 2,4-Diaminobuttersäure, Arginin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, Norvalin, $\beta$-2-Benzo[b]thienylalanin, $\beta$-3-Benzo[b]thienylalanin, 2-Fluorphenylalanin, 3-Fluorphenylalanin, 4-Fluorphenylalanin, Norleucin, Cystein, S-Methyl-Cystein, 1,2,3,4-Tetrahydroisochinolin-3-carbonsäure, Homophenylalanin, DOPA, O-Dimethyl-DOPA, 2-Amino-4-(2-thienyl)-buttersäure, Benzodioxol-5-yl-alanin, N-Methyl-histidin, 2-Amino-4-(3-thienyl)-buttersäure, 3-(2-Thienyl)-serin, (Z)-Dehydrophenylalanin, (E)-Dehydrophenylalanin, (1,3-Dioxolan-2-yl)-alanin, N-Pyrrolylalanin, (1-, 3-oder 4-Pyrazolyl)alanin, (4-Thiazolyl)alanin, (2-, 4- oder 5-Pyrimidyl)alanin, Cyclopentylglycin, tert.Butylglycin oder Phenylserin, bedeutet und $R^8$ Wasserstoff, ($C_1$-$C_6$)-Alkyl, Formyl, ($C_1$-$C_6$)-Alkoxycarbonyl oder Benzyloxycarbonyl bedeutet:

$R^2$ Wasserstoff, ($C_1$-$C_{10}$)-Alkyl, ($C_4$-$C_7$)-cycloalkyl, ($C_4$-$C_7$)-Cycloalkyl-($C_1$-$C_4$)-alkyl, ($C_6$-$C_{14}$)-Aryl, ($C_6$-$C_{14}$)-Aryl-($C_1$-$C_4$)-alkyl oder (Heterocyclyl)-($C_1$-$C_4$)-alkyl bedeutet, wobei der Heterocyclus 4-7 Ringglieder, davon 1 oder 2 Schwefel- und/oder Sauerstoffatome besitzt, bedeutet; und

$R^4$ ($C_1$-$C_8$)-Alkyl, ($C_3$-$C_8$)-Cycloalkyl, ($C_3$-$C_8$)-Cycloalkyl-($C_1$-$C_4$)-alkyl, die jeweils durch Amino, Hydroxy, Mercapto, Halogen, ($C_1$-$C_4$) Alkoxy, Mono- oder Di-($C_1$-$C_4$)-alkylami-

no, Carboxy, Guanidino, ($C_1$-$C_4$)-Alkoxycarbonyl oder einen Rest -CONR$^5$R$^6$, worin R$^5$ und R$^6$ wie oben definiert sind, einfach, zweifach oder dreifach substituiert sein können; ($C_6$-$C_{14}$)-Aryl, ($C_6$-$C_{14}$)-Aryl($C_1$-$C_4$)-alkyl, wobei der Arylrest wie unter R$^1$ beschrieben und die Alkylkette wie oben für R$^4$ beschrieben, substituiert sein kann; Het oder Het-($C_1$-$C_4$)-alkyl, wobei Het wie unter R$^1$ beschrieben, definiert ist und die Alkylkette wie oben für R$^4$ beschrieben, substituiert sein kann;

sowie deren physiologisch verträgliche Salze.

**2.** Verbindung der Formel I, gemäß Anspruch 1, dadurch gekennzeichnet, daß

R$^1$ methyl, Ethyl, Isopropyl, tert.-Butyl, Isobutyl, 2-Hydroxyethyl, 2-Methoxyethyl, Carboxymethyl, 2-Carboxyethyl, Methoxycarbonylmethyl, 2-Methoxycarbonylethyl, Ethoxycarbonylmethyl, 2-Ethoxycarbonylethyl, Carbamoylmethyl, 2-Carbamoylethyl, 2-Aminoethyl, 2-Dimethylaminoethyl, 2-Morpholinoethyl, Aminoisobutyl, 2-Piperidinoethyl, Aminopropyl, Dimethylaminopropyl, Methylaminopropyl, Piperidinopropyl, Morpholinopropyl, Methylaminoisobutyl, Dimethylaminoisobutyl, Piperidinoisobutyl, Morpholinoisobutyl, Cyclopropyl, Cyclobutyl, Cyclopentyl oder Cyclohexyl, Phenyl, 1- oder 2-Naphthyl, o-, m- oder p-Methylphenyl, o-, m- oder p-Hydroxyphenyl oder o-, m-oder p-Aminophenyl, Benzyl, 2-Phenylethyl oder $\alpha$- oder $\beta$-Naphthylmethyl, unsubstituiertes oder substituiertes Heteroaryl, z.B. 2- oder 3-Pyrrolyl, 2-Furyl, 2-Thienyl, 2- oder 4-Imidazolyl, 1-Methyl-2-, -4- oder -5-Imidazolyl, 1,3-Thiazol-2-yl, 2-, 3- oder 4-Pyridyl, 1-Oxido-2-, 3- oder 4-pyridinio, 2-Pyrazinyl, 2-, 4- oder 5-Pyrimidinyl, 2-, 3- oder 4-Chinolyl, 1-, 3- oder 4-Isochinolyl oder 2-Benzoxazolyl, Methoxy, Ethoxy oder n-Butoxy oder einen Rest -NR$^5$R$^6$ bedeutet, worin

R$^5$ und R$^6$ gleich oder verschieden und voneinander unabhängig Wasserstoff, Methyl, Ethyl, Propyl, Butyl, Isobutyl, sec.-Butyl, tert.-Butyl, Aminoethyl, Aminopropyl, Methylaminoethyl, Dimethylaminoethyl, Methylaminopropyl, Dimethylaminopropyl, Hydroxyethyl, Methoxyethyl, Cyclohexylmethyl, Mercaptoethyl, Methylthioethyl, Benzyl, 1-Phenethyl, 2-Phenethyl, 2-(3,4-Dimethoxy)-phenethyl, 2-Pyridylmethyl, 3-Pyridylmethyl bedeuten oder worin

R$^5$ und R$^6$ zusammen mit dem sie tragenden Stickstoffatom einen Pyrrolidin-, Piperidin-, Azepin, Azocin-, Morpholin-, Piperazin-, 4-Methyl-piperazin-, 4-Ethyl-piperazin-, Homopiperazin- oder Thiomorpholin-Ring bilden;

A einen Rest der Gruppe S, SO, $SO_2$, O, CO oder CS bedeutet;

D eine $CH_2$-Gruppe, eine NH-Gruppe oder eine N($CH_3$)-Gruppe bedeutet;

R$^3$ Phenyl, 2-Thienyl, 2-Pyridyl, 1-Naphthyl, Phenyl-($C_1$-$C_4$)-alkyl, 2-Thienyl-($C_1$-$C_4$)-alkyl, 2-Pyridyl-($C_1$-$C_4$)-alkyl, 1-Naphthyl-($C_1$-$C_4$)-alkyl, die jeweils gegebenenfalls durch einen, zwei oder drei Reste aus der Gruppe Methyl, Ethyl, Isopropyl, tert.-Butyl, Methoxy, Hydroxy, Fluor, Chlor oder Nitro oder einen Methylendioxyrest substituiert sind, bedeutet;

B einen N-terminal über -NH- mit R$^1$-A-D-CHR$^3$-CO- und C-terminal über -CO- mit

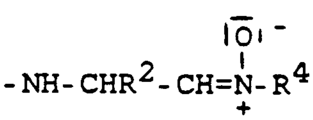

verknüpften Rest einer Aminosäure aus der Reihe Phenylalanin, Histidin, Tyrosin, Tryptophan, Methionin, Leucin, Isoleucin, Asparagin, Asparaginsäure, $\beta$-2-Thienylalanin, $\beta$-3-Thienylalanin, $\beta$-2-Furylalanin, $\beta$-3-Furylalanin, Lysin, Ornithin, 2,4-Diaminobuttersäure, Arginin, Norvalin, 4-chlorphenylalanin, Methioninsulfon, Methioninsulfoxid, 2-pyridylalanin. 3-pyridylalanin, Cyclohexylalanin, Cyclohexylglycin, im-Methylhistidin, O-methyltyrosin, O-Benzyltyrosin, O-tert.-Butyltyrosin, Phenylglycin, 1-Naphthylalanin, 2-Naphthylalanin, 4-Nitrophenylalanin, Norleucin, Valin, Alanin, Cystein, S-Methylcystein, N-Methyl-Histidin, Benzodioxol-5-yl-alanin, 1,2,3,4-Tetrahydroisochinolin-3-carbonsäure, Homophenylalanin, 2-Amino-4-(3-thienyl)-buttersäure, (Z)-Dehydrophenylalanin, (E)-Dehydrophenylalanin, (1,3-Dioxolan-2-yl)alanin, (4-Thiazolyl)alanin, (2-, 4- oder 5-Pyrimidyl)alanin, (1-,3- oder 4-Pyrazolyl)alanin, (2-,3- oder 4-Fluorphenyl)alanin, Cyclopentylglycin, tert.-Butylglycin oder Phenylserin be-

deutet;

$R^2$ wie in Anspruch 1 definiert ist, und

$R^4$ Methyl, Ethyl, Propyl, Isopropyl, Butyl, Isobutyl, sec.-Butyl, tert.-Butyl, Pentyl, Isopentyl, Neopentyl, sec.-Pentyl, tert.-Pentyl, Hexyl, Cyclopentyl, Cyclohexyl, Cycloheptyl, Cyclopentylmethyl, Cyclohexylmethyl, Cycloheptylmethyl, Phenyl, Benzyl, Phenylethyl, Phenylpropyl, 2-, 3- oder 4-Pyridyl, 2-, 4- oder 5-Imidazolyl, (2-, 3- oder 4-Pyridyl)-methyl, (2-, 4- oder 5-Imidazolyl)methyl, wobei die genannten Alkyl-und Cycloalkylreste jeweils durch Amino, Halogen, Mono-oder Di-($C_1$-$C_4$)-alkylamino, Carboxy, Guanidino, ($C_1$-$C_4$)-Alkoxycarbonyl oder einen Rest -CON$R^5R^6$-, worin $R^5$ und $R^6$ wie oben definiert sind, einfach oder zweifach substituiert sein können; die genannten Phenylreste einfach oder zweifach durch ($C_1$-$C_4$)-Alkyl, Methoxy, Hydroxy, Amino, Aminomethyl, Fluor, Chlor oder Trifluormethyl substituiert sein können und die genannten heteroaromatischen Reste jeweils durch ein oder zwei Reste aus der Reihe ($C_1$-$C_4$)-Alkyl, Methoxy, Fluor, Chlor, Brom oder Trifluormethyl substituiert sein können, bedeutet

sowie deren physiologisch verträgliche Salze.

**3.** Verbindung der Formel I, gemäß einem oder mehreren der Ansprüche 1 bis 2, dadurch gekennzeichnet, daß

$R^1$ Methyl, Ethyl, Isopropyl, tert.-Butyl, Isobutyl, 2-Hydroxyethyl, 2-Methoxyethyl, Carboxymethyl, 2-Carboxyethyl, Methoxycarbonylmethyl, 2-Methoxycarbonylethyl, Ethoxycarbonylmethyl, 2-Ethoxycarbonylethyl, Carbamoylmethyl, 2-Carbamoylethyl, 2 Aminoethyl, 2-Dimethylaminoethyl, 2-Morpholinoethyl, Aminopropyl, Aminoisobutyl, Methylaminosobutyl, Dimethylaminoisobutyl, 2-Piperidinoethyl, Cyclopropyl, Cyclobutyl, Cyclopentyl oder Cyclohexyl, Phenyl, 1- oder 2-Naphthyl, o-, m- oder p-Methylphenyl, o-, m- oder p-Hydroxyphenyl oder o-, m- oder p-Aminophenyl, Benzyl, 2-Phenylethyl oder $\alpha$- oder $\beta$-Naphthylmethyl, unsubstituiertes oder substituiertes Heteroaryl, z.B. 2- oder 3-Pyrrolyl, 2-Furyl, 2-Thienyl, 2- oder 4-Imidazolyl, 1-Methyl-2-, -4- oder -5-Imidazolyl, 1,3-Thiazol-2-yl, 2-, 3- oder 4-Pyridyl, 1-Oxido-2-, 3- oder 4-pyridinio, 2-Pyrazinyl, 2-, 4- oder 5-Pyrimidinyl, 2-, 3- oder 4-Chinolyl, 1-, 3- oder 4-Isochinolyl oder 2-Benzoxazolyl, Methoxy, Ethoxy oder n-Butoxy oder einen Rest -N$R^5R^6$ bedeuten, worin

$R^5$ und $R^6$ gleich oder verschieden und voneinander unabhängig Wasserstoff, Methyl, Ethyl, Propyl, Butyl, Isobutyl, sec.-Butyl, tert.-Butyl, Hydroxyethyl, Methoxyethyl, Aminoethyl, Aminopropyl, Benzyl- oder Pyridylmethyl bedeuten oder worin

$R^5$ und $R^6$ zusammen mit dem sie tragenden Stickstoffatom einen Pyrrolidin-, Piperidin-, Morpholin- oder Piperazinring bilden;

A einen Rest der Gruppe S, SO, $SO_2$, O, CO oder CS bedeutet;

D eine $CH_2$-Gruppe, eine NH-Gruppe oder eine N($CH_3$)-Gruppe bedeutet;

$R^3$ Aryl oder Arylmethyl bedeutet, wobei Aryl Phenyl, 2-Thienyl, 2-Pyridyl, 1-Naphthyl bedeutet, die jeweils gegebenenfalls durch Hydroxy, Dihydroxy, Methoxy, Dimethoxy, Fluor, Chlor oder Methylendioxy substituiert sind;

B einen N-terminal über -NH mit $R^1$-A-D-CH$R^3$-CO- und C-terminal über -CO- mit

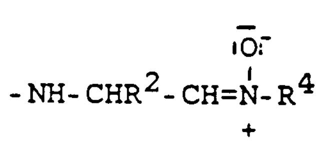

verknüpften Rest einer Aminosäure aus der Reihe Phenylalanin, Histidin, Leucin, $\beta$-2-Thienylalanin, $\beta$-3-Thienylalanin, Lysin, Norvalin, 2-Fluorphenylalanin, 3-Fluorphenylalanin, 4-Fluorphenylalanin, Norleucin, S-Methylcystein, (1,3-Dioxolan-2-yl)alanin, (1-,3- oder 4-Pyrazolyl)alanin, 4-Thiazolylalanin, (2-,4- oder 5-Pyrimidyl)alanin, bedeutet;

$R^2$ Isobutyl, Cyclohexylmethyl, Benzyl oder (1,3-Dithiolan-2-yl)methyl bedeutet; und

$R^4$ Methyl, Ethyl, Propyl, Isopropyl, Butyl, Isobutyl, sec.-Butyl, tert. Butyl, Pentyl, Isopentyl, Neopentyl, sec.-Pentyl, tert. Pentyl, Hexyl, Cyclopentyl, Cyclohexyl, Cyclopentylmethyl, Cyclohexylmethyl, Phenyl, Benzyl, Phenylethyl, 2-,3- oder 4-Pyridyl, 2-Imidazolyl, (2-,3- oder 4-Pyridyl)methyl, 2-Imidazolylmethyl, wobei die genannten Alkyl- und Cycloalkylreste jeweils durch Amino, Fluor, Chlor, Methylamino, Ethylamino, Dimethylamino, Diethylamino, Carboxy, Guanidino, Methoxycarbonyl, Ethoxycarbonyl, tert. Butyloxycarbonyl, oder einen Rest -CON$R^5R^6$, worin $R^5$ und $R^6$ wie oben definiert sind, einfach oder zweifach, substituiert sein können, die genannten Phenylreste ein- oder zweifach durch Methyl, Methoxy, Hydroxy, Amino, Aminomethyl, Fluor, Chlor oder Trifluormethyl substituiert sein können und die genannten heteroaromatischen Reste mit jeweils ein oder zwei Resten aus der Reihe Methyl, Methoxy, Fluor, Chlor oder Trifluormethyl substituiert sein können, bedeutet, sowie deren physiologisch vertäglichen Salze.

**4.** Verfahren zur Herstellung einer Verbindung der Formel I, gemäß einem oder mehreren der Ansprüche 1 bis 3, dadurch gekennzeichnet, daß man

a) eine Verbindung der allgemeinen Formel II

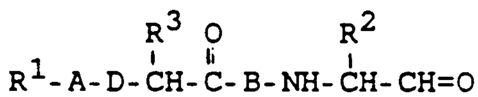

(II)

in welcher $R^1$, $R^2$, $R^3$, A, B und D die gleiche Bedeutung wie in Formel I besitzen, mit einem Hydroxylaminderivat der allgemeinen Formel III

HO-NH-$R^4$ (III)

worin $R^4$ die gleiche Bedeutung wie in Formel I besitzt, in einem organischen Lösungsmittel wie z.B. Diethylether, Ethanol Benzol, Toluol oder in Wasser oder ohne jedes Lösungsmittel bei einer Temperatur zwischen -20 °C und dem Siedepunkt des Lösungsmittels, vorzugsweise zwischen -20 °C und +60 °C, mit oder ohne Gegenwart einer Säure, z.B. Chlorwasserstoff oder Essigsäure, die z.B. in Form der Salze der Hydroxylamine der Formel III eingebracht werden kann, mit oder ohne Gegenwart einer Hilfsbase wie z.B. Natriumhydrogencarbonat, Natriumacetat, Natriumcarbonat, Natriumethanolat oder Kaliumhydroxid sowie mit oder ohne Gegenwart eines wasserentziehenden Mittels wie z.B. Calciumchlorid oder Molekularsieb, umsetzt und gegebenenfalls zum Schutz weiterer funktioneller Gruppen (eine) temporär eingeführte Schutzgruppe(n) abspaltet

oder

b) ein Fragment mit endständiger Carboxylgruppe oder dessen reaktives Derivat mit einem entsprechenden Fragment mit freier Aminogruppe kuppelt, gegebenenfalls zum Schutz weiterer funktioneller Gruppen (eine) temporär eingeführte Schutzgruppe(n) abspaltet und die so erhaltene Verbindung gegebenenfalls in ihr physiologisch verträgliches Salz überführt.

**5.** Verbindung der Formel I gemäß einem oder mehreren der Ansprüche 1 bis 3 zur Anwendung als Heilmittel.

**6.** Verbindung der Formel I gemäß einem oder mehreren der Ansprüche 1 bis 3 zur Anwendung als Heilmittel bei der Behandlung des Bluthochdrucks.

**7.** Verbindung der Formel I gemäß einem oder mehreren der Ansprüche 1 bis 3 zur Anwendung als Heilmittel bei der Behandlung von Viruserkrankungen.

**8.** Pharmazeutische Zübereitung enthaltend eine Verbindung der Formel I gemäß einem oder mehreren der Ansprüche 1 bis 3.

**Patentansprüche für folgende Vertragsstaaten : ES, GR**

1. Verfahren zur Herstellung einer Verbindung der Formel I

$$R^1\text{-}A\text{-}D\text{-}\underset{\displaystyle |}{\overset{R^3}{C}}H\text{-}\overset{\overset{\displaystyle O}{\|}}{C}\text{-}B\text{-}NH\text{-}\overset{R^2}{C}H\text{-}CH{=}\underset{+}{\overset{\overline{|O|}^{-}}{N}}\text{-}R^4 \qquad (I)$$

in welcher

| | |
|---|---|
| $R^1$ | Wasserstoff, $(C_1\text{-}C_{18})$-Alkyl, $(C_3\text{-}C_7)$-Cycloalkyl, die jeweils durch Amino, Hydroxy, Mercapto, Halogen, $(C_1\text{-}C_4)$-Alkoxy, Mono- oder Di-$(C_1\text{-}C_4)$-alkylamino, Carboxy, $(C_1\text{-}C_4)$-Alkoxycarbonyl, Phenoxy, Phenyl-$(C_1\text{-}C_4)$-alkoxy, Phenyl-$(C_1\text{-}C_4)$-alkoxycarbonyl oder einen Rest $-CONR^5R^6$ substituiert sein können; $(C_1\text{-}C_4)$-Alkoxy, $(C_6\text{-}C_{14})$-Aryl, $(C_6\text{-}C_{14})$-Aryl-$(C_1\text{-}C_4)$alkyl, $(C_6\text{-}C_{14})$-Aryl-$(C_1\text{-}C_4)$-alkoxy, wobei der Arylrest jeweils durch ein, zwei oder drei Reste der Gruppe $(C_1\text{-}C_6)$-Alkyl, Amino, Mono- oder Di-$(C_1\text{-}C_4)$-alkylamino, Amino-$(C_1\text{-}C_4)$-alkyl, Hydroxy-$(C_1\text{-}C_4)$-alkyl, Mono- oder Di-$(C_1\text{-}C_4)$-alkylamino-$(C_1\text{-}C_4)$-alkyl, Hydroxy, $(C_1\text{-}C_4)$-Alkoxy, Halogen, Formyl, $(C_1\text{-}C_4)$-Alkoxycarbonyl, Carboxamido, Mono- oder Di-$(C_1\text{-}C_4)$-alkylaminocarbonyl oder Nitro oder durch einen Methyldioxyrest substituiert sein kann; Het oder Het-$(C_1\text{-}C_4)$-alkyl, wobei Het einen 5-, 6- oder 7-gliedrigen heterocyclischen Ring darstellt, der benzanneliert und entweder aromatisch, teilhydriert oder vollständig hydriert sein kann und der als Heteroelemente ein oder zwei Reste der Gruppe N, O, S, NO, SO oder $SO_2$ enthalten und durch ein oder zwei Reste der Gruppe $(C_1\text{-}C_4)$-Alkyl, $(C_1\text{-}C_4)$-Alkoxy, $(C_1\text{-}C_4)$-Alkoxycarbonyl, Hydroxy, Halogen, Amino, Mono- oder Di-$(C_1\text{-}C_4)$-alkylamino substituiert sein kann oder einen Rest $-NR^5R^6$ bedeutet, wobei |
| $R^5$ und $R^6$ | gleich oder verschieden und voneinander unabhängig Wasserstoff, $(C_1\text{-}C_8)$-Alkyl, das durch Amino, $(C_1\text{-}C_4)$-Alkylamino, Di-$(C_1\text{-}C_4)$-alkylamino, Hydroxy oder $(C_1\text{-}C_4)$-Alkoxy substituiert sein kann, $(C_3\text{-}C_7)$-Cycloalkyl, Mercapto, $(C_1\text{-}C_4)$-Alkylthio, Phenylthio, $(C_1\text{-}C_4)$-Alkoxycarbonyl, Carboxy, $(C_6\text{-}C_{14})$-Aryl, das im Arylrest wie bei $R^1$ beschrieben, substituiert sein kann, Het oder Het-$(C_1\text{-}C_4)$-alkyl, wobei Het wie bei $R^1$ beschrieben definiert ist, bedeutet oder wobei |
| $R^5$ und $R^6$ | zusammen mit dem sie tragenden Stickstoffatom einen 5- bis 12-gliedrigen Ring bilden, der mono-oder bicyclisch sein kann und als weitere Ringglieder noch 1 oder 2 Stickstoffatome, 1 Schwefelatom oder 1 Sauerstoffatom enthalten und durch $(C_1\text{-}C_4)$-Alkyl substituiert sein kann, bilden; |
| A | einen Rest der Gruppe S, SO, $SO_2$, O, CO, CS oder eine direkte Bindung bedeutet; |
| D | eine $CH_2$-Gruppe oder einen Rest $-NR^7-$, wobei $R^7$ Wasserstoff oder ein $(C_1\text{-}C_4)$-Alkylrest sein kann, bedeutet; |
| $R^3$ | $(C_6\text{-}C_{14})$-Aryl, $(C_6\text{-}C_{14})$-Aryl-$(C_1\text{-}C_4)$-alkyl, wobei der Arylrest jeweils durch ein, zwei oder drei Reste wie unter $R^1$ beschrieben, substituiert sein kann, Thienyl oder Thienyl-$(C_1\text{-}C_4)$-alkyl, wobei der Thiophenrest jeweils durch ein oder zwei Reste der Gruppe $(C_1\text{-}C_4)$-Alkyl, $(C_1\text{-}C_4)$-Alkoxy oder Halogen substituiert sein kann, 2-, 3- oder 4-Pyridyl oder 2-, 3- oder 4-Pyridyl-$(C_1\text{-}C_4)$-alkyl, wobei der Pyridinrest durch ein oder zwei Reste der Gruppe $(C_1\text{-}C_4)$-Alkyl, $(C_1\text{-}C_4)$-Alkoxy oder Halogen substituiert sein kann, bedeutet; |
| B | einen N-terminal über $-NR^8-$ mit $R^1$-A-D-$CHR^3$-CO- und C-terminal über -CO- mit |

$$-NH\text{-}CHR^2\text{-}CH{=}\underset{+}{\overset{O^-}{N}}\text{-}R^4$$

verknüpften Rest einer Aminosäure aus der Reihe Phenylalanin, Histidin, Tyrosin, Tryptophan, Methionin, Leucin, Isoleucin, Asparagin, Asparaginsäure, $\beta$-2-Thienylalanin, $\beta$-3-Thienylalanin, $\beta$-2-Furylalanin, $\beta$-3-Furylalanin, Lysin, Ornithin, Valin, Alanin, 2,4-Diaminobuttersäure, Arginin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, Norvalin, β-2-Benzo[b]thienylalanin, β-3-Benzo[b]thienylalanin, 2-Fluorphenylalanin, 3-Fluorphenylalanin, 4-Fluorphenylalanin, Norleucin, Cystein, S-Methyl-Cystein, 1,2,3,4-Tetrahydroisochinolin-3-carbonsäure, Homophenylalanin, DOPA, O-Dimethyl-DOPA, 2-Amino-4-(2-thienyl)-buttersäure, Benzodioxol-5-yl-alanin, N-Methyl-histidin, 2-Amino-4-(3-thienyl)-buttersäure, 3-(2-Thienyl)-serin, (Z)-Dehydrophenylalanin, (E)-Dehydrophenylalanin, (1,3-Dioxolan-2-yl)-alanin, N-Pyrrolylalanin, (1-, 3-oder 4-Pyrazolyl)alanin, (4-Thiazolyl)alanin, (2-, 4- oder 5-Pyrimidyl)alanin, Cyclopentylglycin, tert.Butylglycin oder Phenylserin, bedeutet und

$R^8$ Wasserstoff, $(C_1$-$C_6)$-Alkyl, Formyl, $(C_1$-$C_6)$-Alkoxycarbonyl oder Benzyloxycarbonyl bedeutet;

$R^2$ Wasserstoff, $(C_1$-$C_{10})$-Alkyl, $(C_4$-$C_7)$-Cycloalkyl, $(C_4$-$C_7)$-Cycloalkyl-$(C_1$-$C_4)$-alkyl, $(C_6$-$C_{14})$-Aryl, $(C_6$-$C_{14})$-Aryl-$(C_1$-$C_4)$-alkyl oder (Heterocyclyl)-$(C_1$-$C_4)$-alkyl bedeutet, wobei der Heterocyclus 4-7 Ringglieder, davon 1 oder 2 Schwefel- und/oder Sauerstoffatome besitzt, bedeutet; und

$R^4$ $(C_1$-$C_8)$-Alkyl, $(C_3$-$C_8)$-Cycloalkyl, $(C_3$-$C_8)$-Cycloalkyl-$(C_1$-$C_4)$-alkyl, die jeweils durch Amino, Hydroxy, Mercapto, Halogen, $(C_1$-$C_4)$-Alkoxy, Mono- oder Di-$(C_1$-$C_4)$-alkylamino, Carboxy, Guanidino, $(C_1$-$C_4)$-Alkoxycarbonyl oder einen Rest -$CONR^5R^6$, worin $R^5$ und $R^6$ wie oben definiert sind, einfach, zweifach oder dreifach substituiert sein können; $(C_6$-$C_{14})$-Aryl, $(C_6$-$C_{14})$-Aryl$(C_1$-$C_4)$-alkyl, wobei der Arylrest wie unter $R^1$ beschrieben und die Alkylkette wie oben für $R^4$ beschrieben, substituiert sein kann; Het oder Het-$(C_1$-$C_4)$-alkyl, wobei Het wie unter $R^1$ beschrieben, definiert ist und die Alkylkette wie oben für $R^4$ beschrieben, substituiert sein kann;

sowie deren physiologisch verträgliche Salze, dadurch gekennzeichnet, daß man

a) eine Verbindung der allgemeinen Formel II

$$R^1\text{-}A\text{-}D\text{-}\underset{|}{\overset{R^3}{C}}H\text{-}\overset{O}{\overset{||}{C}}\text{-}B\text{-}NH\text{-}\underset{|}{\overset{R^2}{C}}H\text{-}CH{=}O \qquad (II)$$

in welcher $R^1$, $R^2$, $R^3$, A, B und D die gleiche Bedeutung wie in Formel I besitzen, mit einem Hydroxylaminderivat der allgemeinen Formel III

HO-NH-$R^4$ (III)

worin $R^4$ die gleiche Bedeutung wie in Formel I besitzt, in einem organischen Lösungsmittel wie z.B. Diethylether, Ethanol, Benzol, Toluol oder in Wasser oder ohne jedes Lösungsmittel bei einer Temperatur zwischen -20 °C und dem Siedepunkt des Lösungsmittels, vorzugsweise zwischen -20 °C und +60 °C, mit oder ohne Gegenwart einer Säure, z.B. Chlorwasserstoff oder Essigsäure, die z.B. in Form der Salze der Hydroxylamine der Formel III eingebracht werden kann, mit oder ohne Gegenwart einer Hilfsbase wie z.B. Natriumhydrogencarbonat, Natriumacetat, Natriumcarbonat, Natriumethanolat oder Kaliumhydroxid sowie mit oder ohne Gegenwart eines wasserentziehenden Mittels wie z.B. Calciumchlorid oder Molekularsieb, umsetzt und gegebenenfalls zum Schutz weiterer funktioneller Gruppen (eine) temporär eingeführte Schutzgruppe(n) abspaltet

oder

b) ein Fragment mit endständiger Carboxylgruppe oder dessen reaktives Derivat mit einem entsprechenden Fragment mit freier Aminogruppe kuppelt, gegebenenfalls zum Schutz weiterer funktioneller Gruppen (eine) temporär eingeführte Schutzgruppe(n) abspaltet und die so erhaltene Verbindung gegebenenfalls in ihr physiologisch verträgliches Salz überführt.

**2.** Verfahren gemäß Anspruch 1, dadurch gekennzeichnet, daß

$R^1$ Methyl, Ethyl Isopropyl, tert.-Butyl, Isobutyl, 2-Hydroxyethyl, 2-Methoxyethyl, Carboxymethyl, 2-Carboxyethyl, Methoxycarbonylmethyl, 2-Methoxycarbonylethyl, Ethoxycarbonylmethyl, 2-Ethoxycarbonylethyl, Carbamoylmethyl, 2-Carbamoylethyl, 2-Aminoethyl, 2-Dimethylaminoethyl, 2-Morpholinoethyl, Aminoisobutyl, 2-Piperidinoethyl,

Aminopropyl, Dimethylaminopropyl, Methylaminopropyl, Piperidinopropyl, Morpholinopropyl, Methylaminoisobutyl, Dimethylaminoisobutyl, Piperidinoisobutyl, Morpholinoisobutyl, Cyclopropyl, Cyclobutyl, Cyclopentyl oder Cyclohexyl, Phenyl, 1- oder 2-Naphthyl, o-, m- oder p-Methylphenyl, o-, m- oder p-Hydroxyphenyl oder o-, m-oder p-Aminophenyl, Benzyl, 2-Phenylethyl oder $\alpha$- oder $\beta$-Naphthylmethyl, unsubstituiertes oder substituiertes Heteroaryl, z.B. 2- oder 3-Pyrrolyl, 2-Furyl, 2-Thienyl, 2- oder 4-Imidazolyl, 1-Methyl-2-, -4- oder -5-Imidazolyl, 1,3-Thiazol-2-yl, 2-, 3- oder 4-Pyridyl, 1-Oxido-2-, 3- oder 4-pyridinio, 2-Pyrazinyl, 2-, 4- oder 5-Pyrimidinyl, 2-, 3- oder 4-Chinolyl, 1-, 3- oder 4-Isochinolyl oder 2-Benzoxazolyl, Methoxy, Ethoxy oder n-Butoxy oder einen Rest $-NR^5R^6$ bedeutet, worin

$R^5$ und $R^6$ gleich oder verschieden und voneinander unabhängig Wasserstoff, Methyl, Ethyl, Propyl, Butyl, Isobutyl, sec.-Butyl, tert.-Butyl, Aminoethyl, Aminopropyl, Methylaminoethyl, Dimethylaminoethyl, Methylaminopropyl, Dimethylaminopropyl, Hydroxyethyl, Methoxyethyl, Cyclohexylmethyl, Mercaptoethyl, Methylthioethyl, Benzyl, 1-Phenethyl, 2-Phenethyl, 2-(3,4-Dimethoxy)-phenethyl, 2-Pyridylmethyl, 3-Pyridylmethyl bedeuten oder worin

$R^5$ und $R^6$ zusammen mit dem sie tragenden Stickstoffatom einen Pyrrolidin-, Piperidin-, Azepin, Azocin-, Morpholin-, Piperazin-, 4-Methyl-piperazin-, 4-Ethyl-piperazin-, Homopiperazin- oder Thiomorpholin-Ring bilden;

A einen Rest der Gruppe S, SO, $SO_2$, O, CO oder CS bedeutet;

D eine $CH_2$-Gruppe, eine NH-Gruppe oder eine $N(CH_3)$-Gruppe bedeutet;

$R^3$ Phenyl, 2-Thienyl, 2-Pyridyl, 1-Naphthyl, Phenyl-($C_1$-$C_4$)-alkyl, 2-Thienyl-($C_1$-$C_4$)-alkyl, 2-Pyridyl-($C_1$-$C_4$)-alkyl, 1-Naphthyl-($C_1$-$C_4$)-alkyl, die jeweils gegebenenfalls durch einen, zwei oder drei Reste aus der Gruppe Methyl, Ethyl, Isopropyl, tert.-Butyl, Methoxy, Hydroxy, Fluor, Chlor oder Nitro oder einen Methylendioxyrest substituiert sind, bedeutet;

B einen N-terminal über -NH- mit $R^1$-A-D-$CHR^3$-CO- und C-terminal über -CO- mit

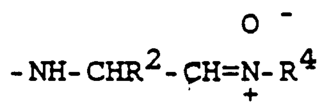

verknüpften Rest einer Aminosäure aus der Reihe Phenylalanin, Histidin, Tyrosin, Tryptophan, Methionin, Leucin, Isoleucin, Asparagin, Asparaginsäure $\beta$-2-Thienylalanin, $\beta$-3-Thienylalanin, $\beta$-2-Furylalanin, $\beta$-3-Furylalanin, Lysin, Ornithin, 2,4-Diaminobuttersäure, Arginin, Norvalin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-benzyltyrosin, O-tert.-Butyltyrosin, Phenylglycin, 1-Naphthylalanin, 2-Naphthylalanin, 4-Nitrophenylalanin, Norleucin, Valin, Alanin, Cystein, S-Methylcystein, N-Methyl-Histidin, Benzodioxol-5-yl-alanin, 1,2,3,4-Tetrahydroisochinolin-3-carbonsäure, Homophenylalanin, 2-Amino-4-(3-thienyl)-buttersäure, (Z)-Dehydrophenylalanin, (E)-Dehydrophenylalanin, (1,3-Dioxolan-2-yl)alanin, (4-Thiazolyl)alanin, (2-, 4- oder 5-Pyrimidyl)alanin, (1-,3- oder 4-Pyrazolyl)alanin, (2-,3- oder 4-Fluorphenyl)alanin, Cyclopentylglycin, tert.-Butylglycin oder Phenylserin bedeutet;

$R^2$ wie in Anspruch 1 definiert ist, und

$R^4$ Methyl, Ethyl, Propyl, Isopropyl, Butyl, Isobutyl, sec.-Butyl, tert.-Butyl, Pentyl, Isopentyl, Neopentyl, sec.-Pentyl, tert.-Pentyl, Hexyl, Cyclopentyl, Cyclohexyl, Cycloheptyl, Cyclopentylmethyl, Cyclohexylmethyl, Cycloheptylmethyl, Phenyl, Benzyl, Phenylethyl, Phenylpropyl, 2-, 3- oder 4-pyridyl, 2-, 4- oder 5-Imidazolyl, (2-, 3- oder 4-pyridyl)-methyl, (2-, 4- oder 5-Imidazolyl)methyl, wobei die genannten Alkyl-und Cycloalkylreste jeweils durch Amino, Halogen, Mono-oder Di-($C_1$-$C_4$)-alkylamino, Carboxy, Guanidino, ($C_1$-$C_4$)-Alkoxycarbonyl oder einen Rest $-CONR^5R^6$-, worin $R^5$ und $R^6$ wie oben definiert sind, einfach oder zweifach substituiert sein können; die genannten Phenylreste einfach oder zweifach durch ($C_1$-$C_4$)-Alkyl, Methoxy, Hydroxy, Amino, Aminomethyl, Fluor, Chlor oder Trifluormethyl substituiert sein können und die genannten heteroaromatischen Reste jeweils durch ein oder zwei Reste aus der Reihe ($C_1$-$C_4$)-

Alkyl, Methoxy, Fluor, Chlor, Brom oder Trifluormethyl substituiert sein können, bedeutet.

**3.** Verfahren gemäß einem oder mehreren der Ansprüche 1 bis 2, dadurch gekennzeichnet, daß

$R^1$ Methyl, Ethyl, Isopropyl, tert.-Butyl, Isobutyl, 2-Hydroxyethyl, 2-Methoxyethyl, Carboxymethyl, 2-Carboxyethyl, Methoxycarbonylmethyl, 2-Methoxycarbonylethyl, Ethoxycarbonylmethyl, 2-Ethoxycarbonylethyl, Carbamoylmethyl, 2-Carbamoylethyl, 2-Aminoethyl, 2-Dimethylaminoethyl, 2-Morpholinoethyl, Aminopropyl, Aminoisobutyl, Methylaminosobutyl, Dimethylaminoisobutyl, 2-Piperidinoethyl, Cyclopropyl, Cyclobutyl, Cyclopentyl oder Cyclohexyl, Phenyl, 1- oder 2-Naphthyl, o-, m- oder p-Methylphenyl, o-, m- oder p-Hydroxyphenyl oder o-, m- oder p-Aminophenyl, Benzyl, 2-Phenylethyl oder $\alpha$- oder $\beta$-Naphthylmethyl, unsubstituiertes oder substituiertes Heteroaryl, z.B. 2- oder 3-Pyrrolyl, 2-Furyl, 2-Thienyl, 2- oder 4-Imidazolyl, 1-Methyl-2-, -4- oder -5-Imidazolyl, 1,3-Thiazol-2-yl, 2-, 3- oder 4-Pyridyl, 1-Oxido-2-, 3- oder 4-pyridinio, 2-Pyrazinyl, 2-, 4- oder 5-Pyrimidinyl, 2-, 3- oder 4-Chinolyl, 1-, 3- oder 4-Isochinolyl oder 2-Benzoxazolyl, Methoxy, Ethoxy oder n-Butoxy oder einen Rest $-NR^5R^6$ bedeuten, worin

$R^5$ und $R^6$ gleich oder verschieden und voneinander unabhängig Wasserstoff, Methyl, Ethyl, Propyl, Butyl, Isobutyl, sec.-Butyl, tert.-Butyl, Hydroxyethyl, Methoxyethyl, Aminoethyl, Aminopropyl, Benzyl- oder Pyridylmethyl bedeuten oder worin

$R^5$ und $R^6$ zusammen mit dem sie tragenden Stickstoffatom einen Pyrrolidin-, Piperidin-, Morpholin- oder Piperazinring bilden;

A einen Rest der Gruppe S, SO, $SO_2$, O, CO oder CS bedeutet;

D eine $CH_2$-Gruppe, eine NH-Gruppe oder eine $N(CH_3)$-Gruppe bedeutet;

$R^3$ Aryl oder Arylmethyl bedeutet, wobei Aryl Phenyl, 2-Thienyl, 2-Pyridyl, 1-Naphthyl bedeutet, die jeweils gegebenenfalls durch Hydroxy, Dihydroxy, Methoxy, Dimethoxy, Fluor, Chlor oder Methylendioxy substituiert sind;

B einen N-terminal über -NH- mit $R^1$-A-D-$CHR^3$-CO- und C-terminal über -CO- mit

$$-NH-CHR^2-CH=\overset{O^-}{\underset{+}{N}}-R^4$$

verknüpften Rest einer Aminosäure aus der Reihe Phenylalanin, Histidin, Leucin, $\beta$-2-Thienylalanin, $\beta$-3-Thienylalanin, Lysin, Norvalin, 2-Fluorphenylalanin, 3-Fluorphenylalanin, 4-Fluorphenylalanin, Norleucin, S-Methylcystein, (1,3-Dioxolan-2-yl)alanin, (1-,3- oder 4-Pyrazolyl)alanin, 4-Thiazolylalanin, (2-,4- oder 5-Pyrimidyl)alanin, bedeutet;

$R^2$ Isobutyl, Cyclohexylmethyl, Benzyl oder (1,3-Dithiolan-2-yl)methyl bedeutet; und

$R^4$ Methyl, Ethyl, Propyl, Isopropyl, Butyl, Isobutyl, sec.-Butyl, tert. Butyl, Pentyl, Isopentyl, Neopentyl, sec.-Pentyl, tert. Pentyl, Hexyl, Cyclopentyl, Cyclohexyl, Cyclopentylmethyl, Cyclohexylmethyl, Phenyl, Benzyl, Phenylethyl, 2-,3- oder 4-Pyridyl, 2-Imidazolyl, (2-,3- oder 4-Pyridyl)methyl, 2-Imidazolylmethyl, wobei die genannten Alkyl- und Cycloalkylreste jeweils durch Amino, Fluor, Chlor, Methylamino, Ethylamino, Dimethylamino, Diethylamino, Carboxy, Guanidino, Methoxycarbonyl, Ethoxycarbonyl, tert. Butyloxycarbonyl, oder einen Rest $-CONR^5R^6$, worin $R^5$ und $R^6$ wie oben definiert sind, einfach oder zweifach, substituiert sein können, die genannten Phenylreste ein- oder zweifach durch Methyl, Methoxy, Hydroxy, Amino, Aminomethyl, Fluor, Chlor oder Trifluormethyl substituiert sein können und die genannten heteroaromatischen Reste mit jeweils ein oder zwei Resten aus der Reihe Methyl, Methoxy, Fluor, Chlor oder Trifluormethyl substituiert sein können, bedeutet.

**4.** Verwendung einer Verbindung gemäß einem der Ansprüche 1 bis 3 als Heilmittel.

**5.** Verwendung einer Verbindung gemäß einem der Ansprüche 1 bis 3 als Heilmittel bei der Behandlung des Bluthochdrucks.

6. Verwendung einer Verbindung gemäß einem der Ansprüche 1 bis 3 als Heilmittel bei der Behandlung von Viruserkrankungen.

7. Verfahren zur Herstellung einer pharmazeutischen Zübereitung enthaltend eine nach einem der Verfahren der Ansprüche 1 bis 3 hergestellten Verbindung der Formel I, dadurch gekennzeichnet, daß man diese zusammen mit einem physiologisch unbedenklichen Träger und gegebenenfalls weiteren Hilfs- und Zusatzstoffen in eine geeignete Darreichungsform bringt.

**Claims**

**Claims for the following Contracting States : AT, BE, CH, DE, FR, GB, IT, LI, LU, NL, SE**

1. A compound of the formula I

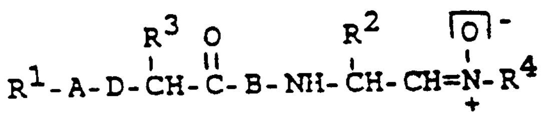

(I)

in which

R[1] denotes hydrogen, $(C_1\text{-}C_{18})$-alkyl, $(C_3\text{-}C_7)$-cycloalkyl, each of which can be substituted by amino, hydroxyl, mercapto, halogen, $(C_1\text{-}C_4)$-alkoxy, mono- or di-$(C_1\text{-}C_4)$-alkylamino, carboxyl, $(C_1\text{-}C_4)$-alkoxycarbonyl, phenoxy, phenyl-$(C_1\text{-}C_4)$-alkoxy, phenyl-$(C_1\text{-}C_4)$-alkoxycarbonyl or a radical $-CONR^5R^6$; $(C_1\text{-}C_4)$-alkoxy, $(C_6\text{-}C_{14})$-aryl, $(C_6\text{-}C_{14})$-aryl-$(C_1\text{-}C_4)$-alkyl, $(C_6\text{-}C_{14})$-aryl-$(C_1\text{-}C_4)$-alkoxy, it being possible for each aryl radical to be substituted by one, two or three radicals from the group comprising $(C_1\text{-}C_6)$-alkyl, amino, mono- or di-$(C_1\text{-}C_4)$-alkylamino, amino-$(C_1\text{-}C_4)$-alkyl, hydroxy-$(C_1\text{-}C_4)$-alkyl, mono- or di-$(C_1\text{-}C_4)$-alkylamino-$(C_1\text{-}C_4)$-alkyl, hydroxyl, $(C_1\text{-}C_4)$-alkoxy, halogen, formyl, $(C_1\text{-}C_4)$-alkoxycarbonyl, carboxamido, mono- or di-$(C_1\text{-}C_4)$-alkylaminocarbonyl or nitro or by one methylenedioxy radical; Het or Het-$(C_1\text{-}C_4)$-alkyl,where Het represents a 5-, 6- or 7-membered heterocyclic ring which can be benzo-fused and either aromatic, partially hydrogenated or completely hydrogenated and which can contain as heteroelements one or two radicals from the group comprising N, O, S, NO, SO or $SO_2$ and can be substituted by one or two radicals from the group comprising $(C_1\text{-}C_4)$-alkyl, $(C_1\text{-}C_4)$-alkoxy, $(C_1\text{-}C_4)$-alkoxycarbonyl, hydroxyl, halogen, amino, mono- or di-$(C_1\text{-}C_4)$-alkylamino, or denotes a radical $-NR^5R^6$, where

$R^5$ and $R^6$ denote, identically or differently and independently of one another, hydrogen, $(C_1\text{-}C_8)$-alkyl which can be substituted by amino, $(C_1\text{-}C_4)$-alkylamino, di-$(C_1\text{-}C_4)$-alkylamino, hydroxyl or $(C_1\text{-}C_4)$-alkoxy, or $(C_3\text{-}C_7)$-cycloalkyl, mercapto, $(C_1\text{-}C_4)$-alkylthio, phenylthio, $(C_1\text{-}C_4)$-alkoxycarbonyl, carboxyl, $(C_6\text{-}C_{14})$-aryl which can be substituted in the aryl radical as described for $R^1$, or Het or Het-$(C_1\text{-}C_4)$-alkyl, where Het is defined as described for $R^1$, or where

$R^5$ and $R^6$ form, together with the nitrogen atom carrying them, a 5- to 12-membered ring which can be mono- or bicyclic and can contain as further ring members also 1 or 2 nitrogen atoms, 1 sulfur atom or 1 oxygen atom and can be substituted by $(C_1\text{-}C_4)$-alkyl;

A denotes a radical from the group comprising S, SO, $SO_2$, O, CO, CS or a direct bond;

D denotes a $CH_2$ group or a radical $-NR^7-$, where $R^7$ can be hydrogen or a $(C_1\text{-}C_4)$-alkyl radical;

$R^3$ denotes $(C_6\text{-}C_{14})$-aryl, $(C_6\text{-}C_{14})$-aryl-$(C_1\text{-}C_4)$-alkyl, it being possible for each aryl radical to be substituted by one, two or three radicals as described under $R^1$, or thienyl or thienyl-$(C_1\text{-}C_4)$-alkyl, it being possible for each thiophene radical to be substituted by one or two radicals from the group comprising $(C_1\text{-}C_4)$-alkyl, $(C_1\text{-}C_4)$-alkoxy or halogen, or 2-, 3- or 4-pyridyl or 2-, 3- or 4-pyridyl-$(C_1\text{-}C_4)$-alkyl, it being possible for the pyridine radical to be substituted by one or two radicals from the group comprising $(C_1\text{-}C_4)$-alkyl, $(C_1\text{-}C_4)$-alkoxy or halogen;

B denotes a radical, which is linked N-terminal via $-NR^8-$ with $R^1$-A-D-$CHR^3$-CO- and C-

terminal via -CO- with

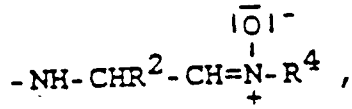

of an amino acid from the series comprising phenylalanine, histidine, tyrosine, tryptophan, methionine, leucine, isoleucine, asparagine, aspartic acid, b-2-thienylalanine, b-3-thienylalanine, b-2-furylalanine, b-3-furylalanine, lysine, ornithine, valine, alanine, 2,4-diaminobutyric acid, arginine, 4-chlorophenylalanine, methionine sulfone, methionine sulfoxide, 2-pyridylalanine, 3-pyridylalanine, cyclohexylalanine, cyclohexylglycine, im-methylhistidine, O-methyltyrosine, O-benzyltyrosine, O-tert.-butyltyrosine, phenylglycine, 1-naphthylalanine, 2-naphthylalanine, 4-nitrophenylalanine, norvaline, b-2-benzo[b]thienylalanine, b-3-benzo[b]thienylalanine, 2-fluorophenylalanine, 3-fluorophenylalanine, 4-fluorophenylalanine, norleucine, cysteine, S-methylcysteine, 1,2,3,4-tetrahydroisoquinoline-3-carboxylic acid, homophenylalanine, DOPA, O-dimethyl-DOPA, 2-amino-4-(2-thienyl)-butyric acid, benzodioxol-5-yl-alanine, N-methyl-histidine, 2-amino-4-(3-thienyl)-butyric acid, 3-(2-thienyl)-serine, (Z)-dehydrophenylalanine, (E)-dehydrophenylalanine, (1,3-dioxolan-2-yl)alanine, N-pyrrolylalanine, (1-, 3- or 4-pyrazolyl)alanine, (4-thiazolyl)alanine, (2-,4- or 5-pyrimidyl)alanine, cyclopentylglycine, tert.butylglycine or phenylserine,

and $R^8$ denotes hydrogen, $(C_1-C_6)$-alkyl, formyl, $(C_1-C_6)$-alkoxycarbonyl or benzyloxycarbonyl;

$R^2$ denotes hydrogen, $(C_1-C_{10})$-alkyl, $(C_4-C_7)$-cycloalkyl, $(C_4-C_7)$-cycloalkyl-$(C_1-C_4)$-alkyl, $(C_6-C_{14})$-aryl, $(C_6-C_{14})$-aryl-$(C_1-C_4)$-alkyl or (heterocyclyl)-$(C_1-C_4)$-alkyl, where the heterocycle has 4-7 ring members, of which 1 or 2 are sulfur and/or oxygen atoms; and

$R^4$ denotes $(C_1-C_8)$-alkyl, $(C_3-C_8)$-cycloalkyl, $(C_3-C_8)$-cycloalkyl-$(C_1-C_4)$-alkyl, each of which can be substituted once, twice or three times by amino, hydroxyl, mercapto, halogen, $(C_1-C_4)$-alkoxy, mono- or di-$(C_1-C_4)$-alkylamino, carboxyl, guanidino, $(C_1-C_4)$-alkoxycarbonyl or a radical $-CONR^5R^6$, in which $R^5$ and $R^6$ are as defined above; $(C_6-C_{14})$-aryl, $(C_6-C_{14})$-aryl-$(C_1-C_4)$-alkyl, it being possible for the aryl radical to be substituted as described under $R^1$ and for the alkyl chain to be substituted as described above for $R^4$; Het or Het-$(C_1-C_4)$-alkyl, where Het is defined as described under $R^1$, and the alkyl chain can be substituted as described above for $R^4$;

as well as the physiologically tolerated salts thereof.

**2.** A compound of the formula I as claimed in claim 1, wherein

$R^1$ denotes methyl, ethyl, isopropyl, tert.-butyl, isobutyl, 2-hydroxyethyl, 2-methoxyethyl, carboxymethyl, 2-carboxyethyl, methoxycarbonylmethyl, 2-methoxycarbonylethyl, ethoxycarbonylmethyl, 2-ethoxycarbonylethyl, carbamoylmethyl, 2-carbamoylethyl, 2-aminoethyl, 2-dimethylaminoethyl, 2-morpholinoethyl, aminoisobutyl, 2-piperidinoethyl, aminopropyl, dimethylaminopropyl, methylaminopropyl, piperidinopropyl, morpholinopropyl, methylaminoisobutyl, dimethylaminoisobutyl, piperidinoisobutyl, morpholinoisobutyl, cyclopropyl, cyclobutyl, cyclopentyl or cyclohexyl, phenyl, 1- or 2-naphthyl, o-, m- or p-methylphenyl, o-, m- or p-hydroxyphenyl or o-, m- or p-aminophenyl, benzyl, 2-phenylethyl or a- or b-naphthylmethyl, unsubstituted or substituted heteroaryl, for example 2- or 3-pyrrolyl, 2-furyl, 2-thienyl, 2- or 4-imidazolyl, 1-methyl-2-, -4- or -5-imidazolyl, 1,3-thiazol-2-yl, 2-, 3- or 4-pyridyl, 1-oxido-2-, 3- or 4-pyridinio, 2-pyrazinyl, 2, 4- or 5-pyrimidinyl, 2-, 3- or 4-quinolyl, 1-, 3- or 4-isoquinolyl or 2-benzoxazolyl, methoxy, ethoxy or n-butoxy or a radical $-NR^5R^6$, in which

$R^5$ and $R^6$ denote, identically or differently and independently of one another, hydrogen, methyl, ethyl, propyl, butyl, isobutyl, sec.-butyl, tert.-butyl, aminoethyl, aminopropyl, methylaminoethyl, dimethylaminoethyl, methylaminopropyl, dimethylaminopropyl, hydroxyethyl, methoxyethyl, cyclohexylmethyl, mercaptoethyl, methylthioethyl, benzyl, 1-phenethyl, 2-phenethyl, 2-(3,4-dimethoxy)-phenethyl, 2-pyridylmethyl, 3-pyridylmethyl, or in which

$R^5$ and $R^6$ form, together with the nitrogen atom carrying them, a pyrrolidine, piperidine, azepine, azocine, morpholine, piperazine, 4-methyl-piperazine, 4-ethyl-piperazine, homopiperazine or thiomorpholine ring;

A denotes a radical from the group comprising S, SO, $SO_2$, O, CO or CS;

D denotes a $CH_2$ group, an NH group or an $N(CH_3)$ group;

$R^3$ denotes phenyl, 2-thienyl, 2-pyridyl, 1-naphthyl, phenyl-($C_1$-$C_4$)-alkyl, 2-thienyl-($C_1$-$C_4$)-alkyl, 2-pyridyl-($C_1$-$C_4$)-alkyl, 1-naphthyl-($C_1$-$C_4$)-alkyl, each of which is optionally substituted by one, two or three radicals from the group comprising methyl, ethyl, isopropyl, tert.-butyl, methoxy, hydroxyl, fluorine, chlorine or nitro or one methylenedioxy radical;

B denotes a radical, which is linked N-terminal via -NH- with $R^1$-A-D-$CHR^3$-CO- and C-terminal via -CO- with

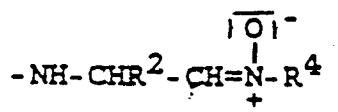 ,

of an amino acid from the series comprising phenylalanine, histidine, tyrosine, tryptophan, methionine, leucine, isoleucine, asparagine, aspartic acid, b-2-thienylalanine, b-3-thienylalanine, b-2-furylalanine, b-3-furylalanine, lysine, ornithine, 2,4-diaminobutyric acid, arginine, norvaline, 4-chlorophenylalanine, methionine sulfone, methionine sulfoxide, 2-pyridylalanine, 3-pyridylalanine, cyclohexylalanine, cyclohexylglycine, im-methylhistidine, O-methyltyrosine, O-benzyltyrosine, O-tert.-butyltyrosine, phenylglycine, 1-naphthylalanine, 2-naphthylalanine, 4-nitrophenylalanine, norleucine, valine, alanine, cysteine, S-methylcysteine, N-methylhistidine, benzodioxol-5-yl-alanine, 1,2,3,4-tetrahydroisoquinoline-3-carboxylic acid, homophenylalanine, 2-amino-4-(3-thienyl)butyric acid, (Z)-dehydrophenylalanine, (E)-dehydrophenylalanine, (1,3-dioxolan-2-yl)alanine, (4-thiazolyl)alanine, (2, 4- or 5-pyrimidyl)alanine, (1-, 3- or 4-pyrazolyl)alanine, (2-, 3- or 4-fluorophenyl)alanine, cyclopentylglycine, tert. -butylglycine or phenylserine;

$R^2$ is as defined in claim 1, and

$R^4$ denotes methyl, ethyl, propyl, isopropyl, butyl, isobutyl, sec.-butyl, tert.-butyl, pentyl, isopentyl, neopentyl, sec.-pentyl, tert.-pentyl, hexyl, cyclopentyl, cyclohexyl, cycloheptyl, cyclopentylmethyl, cyclohexylmethyl, cycloheptylmethyl, phenyl, benzyl, phenylethyl, phenylpropyl, 2-, 3- or 4-pyridyl, 2-, 4- or 5-imidazolyl, (2-, 3- or 4-pyridyl)methyl, (2-, 4- or 5-imidazolyl)methyl, it being possible for the said alkyl and cycloalkyl radicals in each case to be substituted once or twice by amino, halogen, mono- or di-($C_1$-$C_4$)-alkylamino, carboxyl, guanidino, ($C_1$-$C_4$)-alkoxycarbonyl or a radical -$CONR^5R^6$ in which $R^5$ and $R^6$ are as defined above; it being possible for the said phenyl radicals to be substituted once or twice by ($C_1$-$C_4$)-alkyl, methoxy, hydroxyl, amino, aminomethyl, fluorine, chlorine or trifluoromethyl, and it being possible for the said heteroaromatic radicals in each case to be substituted by one or two radicals from the series comprising ($C_1$-$C_4$)-alkyl, methoxy, fluorine, chlorine, bromine or trifluoromethyl,

as well as the physiologically tolerated salts thereof.

**3.** A compound of the formula I as claimed in one or more of claims 1 to 2, wherein

$R^1$ denotes methyl, ethyl, isopropyl, tert.-butyl, isobutyl, 2-hydroxyethyl, 2-methoxyethyl, carboxymethyl, 2-carboxyethyl, methoxycarbonylmethyl, 2-methoxycarbonylethyl, ethoxycarbonylmethy], 2-ethoxycarbonylethyl, carbamoylmethyl, 2-carbamoylethyl, 2-aminoethyl, 2-dimethylaminoethyl, 2-morpholinoethyl, aminopropyl, aminoisobutyl, methylaminoisobutyl, dimethylaminoisobutyl, 2-piperidinoethyl, cyclopropyl, cyclobutyl, cyclopentyl or cyclohexyl, phenyl, 1- or 2-naphthyl, o-, m- or p-methylphenyl, o-, m- or p-hydroxyphenyl or o-, m- or p-aminophenyl, benzyl, 2-phenylethyl or a- or b-naphthylmethyl, unsubstituted or substituted heteroaryl, for example 2- or 3-pyrrolyl, 2-furyl, 2-thienyl, 2- or 4-imidazolyl, 1-methyl-2-, -4- or -5-imidazolyl, 1,3-thiazol-2-yl, 2-, 3- or 4-pyridyl, 1-oxido-2-, 3- or 4-pyridinio, 2-pyrazinyl, 2-, 4- or 5-pyrimidinyl, 2-, 3- or 4-quinolyl, 1-, 3- or 4-isoquinolyl or 2-benzoxazolyl, methoxy, ethoxy or n-butoxy or a radical $-NR^5R^6$, in which

$R^5$ and $R^6$ denote, identically or differently and independently of one another, hydrogen, methyl, ethyl, propyl, butyl, isobutyl, sec.-butyl, tert.-butyl, hydroxyethyl, methoxyethyl, aminoethyl, aminopropyl, benzyl- or pyridylmethyl, or in which

$R^5$ and $R^6$ form, together with the nitrogen atom carrying them, a pyrrolidine, piperidine, morpholine or piperazine ring;

A denotes a radical from the group comprising S, SO, $SO_2$, O, CO or CS;

D denotes a $CH_2$ group, an NH group or an $N(CH_3)$ group;

$R^3$ denotes aryl or arylmethyl, where aryl denotes phenyl, 2-thienyl, 2-pyridyl or 1-naphthyl, each of which is optionally substituted by hydroxyl, dihydroxyl, methoxy, dimethoxy, fluorine, chlorine or methylenedioxy;

B denotes a radical, which is linked N-terminal via -NH- with $R^1$-A-D-$CHR^3$-CO- and C-terminal via -CO- with

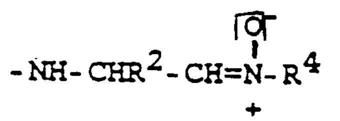 ,

of an amino acid from the series comprising phenylalanine, histidine, leucine, b-2-thienylalanine, b-3-thienylalanine, lysine, norvaline, 2-fluorophenylalanine, 3-fluorophenylalanine, 4-fluorophenylalanine, norleucine, S-methylcysteine, (1,3-dioxolan-2-yl)alanine, (1-, 3- or 4-pyrazolyl)alanine, 4-thiazolylalanine, (2-, 4- or 5-pyrimidyl)alanine;

$R^2$ denotes isobutyl, cyclohexylmethyl, benzyl or (1,3-dithiolan-2-yl)methyl; and

$R^4$ denotes methyl, ethyl, propyl, isopropyl, butyl, isobutyl, sec.-butyl, tert.-butyl, pentyl, isopentyl, neopentyl, sec.-pentyl, tert.-pentyl, hexyl, cyclopentyl, cyclohexyl, cyclopentylmethyl, cyclohexylmethyl, phenyl, benzyl, phenylethyl, 2-, 3- or 4-pyridyl, 2-imidazolyl, (2-, 3- or 4-pyridyl)methyl, 2-imidazolylmethyl, it being possible for the said alkyl and cycloalkyl radicals in each case to be substituted once or twice by amino, fluorine, chlorine, methylamino, ethylamino, dimethylamino, diethylamino, carboxyl, guanidino, methoxycarbonyl, ethoxycarbonyl, tert.-butyloxycarbonyl or a radical $-CONR^5R^6$, in which $R^5$ and $R^6$ are defined as above, it being possible for the said phenyl radicals to be substituted once or twice by methyl, methoxy, hydroxyl, amino, aminomethyl, fluorine, chlorine or trifluoromethyl, and it being possible for the said heteroaromatic radicals to be substituted with, in each case, one or two radicals from the series comprising methyl, methoxy, fluorine, chlorine or trifluoromethyl, as well as the physiologically tolerated salts thereof.

**4.** A process for the preparation of a compound of the formula I as claimed in one or more of claims 1 to 3, which comprises

a) reacting a compound of the general formula (II)

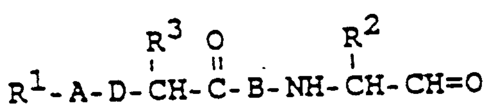 (II)

in which $R^1$, $R^2$, $R^3$, A, B and D have the same meaning as in formula I, with a hydroxylamine derivative of the general formula III

$HO-NH-R^4$ (III)

in which $R^4$ has the same meaning as in formula I, in an organic solvent such as, for example, diethyl ether, ethanol, benzene, toluene or in water or without any solvent at a temperature between -20 °C and the boiling point of the solvent, preferably between -20 °C and +60 °C, with or without the presence of an acid, for example hydrogen chloride or acetic acid, which can be introduced, for

example, in the form of the salts of the hydroxylamines of the formula III, with or without the presence of an auxiliary base such as, for example, sodium bicarbonate, sodium acetate, sodium carbonate, sodium ethanolate or potassium hydroxide, and with or without the presence of a water-extracting agent such as, for example, calcium chloride or molecular sieves, and, where appropriate, eliminating (a) protective group(s) temporarily introduced to protect other functional groups,
or
b) coupling a fragment with a terminal carboxyl group, or the reactive derivative thereof, with a corresponding fragment with a free amino group, where appropriate eliminating (a) protective group-(s) temporarily introduced to protect other functional groups, and converting the compound obtained in this way into the physiologically tolerated salt thereof where appropriate.

**5.** A compound of the formula I as claimed in one or more of claims 1 to 3 for use as a medicine.

**6.** A compound of the formula I as claimed in one or more of claims 1 to 3 for use as a medicine for the treatment of high blood pressure.

**7.** A compound of the formula I as claimed in one or more of claims 1 to 3 for use as a medicine for the treatment of viral diseases.

**8.** A pharmaceutical composition containing a compound of the formula I as claimed in one or more of claims 1 to 3.

**Claims for the following Contracting States : ES, GR**

**1.** A process for the preparation of a compound of the formula I

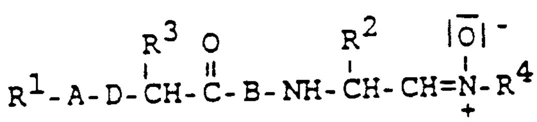

(I)

in which

$R^1$ denotes hydrogen, $(C_1\text{-}C_{18})$-alkyl, $(C_3\text{-}C_7)$-cycloalkyl, each of which can be substituted by amino, hydroxyl, mercapto, halogen, $(C_1\text{-}C_4)$-alkoxy, mono- or di-$(C_1\text{-}C_4)$-alkylamino, carboxyl, $(C_1\text{-}C_4)$-alkoxycarbonyl, phenoxy, phenyl-$(C_1\text{-}C_4)$-alkoxy, phenyl-$(C_1\text{-}C_4)$-alkoxycarbonyl or a radical $-CONR^5R^6$; $(C_1\text{-}C_4)$-alkoxy, $(C_6\text{-}C_{14})$-aryl, $(C_6\text{-}C_{14})$-aryl-$(C_1\text{-}C_4)$-alkyl, $(C_6\text{-}C_{14})$-aryl-$(C_1\text{-}C_4)$-alkoxy, it being possible for each aryl radical to be substituted by one, two or three radicals from the group comprising $(C_1\text{-}C_6)$-alkyl, amino, mono- or di-$(C_1\text{-}C_4)$-alkylamino, amino-$(C_1\text{-}C_4)$-alkyl, hydroxy-$(C_1\text{-}C_4)$-alkyl, mono- or di-$(C_1\text{-}C_4)$-alkylamino-$(C_1\text{-}C_4)$-alkyl, hydroxyl, $(C_1\text{-}C_4)$-alkoxy, halogen, formyl, $(C_1\text{-}C_4)$-alkoxycarbonyl, carboxamido, mono- or di-$(C_1\text{-}C_4)$-alkylaminocarbonyl or nitro or by one methylenedioxy radical; Het or Het-$(C_1\text{-}C_4)$-alkyl,where Het represents a 5-, 6- or 7-membered heterocyclic ring which can be benzo-fused and either aromatic, partially hydrogenated or completely hydrogenated and which can contain as heteroelements one or two radicals from the group comprising N, O, S, NO, SO or $SO_2$ and can be substituted by one or two radicals from the group comprising $(C_1\text{-}C_4)$-alkyl, $(C_1\text{-}C_4)$-alkoxy, $(C_1\text{-}C_4)$-alkoxycarbonyl, hydroxyl, halogen, amino, mono- or di-$(C_1\text{-}C_4)$-alkylamino, or denotes a radical $-NR^5R^6$, where

$R^5$ and $R^6$ denote, identically or differently and independently of one another, hydrogen, $(C_1\text{-}C_8)$-alkyl which can be substituted by amino, $(C_1\text{-}C_4)$-alkylamino, di-$(C_1\text{-}C_4)$-alkylamino, hydroxyl or $(C_1\text{-}C_4)$-alkoxy, or $(C_3\text{-}C_7)$-cycloalkyl, mercapto, $(C_1\text{-}C_4)$-alkylthio, phenylthio, $(C_1\text{-}C_4)$-alkoxycarbonyl, carboxyl, $(C_6\text{-}C_{14})$-aryl which can be substituted in the aryl radical as described for $R^1$, or Het or Het-$(C_1\text{-}C_4)$-alkyl, where Het is defined as described for $R^1$, or where
$R^5$ and $R^6$ form, together with the nitrogen atom carrying them, a 5- to 12-membered ring which can be mono- or bicyclic and can contain as further ring members also 1

or 2 nitrogen atoms, 1 sulfur atom or 1 oxygen atom and can be substituted by ($C_1$-$C_4$)-alkyl;

A denotes a radical from the group comprising S, SO, $SO_2$, O, CO, CS or a direct bond;

D denotes a $CH_2$ group or a radical $-NR^7-$, where $R^7$ can be hydrogen or a ($C_1$-$C_4$)-alkyl radical;

$R^3$ denotes ($C_6$-$C_{14}$)-aryl, ($C_6$-$C_{14}$)-aryl-($C_1$-$C_4$)-alkyl, it being possible for each aryl radical to be substituted by one, two or three radicals as described under $R^1$, or thienyl or thienyl-($C_1$-$C_4$)-alkyl, it being possible for each thiophene radical to be substituted by one or two radicals from the group comprising ($C_1$-$C_4$)-alkyl, ($C_1$-$C_4$)-alkoxy or halogen, or 2-, 3- or 4-pyridyl or 2-, 3- or 4-pyridyl-($C_1$-$C_4$)-alkyl, it being possible for the pyridine radical to be substituted by one or two radicals from the group comprising ($C_1$-$C_4$)-alkyl, ($C_1$-$C_4$)-alkoxy or halogen;

B denotes a radical, which is linked N-terminal via $-NR^8-$ with $R^1$-A-D-$CHR^3$-CO- and C-terminal via -CO- with

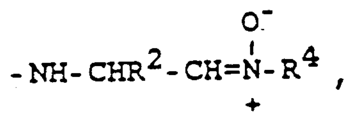

of an amino acid from the series comprising phenylalanine, histidine, tyrosine, tryptophan, methionine, leucine, isoleucine, asparagine, aspartic acid, b-2-thienylalanine, b-3-thienylalanine, b-2-furylalanine, b-3-furylalanine, lysine, ornithine, valine, alanine, 2,4-diaminobutyric acid, arginine, 4-chlorophenylalanine, methionine sulfone, methionine sulfoxide, 2-pyridylalanine, 3-pyridylalanine, cyclohexylalanine, cyclohexylglycine, im-methylhistidine, O-methyltyrosine, O-benzyltyrosine, O-tert.-butyltyrosine, phenylglycine, 1-naphthylalanine, 2-naphthylalanine, 4-nitrophenylalanine, norvaline, b-2-benzo[b]thienylalanine, b-3-benzo[b]thienylalanine, 2-fluorophenylalanine, 3-fluorophenylalanine, 4-fluorophenylalanine, norleucine, cysteine, S-methylcysteine, 1,2,3,4-tetrahydroisoquinoline-3-carboxylic acid, homophenylalanine, DOPA, O-dimethyl-DOPA, 2-amino-4-(2-thienyl)-butyric acid, benzodioxol-5-yl-alanine, N-methyl-histidine, 2-amino-4-(3-thienyl)-butyric acid, 3-(2-thienyl)-serine, (Z)-dehydrophenylalanine, (E)-dehydrophenylalanine, (1,3-dioxolan-2-yl)alanine, N-pyrrolylalanine, (1-, 3- or 4-pyrazolyl)alanine, (4-thiazolyl)alanine, (2-,4- or 5-pyrimidyl)alanine, cyclopentylglycine, tert.butylglycine or phenylserine,

and $R^8$ denotes hydrogen, ($C_1$-$C_6$)-alkyl, formyl, ($C_1$-$C_6$)-alkoxycarbonyl or benzyloxycarbonyl;

$R^2$ denotes hydrogen, ($C_1$-$C_{10}$)-alkyl, ($C_4$-$C_7$)-cycloalkyl, ($C_4$-$C_7$)-cycloalkyl-($C_1$-$C_4$)-alkyl, ($C_6$-$C_{14}$)-aryl, ($C_6$-$C_{14}$)-aryl-($C_1$-$C_4$)-alkyl or (heterocyclyl)-($C_1$-$C_4$)-alkyl, where the heterocycle has 4-7 ring members, of which 1 or 2 are sulfur and/or oxygen atoms; and

$R^4$ denotes ($C_1$-$C_8$)-alkyl, ($C_3$-$C_8$)-cycloalkyl, ($C_3$-$C_8$)-cycloalkyl-($C_1$-$C_4$)-alkyl, each of which can be substituted once, twice or three times by amino, hydroxyl, mercapto, halogen, ($C_1$-$C_4$)-alkoxy, mono- or di-($C_1$-$C_4$)-alkylamino, carboxyl, guanidino, ($C_1$-$C_4$)-alkoxycarbonyl or a radical $-CONR^5R^6$, in which $R^5$ and $R^6$ are as defined above; ($C_6$-$C_{14}$)-aryl, ($C_6$-$C_{14}$)-aryl-($C_1$-$C_4$)-alkyl, it being possible for the aryl radical to be substituted as described under $R^1$ and for the alkyl chain to be substituted as described above for $R^4$; Het or Het-($C_1$-$C_4$)-alkyl, where Het is defined as described under $R^1$, and the alkyl chain can be substituted as described above for $R^4$;

as well as the physiologically tolerated salts thereof, which comprises

a) reacting a compound of the general formula (II)

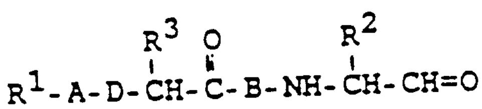

(II)

in which $R^1$, $R^2$, $R^3$, A, B and D have the same meaning as in formula I, with a hydroxylamine

derivative of the general formula III

HO-NH-$R^4$ (III)

in which $R^4$ has the same meaning as in formula I, in an organic solvent such as, for example, diethyl ether, ethanol, benzene, toluene or in water or without any solvent at a temperature between -20 °C and the boiling point of the solvent, preferably between -20 °C and +60 °C, with or without the presence of an acid, for example hydrogen chloride or acetic acid, which can be introduced, for example, in the form of the salts of the hydroxylamines of the formula III, with or without the presence of an auxiliary base such as, for example, sodium bicarbonate, sodium acetate, sodium carbonate, sodium ethanolate or potassium hydroxide, and with or without the presence of a water-extracting agent such as, for example, calcium chloride or molecular sieves, and, where appropriate, eliminating (a) protective group(s) temporarily introduced to protect other functional groups,
or
b) coupling a fragment with a terminal carboxyl group, or the reactive derivative thereof, with a corresponding fragment with a free amino group, where appropriate eliminating (a) protective group-(s) temporarily introduced to protect other functional groups, and converting the compound obtained in this way into the physiologically tolerated salt thereof where appropriate.

**2.** A process as claimed in claim 1, wherein

$R^1$ denotes methyl, ethyl, isopropyl, tert.-butyl, isobutyl, 2-hydroxyethyl, 2-methoxyethyl, carboxymethyl, 2-carboxyethyl, methoxycarbonylmethyl, 2-methoxycarbonylethyl, ethoxycarbonylmethyl, 2-ethoxycarbonylethyl, carbamoylmethyl, 2-carbamoylethyl, 2-aminoethyl, 2-dimethylaminoethyl, 2-morpholinoethyl, aminoisobutyl, 2-piperidinoethyl, aminopropyl, dimethylaminopropyl, methylaminopropyl, piperidinopropyl, morpholinopropyl, methylaminoisobutyl, dimethylaminoisobutyl, piperidinoisobutyl, morpholinoisobutyl, cyclopropyl, cyclobutyl, cyclopentyl or cyclohexyl, phenyl, 1- or 2-naphthyl, o-, m- or p-methylphenyl, o-, m- or p-hydroxyphenyl or o-, m- or p-aminophenyl, benzyl, 2-phenylethyl or a- or b-naphthylmethyl, unsubstituted or substituted heteroaryl, for example 2- or 3-pyrrolyl, 2-furyl, 2-thienyl, 2- or 4-imidazolyl, 1-methyl-2-, -4- or -5-imidazolyl, 1,3-thiazol-2-yl, 2-, 3- or 4-pyridyl, 1-oxido-2-, 3- or 4-pyridinio, 2-pyrazinyl, 2, 4- or 5-pyrimidinyl, 2-, 3- or 4-quinolyl, 1-, 3- or 4-isoquinolyl or 2-benzoxazolyl, methoxy, ethoxy or n-butoxy or a radical $-NR^5R^6$, in which

$R^5$ and $R^6$ denote, identically or differently and independently of one another, hydrogen, methyl, ethyl, propyl, butyl, isobutyl, sec.-butyl, tert.-butyl, aminoethyl, aminopropyl, methylaminoethyl, dimethylaminoethyl, methylaminopropyl, dimethylaminopropyl, hydroxyethyl, methoxyethyl, cyclohexylmethyl, mercaptoethyl, methylthioethyl, benzyl, 1-phenethyl, 2-phenethyl, 2-(3,4-dimethoxy)-phenethyl, 2-pyridylmethyl, 3-pyridylmethyl, or in which

$R^5$ and $R^6$ form, together with the nitrogen atom carrying them, a pyrrolidine, piperidine, azepine, azocine, morpholine, piperazine, 4-methyl-piperazine, 4-ethyl-piperazine, homopiperazine or thiomorpholine ring;

A denotes a radical from the group comprising S, SO, $SO_2$, O, CO or CS;

D denotes a $CH_2$ group, an NH group or an $N(CH_3)$ group;

$R^3$ denotes phenyl, 2-thienyl, 2-pyridyl, 1-naphthyl, phenyl-($C_1$-$C_4$)-alkyl, 2-thienyl-($C_1$-$C_4$)-alkyl, 2-pyridyl-($C_1$-$C_4$)-alkyl, 1-naphthyl-($C_1$-$C_4$)-alkyl, each of which is optionally substituted by one, two or three radicals from the group comprising methyl, ethyl, isopropyl, tert.-butyl, methoxy, hydroxyl, fluorine, chlorine or nitro or one methylenedioxy radical;

B denotes a radical, which is linked N-terminal via -NH- with $R^1$-A-D-$CHR^3$-CO- and C-terminal via -CO- with

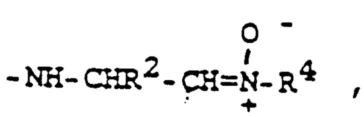

of an amino acid from the series comprising phenylalanine, histidine, tyrosine,

tryptophan, methionine, leucine, isoleucine, asparagine, aspartic acid, b-2-thienylalanine, b-3-thienylalanine, b-2-furylalanine, b-3-furylalanine, lysine, ornithine, 2,4-diaminobutyric acid, arginine, norvaline, 4-chlorophenylalanine, methionine sulfone, methionine sulfoxide, 2-pyridylalanine, 3-pyridylalanine, cyclohexylalanine, cyclohexylglycine, im-methylhistidine, O-methyltyrosine, O-benzyltyrosine, O-tert.-butyltyrosine, phenylglycine, 1-naphthylalanine, 2-naphthylalanine, 4-nitrophenylalanine, norleucine, valine, alanine, cysteine, S-methylcysteine, N-methylhistidine, benzodioxol-5-yl-alanine, 1,2,3,4-tetrahydroisoquinoline-3-carboxylic acid, homophenylalanine, 2-amino-4-(3-thienyl)butyric acid, (Z)-dehydrophenylalanine, (E)-dehydrophenylalanine, (1,3-dioxolan-2-yl)alanine, (4-thiazolyl)alanine, (2, 4- or 5-pyrimidyl)alanine, (1-, 3- or 4-pyrazolyl)alanine, (2-, 3- or 4-fluorophenyl)alanine, cyclopentylglycine, tert.-butylglycine or phenylserine;

$R^2$ is as defined in claim 1, and

$R^4$ denotes methyl, ethyl, propyl, isopropyl, butyl, isobutyl, sec.-butyl, tert.-butyl, pentyl, isopentyl, neopentyl, sec.-pentyl, tert.-pentyl, hexyl, cyclopentyl, cyclohexyl, cycloheptyl, cyclopentylmethyl, cyclohexylmethyl, cycloheptylmethyl, phenyl, benzyl, phenylethyl, phenylpropyl, 2-, 3- or 4-pyridyl, 2-, 4- or 5-imidazolyl, (2-, 3- or 4-pyridyl)methyl, (2-, 4- or 5-imidazolyl)methyl, it being possible for the said alkyl and cycloalkyl radicals in each case to be substituted once or twice by amino, halogen, mono- or di-($C_1$-$C_4$)-alkylamino, carboxyl, guanidino, ($C_1$-$C_4$)-alkoxycarbonyl or a radical -$CONR^5R^6$ in which $R^5$ and $R^6$ are as defined above; it being possible for the said phenyl radicals to be substituted once or twice by ($C_1$-$C_4$)-alkyl, methoxy, hydroxyl, amino, aminomethyl, fluorine, chlorine or trifluoromethyl, and it being possible for the said heteroaromatic radicals in each case to be substituted by one or two radicals from the series comprising ($C_1$-$C_4$)-alkyl, methoxy, fluorine, chlorine, bromine or trifluoromethyl.

**3.** A process as claimed in one or more of claims 1 to 2, wherein

$R^1$ denotes methyl, ethyl, isopropyl, tert.-butyl, isobutyl, 2-hydroxyethyl, 2-methoxyethyl, carboxymethyl, 2-carboxyethyl, methoxycarbonylmethyl, 2-methoxycarbonylethyl, ethoxycarbonylmethyl, 2-ethoxycarbonylethyl, carbamoylmethyl, 2-carbamoylethyl, 2-aminoethyl, 2-dimethylaminoethyl, 2-morpholinoethyl, aminopropyl, aminoisobutyl, methylaminoisobutyl, dimethylaminoisobutyl, 2-piperidinoethyl, cyclopropyl, cyclobutyl, cyclopentyl or cyclohexyl, phenyl, 1- or 2-naphthyl, o-, m- or p-methylphenyl, o-, m- or p-hydroxyphenyl or o-, m- or p-aminophenyl, benzyl, 2-phenylethyl or a- or b-naphthylmethyl, unsubstituted or substituted heteroaryl, for example 2- or 3-pyrrolyl, 2-furyl, 2-thienyl, 2- or 4-imidazolyl, 1-methyl-2-, -4- or -5-imidazolyl, 1,3-thiazol-2-yl, 2-, 3- or 4-pyridyl, 1-oxido-2-, 3- or 4-pyridinio, 2-pyrazinyl, 2-, 4- or 5-pyrimidinyl, 2-, 3- or 4-quinolyl, 1-, 3- or 4-isoquinolyl or 2-benzoxazolyl, methoxy, ethoxy or n-butoxy or a radical -$NR^5R^6$, in which

$R^5$ and $R^6$ denote, identically or differently and independently of one another, hydrogen, methyl, ethyl, propyl, butyl, isobutyl, sec.-butyl, tert.-butyl, hydroxyethyl, methoxyethyl, aminoethyl, aminopropyl, benzyl- or pyridylmethyl, or in which

$R^5$ and $R^6$ form, together with the nitrogen atom carrying them, a pyrrolidine, piperidine, morpholine or piperazine ring;

A denotes a radical from the group comprising S, SO, $SO_2$, O, CO or CS;

D denotes a $CH_2$ group, an NH group or an $N(CH_3)$ group;

$R^3$ denotes aryl or arylmethyl, where aryl denotes phenyl, 2-thienyl, 2-pyridyl or 1-naphthyl, each of which is optionally substituted by hydroxyl, dihydroxyl, methoxy, dimethoxy, fluorine, chlorine or methylenedioxy;

B denotes a radical, which is linked N-terminal via -NH- with $R^1$-A-D-$CHR^3$-CO- and C-terminal via -CO- with

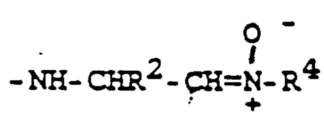,

of an amino acid from the series comprising phenylalanine, histidine, leucine, b-2-thienylalanine, b-3-thienylalanine, lysine, norvaline, 2-fluorophenylalanine, 3-fluorophenylalanine, 4-fluorophenylalanine, norleucine, S-methylcysteine, (1,3-dioxolan-2-yl)alanine, (1-, 3- or 4-pyrazolyl)alanine, 4-thiazolylalanine, (2-, 4- or 5-pyrimidyl)alanine;

$R^2$ denotes isobutyl, cyclohexylmethyl, benzyl or (1,3-dithiolan-2-yl)methyl; and

$R^4$ denotes methyl, ethyl, propyl, isopropyl, butyl, isobutyl, sec.-butyl, tert.-butyl, pentyl, isopentyl, neopentyl, sec.-pentyl, tert.-pentyl, hexyl, cyclopentyl, cyclohexyl, cyclopentylmethyl, cyclohexylmethyl, phenyl, benzyl, phenylethyl, 2-, 3- or 4-pyridyl, 2-imidazolyl, (2-, 3- or 4-pyridyl)-methyl, 2-imidazolylmethyl, it being possible for the said alkyl and cycloalkyl radicals in each case to be substituted once or twice by amino, fluorine, chlorine, methylamino, ethylamino, dimethylamino, diethylamino, carboxyl, guanidino, methoxycarbonyl, ethoxycarbonyl, tert.-butyloxycarbonyl or a radical $-CONR^5R^6$, in which $R^5$ and $R^6$ are defined as above, it being possible for the said phenyl radicals to be substituted once or twice by methyl, methoxy, hydroxyl, amino, aminomethyl, fluorine, chlorine or trifluoromethyl, and it being possible for the said heteroaromatic radicals to be substituted with, in each case, one or two radicals from the series comprising methyl, methoxy, fluorine, chlorine or trifluoromethyl.

**4.** The use of a compound as claimed in one of claims 1 to 3 as a medicine.

**5.** The use of a compound as claimed in one of claims 1 to 3 as a medicine for the treatment of high blood pressure.

**6.** The use of a compound as claimed in one of claims 1 to 3 as a medicine for the treatment of viral diseases.

**7.** A process for the preparation of a pharmaceutical composition containing a compound of the formula I prepared by one of the processes of claims 1 to 3, which comprises converting the latter together with a physiologically acceptable vehicle and, where appropriate, further auxiliaries and additives into a suitable form for administration.

**Revendications**

**Revendications pour les Etats contractants suivants : AT, BE, CH, DE, FR, GB, IT, LI, LU, NL, SE**

**1.** Composés de formule générale I

$$R^1-A-D-\overset{R^3}{\overset{|}{CH}}-\overset{O}{\overset{\|}{C}}-B-NH-\overset{R^2}{\overset{|}{CH}}-CH=\overset{|\bar{O}|^-}{\overset{|}{\underset{+}{N}}}-R^4 \qquad (I)$$

dans laquelle

$R^1$ représente un atome d'hydrogène ou un radical alkyle en $C_1$-$C_{18}$, cycloalkyle en $C_3$-$C_7$, qui peuvent être substitués chacun par un atome d'halogène ou par un groupe amino, hydroxy, marcapto, alcoxy en $C_1$-$C_4$, mono- ou dialkyl-($C_1$-$C_4$)-amino, carboxy, alcoxy-($C_1$-$C_4$)-carbonyle, phénoxy, phényl-alcoxy($C_1$-$C_4$), phényl-alcoxy($C_1$-$C_4$)-carbonyle ou par un reste $-CONR^5R^6$; un radical alcoxy en $C_1$-$C_4$, aryle en $C_6$-$C_{14}$, aryl($C_6$-$C_{14}$)-alkyle($C_1$-$C_4$), aryl($C_6$-$C_{14}$)-alcoxy($C_1$-$C_4$), le fragment aryle pouvant être dans chaque cas substitué par 1, 2 ou 3 atomes d'halogène ou groupes alkyle en $C_1$-$C_6$, amino, mono- ou dialky($C_1$-$C_4$)-amino, aminoalkyle en $C_1$-$C_4$, hydroxyalkyle en $C_1$-$C_4$, mono- ou dialkyl($C_1$-$C_4$)-amino-alkyle($C_1$-$C_4$), hydroxy, alcoxy en $C_1$-$C_4$, formyle, alcoxy($C_1$-$C_4$)-carbonyle, carboxamido, mono- ou dialkyl($C_1$-$C_4$)-aminocarbonyle ou nitro ou par un groupe méthylènedioxy; un radical Het ou Het-alkyle-($C_1$-$C_4$), Het représentant un cycle hétérocyclique à 5, 6 ou 7 chaînons, qui peut être soudé à un noyau benzénique et être soit aromatique, soit partiellement ou totalement hydrogéné, et qui contient en tant qu'hétéroélément(s) un ou deux chaînons choisis parmi N, O, S, NO, SO ou $SO_2$ et qui peut être substitué par un ou deux substituants choisis parmi des halogènes et des

groupes alkyle en $C_1$-$C_4$, alcoxy en $C_1$-$C_4$, alcoxy($C_1$-$C_4$)-carbonyle, hydroxy, amino, mono- ou dialkyl($C_1$-$C_4$)-amino; ou un radical -$NR^5R^6$;

$R^5$ et $R^6$ étant identiques ou différents et représentant, indépendamment l'un de l'autre un atome d'hydrogène ou un radical alkyle en $C_1$-$C_8$ qui peut être substitué par un groupe amino, alkyl($C_1$-$C_4$)-amino, dialkyl-($C_1$-$C_4$)-amino, hydroxy ou alcoxy en $C_1$-$C_4$, un radical cycloalkyle en $C_3$-$C_7$, mercapto, alkyl($C_1$-$C_4$)-thio, phénylthio, alcoxy($C_1$-$C_4$)-carbonyle, carboxy, un radical aryle en $C_6$-$C_{14}$ qui peut être substitué sur le fragment aryle comme décrit pour $R^1$, un radical Het ou Het-alkyle en $C_1$-$C_4$, Het étant défini comme décrit pour $R^1$; ou

$R^5$ et $R^6$ formant conjointement avec l'atome d'azote qui les porte un cycle à 5-12 chaînons qui peut être mono- ou bicyclique et peut encore contenir, en tant qu'autres chaînons de cycle, 1 ou 2 atomes d'azote, un atome de soufre ou un atome d'oxygène, et qui peut être substitué par un groupe alkyle en $C_1$-$C_4$;

A représente S, SO, $SO_2$, O, CO, CS ou une liaison directe;

D représente un groupe $CH_2$ ou un groupe -$NR^7$-, $R^7$ pouvant être un atome d'hydrogène ou un radical alkyle en $C_1$-$C_4$;

$R^3$ représente un radical aryle en $C_6$-$C_{14}$, aryl($C_6$-$C_{14}$)-alkyle($C_1$-$C_4$),le fragment aryle pouvant dans chaque cas être substitué par 1, 2 ou 3 substituants comme décrit à propos de $R^1$, un radical thiényle ou thiényl-alkyle-($C_1$-$C_4$), le reste thiophène pouvant être substitué dans chaque cas par un ou deux radicaux alkyle en $C_1$-$C_4$, alcoxy en $C_1$-$C_4$ ou atomes d'halogène; un radical 2-, 3- ou 4-pyridyle ou 2-, 3- ou 4-pyridyl-alkyle-($C_1$-$C_4$), le reste pyridine pouvant être substitué par un ou deux groupes alkyle en $C_1$-$C_4$, alcoxy en $C_1$-$C_4$ ou atomes d'halogène;

B représente un radical, lié à l'extrémité N-terminale par -$NR^8$- à $R^1$-A-D-$CHR^3$-CO- et, à l'extrémité C-terminale, par -CO- à

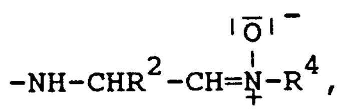

d'un aminoacide de la série phénylalanine, histidine, tyrosine, tryptophane, méthionine, leucine, isoleucine, asparagine, acide aspartique, $\beta$-2-thiénylalanine, $\beta$-3-thiénylalanine, $\beta$-2-furylalanine, $\beta$-3-furylalanine, lysine, ornithine, valine, alanine, acide 2,4-diaminobutyrique, arginine, 4-chlorophénylalanine,méthioninesulfone, méthioninesulfoxyde, 2-pyridylalanine, 3-pyridylalanine, cyclohexylalanine, cyclohexylglycine, im-méthylhistidine, O-méthyltyrosine, O-benzyltyrosine, O-tert-butyltyrosine, phénylglycine, 1-naphtylalanine, 2-naphtylalanine, 4-nitrophénylalanine, norvaline, $\beta$-2-benzo[b]thiénylalanine, $\beta$-3-benzo[b]thiénylalanine, 2-fluorophénylalanine, 3-fluorophénylalanine, 4-fluorophénylalanine, norleucine, cystéine, S-méthyl-cystéine, acide 1,2,3,4-tétrahydroisoquinoléine-3-carboxylique, homophénylalanine, DOPA, O-diméthyl-DOPA, acide 2-amino-4-(2-thiényl)-butyrique, benzodioxol-5-yl-alanine, N-méthyl-histidine, acide 2-amino-4-(3-thiényl)-butyrique, 3-(2-thiényl)-sérine, (Z)-déhydrophénylalanine, (E)-déhydrophénylalanine, (1,3-dioxolanne-2-yl)alanine, N-pyrrolylalanine, (1-, 3- ou 4-pyrazolyl)alanine, (4-thiazolyl)alanine, (2-, 4- ou 5-pyrimidyl)alanine, cyclopentylglycine, tert-butylglycine ou phényl-sérine;

et $R^8$ représente un atome d'hydrogène ou un groupe alkyle en $C_1$-$C_6$, formyle, alcoxy-($C_1$-$C_6$)-carbonyle ou benzyloxycarbonyle;

$R^2$ représente un atome d'hydrogène ou un radical alkyle en $C_1$-$C_{10}$, cycloalkyle en $C_4$-$C_7$, cycloalkyl($C_4$-$C_7$)-alkyle-($C_1$-$C_4$), aryle en $C_6$-$C_{14}$, aryl($C_6$-$C_{14}$)-alkyle($C_1$-$C_4$) ou (radical hétérocyclique)-alkyle($C_1$-$C_4$), l'hétérocycle présentant 4-7 chaînons formant le cycle, dont 1 ou 2 atomes de soufre et/ou d'oxygène; et

$R^4$ représente un radical alkyle en $C_1$-$C_8$, cycloalkyle en $C_3$-$C_8$, cycloalkyl($C_3$-$C_8$)-alkyle-($C_1$-$C_4$), qui peuvent être substitués chacun 1, 2 ou 3 fois par un ou des atomes d'halogène ou groupes amino, hydroxy, mercapto, alcoxy en $C_1$-$C_4$, mono- ou dialkyl-($C_1$-$C_4$)-amino, carboxy, guanidino, alcoxy($C_1$-$C_4$)-carbonyle ou par un reste -$CONR^5R^6$, dans lequel $R^5$ et $R^6$ sont tels que définis plus haut; un radical aryle en $C_6$-$C_{14}$, aryl($C_6$-$C_{14}$)-alkyle($C_1$-$C_4$), le reste aryle étant tel que décrit à propos de $R^1$ et

la chaîne alkyle étant telle que décrite plus haut pour $R^4$; un radical Het ou Het-alkyle-($C_1$-$C_4$), Het étant défini comme décrit à propos de $R^1$, et la chaîne alkyle pouvant être substituée comme décrit plus haut pour $R^4$;

ainsi que leurs sels physiologiquement acceptables.

**2.** Composé de formule I selon la revendication 1, caractérisé en ce que

$R^1$ représente le radical méthyle, éthyle, isopropyle, tert-butyle, isobutyle, 2-hydroxyéthyle, 2-méthoxyéthyle, carboxyméthyle, 2-carboxyéthyle, méthoxycarbonylméthyle, 2-méthoxycarbonyléthyle, éthoxycarbonylméthyle, 2-éthoxycarbonyléthyle, carbamoylméthyle, 2-carbamoyléthyle, 2-aminoéthyle, 2-diméthylaminoéthyle, 2-morpholinoéthyle, aminoisobutyle, 2-pipéridinoéthyle, aminopropyle, diméthylaminopropyle, méthylaminopropyle, pipéridinopropyle, morpholinopropyle, méthylaminoisobutyle, diméthylaminoisobutyle, pipéridinoisobutyle, morpholinoisobutyle, cyclopropyle, cyclobutyle, cyclopentyle, ou cyclohexyle, phényle, 1- ou 2-naphtyle, o-, m- ou p-méthylphényle, o-, m- ou p-hydroxyphényle ou o-, m- ou p-aminophényle, benzyle, 2-phényléthyle ou $\alpha$- ou $\beta$-naphtylméthyle, un radical hétéroaryle substitué ou non substitué, par exemple le radical 2- ou 3-pyrrolyle, 2-furyle, 2-thiényle, 2- ou 4-imidazolyle, 1-méthyl-2-, -4- ou -5-imidazolyle, 1,3-thiazol-2-yle, 2-, 3- ou 4-pyridyle, 1-oxydo-2-, -3- ou -4-pyridinio, 2-pyrazinyle, 2-, 4- ou 5-pyrimidinyle, 2-, 3- ou 4-quinolyle, 1-, 3- ou 4-isoquinolyle ou 2-benzoxazolyle, méthoxy, éthoxy ou n-butoxy, ou un radical $-NR^5R^6$, dans lequel

$R^5$ et $R^6$ sont identiques ou différents et représentent, indépendamment l'un de l'autre, un atome d'hydrogène ou le radical méthyle, éthyle, propyle, butyle, isobutyle, sec-butyle, tert-butyle, aminoéthyle, aminopropyle, méthylaminoéthyle, diméthylaminoéthyle, méthylaminopropyle, diméthylaminopropyle, hydroxyéthyle, méthoxyéthyle, cyclohexylméthyle, mercaptoéthyle, méthylthioéthyle, benzyle, 1-phénéthyle, 2-phénéthyle, 2-(3,4-diméthoxy)-phénéthyle, 2-pyridylméthyle, 3-pyridylméthyle, ou dans lequel

$R^5$ et $R^6$ forment, conjointement avec l'atome d'azote qui les porte, un cycle pyrrolidine, pipéridine, azépine, azocine, morpholine, pipérazine, 4-méthylpipérazine, 4-éthylpipérazine, homopipérazine ou thiomorpholine;

A représente S, SO, $SO_2$, O, CO ou CS;

D représente le groupe $CH_2$, le groupe NH ou le groupe $N(CH_3)$;

$R^3$ représente un radical phényle, 2-thiényle, 2-pyridyle, 1-naphtyle, phényl-alkyle($C_1$-$C_4$), 2-thiényl-alkyle-($C_1$-$C_4$), 2-pyridyl-alkyle($C_1$-$C_4$), 1-naphtyl-alkyle-($C_1$-$C_4$), qui sont éventuellement substitués chacun par 1, 2 ou 3 substituants choisis parmi les atomes de fluor et de chlore et les groupes méthyle, éthyle, isopropyle, tert-butyle, méthoxy, hydroxy et nitro, ou par un reste méthylènedioxy;

B représente un reste, lié à l'extrémité N-terminale par -NH- à $R^1$-A-D-$CHR^3$-CO- et, à l'extrémité C-terminale, par -CO- à

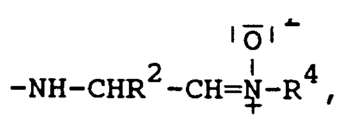

d'un aminoacide de la série phénylalanine, histidine, tyrosine, tryptophane, méthionine, leucine, isoleucine, asparagine, acide aspartique, $\beta$-2-thiénylalanine, $\beta$-3-thiénylalanine, $\beta$-2-furylalanine, $\beta$-3-furylalanine, lysine, ornithine, acide 2,4-diaminobutyrique, arginine, norvaline, 4-chlorophénylalanine, méthioninesulfone, méthioninesulfoxyde, 2-pyridylalanine, 3-pyridylalanine, cyclohexylalanine, cyclohexylglycine, im-méthylhistidine, O-méthyltyrosine, O-benzyltyrosine, O-tert-butyltyrosine, phénylglycine, 1-naphtylalanine, 2-naphtylalanine, 4-nitrophénylalanine, norleucine, valine, alanine, cystéine, S-méthylcystéine, N-méthylhistidine, benzodioxol-5-yl-alanine, acide 1,2,3,4-tétrahyd roisoquinoléine-3-carboxylique, homophénylalanine, acide 2-amino-4-(3-thiényl)-butyrique, (Z)-déhydrophénylalanine, (E)-déhydrophénylalanine, (1,3-dioxolanne-2-yl)-alanine, (4-thiazolyl)alanine, (2-, 4- ou 5-pyrimidyl)alanine, (1-, 3- ou 4-pyrazolyl)alanine, (2-, 3- ou 4-fluorophényl)alanine, cyclopentylglycine, tert-butylglycine et phénylsérine;

$R^2$ est tel que défini dans la revendication 1,

$R^4$ représente un radical méthyle, éthyle, propyle, isopropyle, butyle, isobutyle, sec-butyle,

tert-butyle, pentyle, isopentyle, néopentyle, sec-pentyle, tert-pentyle, hexyle, cyclopentyle, cyclohexyle, cycloheptyle, cyclopentylméthyle, cyclohexylméthyle, cycloheptylméthyle, phényle, benzyle, phényléthyle, phénylpropyle, 2-, 3- ou 4-pyridyle, 2-, 4- ou 5-imidazolyle, (2-, 3- ou 4-pyridyl)méthyle, (2-, 4- ou 5-imidazolyl)méthyle, les radicaux alkyle et cycloalkyle cités pouvant être chacun 1 ou 2 fois substitués par un ou des atomes d'halogène ou groupes amino, mono- ou dialkyl($C_1$-$C_4$)-amino, carboxy, guanidino, alcoxy($C_1$-$C_4$)-carbonyle ou par un radical -CON$R^5R^6$, dans lequel $R^5$ et $R^6$ sont tels que définis plus haut; les radicaux phényle cités pouvant être 1 ou 2 fois substitués par un ou des atomes de fluor ou de chlore ou groupes alkyle en $C_1$-$C_4$, méthoxy, hydroxy, amino, aminométhyle ou trifluorométhyle, et les radicaux hétéroaromatiques cités pouvant être substitués chacun par 1 ou 2 substituants choisis parmi les atomes de fluor, chlore et brome ou des groupes alkyle en $C_1$-$C_4$, méthoxy ou trufluorométhyle,

et sels physiologiquement acceptables de celui-ci.

**3.** Composé de formule I selon la revendication 1 ou 2, caractérisé en ce que

$R^1$ représente le radical méthyle, éthyle, isopropyle, tert-butyle, isobutyle, 2-hydroxyéthyle, 2-méthoxyéthyle, carboxyméthyle, 2-carboxyéthyle, méthoxycarbonylméthyle, 2-méthoxycarbonyléthyle, éthoxycarbonylméthyle, 2-éthoxycarbonyléthyle, carbamoylméthyle, 2-carbamoyléthyle, 2-aminoéthyle, 2-diméthylaminoéthyle, 2-morpholinoéthyle, aminopropyle, aminoisobutyle, méthylaminoisobutyle, diméthylaminoisobutyle, 2-pipéridinoéthyle, cyclopropyle, cyclobutyle, cyclopentyle ou cyclohexyle, phényle, 1- ou 2-naphtyle, o-, m- ou p-méthylphényle, o-, m- ou p-hydroxyphényle ou o-, m- ou p-aminophényle, benzyle, 2-phényléthyle ou $\alpha$- ou $\beta$-naphtylméthyle, un radical hétéroaryle substitué ou non substitué, par exemple 2- ou 3-pyrrolyle, 2-furyle, 2-thiényle, 2- ou 4-imidazolyle, 1-méthyl-2-, -4- ou -5-imidazolyle, 1,3-thiazol-2-yle, 2-, 3- ou 4-pyridyle, 1-oxydo-2-, -3- ou -4-pyridinio, 2-pyrazinyle, 2-, 4- ou 5-pyrimidinyle, 2-, 3- ou 4-quinolyle, 1-, 3- ou 4-isoquinolyle ou 2-benzoxazolyle, méthoxy, éthoxy ou n-butoxy, ou un radical -N$R^5R^6$, dans lequel

$R^5$ et $R^6$ sont identiques ou différents et représentent, indépendamment l'un de l'autre, un atome d'hydrogène ou le groupe méthyle, éthyle, propyle, butyle, isobutyle, sec-butyle, tert-butyle, hydroxyéthyle, méthoxyéthyle, aminoéthyle, aminopropyle, benzyle ou pyridylméthyle, ou dans lequel

$R^5$ et $R^6$ forment, conjointement avec l'atome d'azote qui les porte, un cycle pyrrolidine, pipéridine, morpholine ou pipérazine;

A représente S, SO, $SO_2$, O, CO ou CS;

D représente le groupe CH, le groupe NH ou le groupe N($CH_3$);

$R^3$ représente un radical aryle ou arylméthyle, le fragment aryle étant les radicaux phényle, 2-thiényl, 2-pyridyle, 1-naphtyle qui sont chacun éventuellement substitués par un atome de fluor ou de chlore ou un groupe hydroxy, dihydroxy, méthoxy, diméthoxy ou méthylènedioxy;

B représente un reste, lié à l'extrémité N-terminale par -NH- à $R^1$-A-D-CH$R^3$-CO- et, à l'extrémité C-terminale, par -CO- à

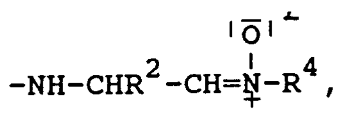

d'un aminoacide de la série phénylalanine, histidine, leucine, $\beta$-2-thiénylalanine, $\beta$-3-thiénylalanine, lysine, norvaline, 2-fluorophénylalanine, 3-fluorophénylalanine, 4-fluorophénylalanine, norleucine, S-méthylcystéine, (1,3-dioxolanne-2-yl)alanine, (1-, 3- ou 4-pyrazolyl)alanine, 4-thiazolylalanine, (2-, 4- ou 5-pyrimidyl)alanine;

$R^2$ représente le radical isobutyle, cyclohexylméthyle, benzyle ou (1,3-dithiolanne-2-yl)-méthyle; et

$R^4$ représente un radical méthyle, éthyle, propyle, isopropyle, butyle, isobutyle, sec-butyle, tert-butyle, pentyle, isopentyle, néopentyle, sec-pentyle, tert-pentyle, hexyle, cyclopentyle, cyclohexyle, cyclopentylméthyle, cyclohexylméthyle, phényle, benzyle, phényléthyle, 2-, 3- ou 4-pyridyle, 2-imidazolyle, (2-, 3- ou 4-pyridyl)méthyle, 2-imidazolylméthyle, les radicaux alkyle et cycloalkyle cités pouvant être chacun 1 ou 2 fois substitués par un ou des atomes de fluor ou de chlore ou groupes amino, méthylamino, éthylamino, diméthylamino, diéthylamino, carboxy, guanidino, méthoxycarbonyle, éthoxycarbonyle, tert-butyloxycarbonyle ou par un radical $-CONR^5R^6$, dans lequel $R^5$ et $R^6$ sont tels que définis plus haut, les radicaux phényle cités pouvant être 1 ou 2 fois substitués par un ou des atomes de fluor ou de chlore ou groupes méthyle, méthoxy, hydroxy, amino, aminométhyle ou trifluorométhyle, et les radicaux hétéroaromatiques cités pouvant être chacun substitué par 1 ou 2 atomes de fluor ou de chlore ou radicaux méthyle, méthoxy ou trifluorométhyle,

et sels physiologiquement acceptables de celui-ci.

**4.** Procédé pour la préparation d'un composé de formule I, selon une ou plusieurs des revendications 1 à 3, caractérisé en ce que

a) on fait réagir un composé de formule générale II

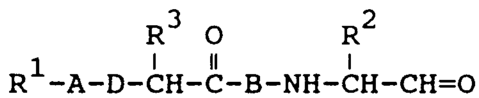

(II)

dans laquelle $R^1$, $R^2$, $R^3$, A, B et D ont la même signification que dans la formule I, avec un dérivé d'hydroxylamine de formule générale III

$HO-NH-R^4$ (III)

dans laquelle $R^4$ a la même signification que dans la formule I, dans un solvant organique, tel que l'éther diéthylique, l'éthanol, le benzène, le toluène ou dans l'eau sans aucun solvant, à une température comprise entre -20 °C et le point d'ébullition du solvant, de préférence entre -20 °C et +60 °C, en présence ou en absence d'un acide, par exemple l'acide chlorhydrique ou l'acide acétique, qui peut être introduit par exemple sous forme des sels des hydroxylamines de formule III, en présence ou en absence d'une base auxiliaire, telle que l'hydrogénocarbonate de sodium, l'acétate de sodium, le carbonate de sodium, l'éthanolate de sodium ou l'hydroxyde de potassium, ainsi qu'en présence ou en absence d'un agent déshydratant, tel que le chlorure de calcium ou un tamis moléculaire, et éventuellement on élimine un ou des groupes protecteurs introduits temporairement pour la protection d'autres groupes fonctionnels, ou

b) on assemble un fragment à groupe carboxy terminal, ou un dérivé réactif de celui-ci, avec un fragment correspondant à groupe amino libre, éventuellement on élimine un ou des groupes protecteurs introduits temporairement pour la protection d'autres groupes fonctionnels, et éventuellement on convertit le composé ainsi obtenu en un de ses sels physiologiquement acceptable.

**5.** Composé de formule I, selon une ou plusieurs des revendications 1 à 3, pour utilisation en tant que médicament.

**6.** Composé de formule I, selon une ou plusieurs des revendications 1 à 3, pour utilisation en tant que médicament dans le traitement de l'hypertension artérielle.

**7.** Composé de formule I, selon une ou plusieurs des revendications 1 à 3, pour utilisation en tant que médicament dans le traitement de maladies virales.

**8.** Composition pharmaceutique contenant un composé de formule II selon une ou plusieurs des revendications 1 à 3.

**Revendications pour les Etats contractants suivants : ES, GR**

**1.** Procédé pour la préparation d'un composé de formule I

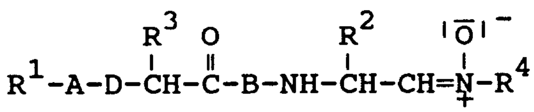

(I)

dans laquelle

- $R^1$ représente un atome d'hydrogène ou un radical alkyle en $C_1$-$C_{18}$, cycloalkyle en $C_3$-$C_7$, qui peuvent être substitués chacun par un atome d'halogène ou par un groupe amino, hydroxy, marcapto, alcoxy en $C_1$-$C_4$, mono- ou dialkyl-($C_1$-$C_4$)-amino, carboxy, alcoxy-($C_1$-$C_4$)-carbonyle, phénoxy, phényl-alcoxy($C_1$-$C_4$), phényl-alcoxy($C_1$-$C_4$)-carbonyle ou par un reste -$CONR^5R^6$; un radical alcoxy en $C_1$-$C_4$, aryle en $C_6$-$C_{14}$, aryl($C_6$-$C_{14}$)-alkyle($C_1$-$C_4$), aryl($C_6$-$C_{14}$)-alcoxy($C_1$-$C_4$), le fragment aryle pouvant être dans chaque cas substitué par 1, 2 ou 3 atomes d'halogène ou groupes alkyle en $C_1$-$C_6$, amino, mono- ou dialky($C_1$-$C_4$)-amino, aminoalkyle en $C_1$-$C_4$, hydroxyalkyle en $C_1$-$C_4$, mono- ou dialkyl($C_1$-$C_4$)-amino-alkyle($C_1$-$C_4$), hydroxy, alcoxy en $C_1$-$C_4$, formyle, alcoxy($C_1$-$C_4$)-carbonyle, carboxamido, mono- ou dialkyl($C_1$-$C_4$)-aminocarbonyle ou nitro ou par un groupe méthylènedioxy; un radical Het ou Het-alkyle-($C_1$-$C_4$), Het représentant un cycle hétérocyclique à 5, 6 ou 7 chaînons, qui peut être soudé à un noyau benzénique et être soit aromatique, soit partiellement ou totalement hydrogéné, et qui contient en tant qu'hétéroélément(s) un ou deux chaînons choisis parmi N, O, S, NO, SO ou $SO_2$ et qui peut être substitué par un ou deux substituants choisis parmi des halogènes et des groupes alkyle en $C_1$-$C_4$, alcoxy en $C_1$-$C_4$, alcoxy($C_1$-$C_4$)-carbonyle, hydroxy, amino, mono- ou dialkyl($C_1$-$C_4$)-amino; ou un radical -$NR^5R^6$;
- $R^5$ et $R^6$ étant identiques ou différents et représentant, indépendamment l'un de l'autre, un atome d'hydrogène ou un radical alkyle en $C_1$-$C_8$ qui peut être substitué par un groupe amino, alkyl($C_1$-$C_4$)-amino, dialkyl-($C_1$-$C_4$)-amino, hydroxy ou alcoxy en $C_1$-$C_4$, un radical cycloalkyle en $C_3$-$C_7$, mercapto, alkyl($C_1$-$C_4$)-thio, phénylthio, alcoxy($C_1$-$C_4$)-carbonyle, carboxy, un radical aryle en $C_6$-$C_{14}$ qui peut être substitué sur le fragment aryle comme décrit pour $R^1$, un radical Het ou Het-alkyle en $C_1$-$C_4$, Het étant défini comme décrit pour $R^1$; ou
- $R^5$ et $R^6$ formant conjointement avec l'atome d'azote qui les porte un cycle à 5-12 chaînons qui peut être mono- ou bicyclique et peut encore contenir, en tant qu'autres chaînons de cycle, 1 ou 2 atomes d'azote, un atome de soufre ou un atome d'oxygène, et qui peut être substitué par un groupe alkyle en $C_1$-$C_4$;
- A représente S, SO, $SO_2$, O, CO, CS ou une liaison directe;
- D représente un groupe $CH_2$ ou un groupe -$NR^7$-, $R^7$ pouvant être un atome d'hydrogène ou un radical alkyle en $C_1$-$C_4$;
- $R^3$ représente un radical aryle en $C_6$-$C_{14}$, aryl($C_6$-$C_{14}$)-alkyle($C_1$-$C_4$),le fragment aryle pouvant dans chaque cas être substitué par 1, 2 ou 3 substituants comme décrit à propos de $R^1$, un radical thiényle ou thiényl-alkyle-($C_1$-$C_4$), le reste thiophène pouvant être substitué dans chaque cas par un ou deux radicaux alkyle en $C_1$-$C_4$, alcoxy en $C_1$-$C_4$ ou atomes d'halogène; un radical 2-, 3- ou 4-pyridyle ou 2-, 3- ou 4-pyridyl-alkyle-($C_1$-$C_4$), le reste pyridine pouvant être substitué par un ou deux groupes alkyle en $C_1$-$C_4$, alcoxy en $C_1$-$C_4$ ou atomes d'halogène;
- B représente un radical, lié à l'extrémité N-terminale par -$NR^8$- à $R^1$-A-D-$CHR^3$-CO- et, à l'extrémité C-terminale, par -CO- à

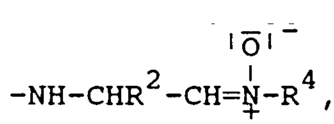

d'un aminoacide de la série phénylalanine, histidine, tyrosine, tryptophane, méthionine, leucine, isoleucine, asparagine, acide aspartique, $\beta$-2-thiénylalanine, $\beta$-3-thiénylalanine,

β-2-furylalanine, β-3-furylalanine, lysine, ornithine, valine, alanine, acide 2,4-diaminobutyrique, arginine, 4-chlorophénylalanine,méthioninesulfone, méthioninesulfoxyde, 2-pyridylalanine, 3-pyridylalanine, cyclohexylalanine, cyclohexylglycine, im-méthylhistidine, O-méthyltyrosine, O-benzyltyrosine, O-tert-butyltyrosine, phénylglycine, 1-naphtylalanine, 2-naphtylalanine, 4-nitrophénylalanine, norvaline, β-2-benzo[b]thiénylalanine, β-3-benzo[b]thiénylalanine, 2-fluorophénylalanine, 3-fluorophénylalanine, 4-fluorophénylalanine, norleucine, cystéine, S-méthyl-cystéine, acide 1,2,3,4-tétrahydroisoquinoléine-3-carboxylique, homophénylalanine, DOPA, O-diméthyl-DOPA, acide 2-amino-4-(2-thiényl)-butyrique, benzodioxol-5-yl-alanine, N-méthyl-histidine, acide 2-amino-4-(3-thiényl)-butyrique, 3-(2-thiényl)-sérine, (Z)-déhydrophénylalanine, (E)-déhydrophénylalanine, (1,3-dioxolanne-2-yl)alanine, N-pyrrolylalanine, (1-, 3- ou 4-pyrazolyl)alanine, (4-thiazolyl)alanine, (2-, 4- ou 5-pyrimidyl)alanine, cyclopentylglycine, tert-butylglycine ou phényl-sérine;

et $R^8$ représente un atome d'hydrogène ou un groupe alkyle en $C_1$-$C_6$, formyle, alcoxy-($C_1$-$C_6$)-carbonyle ou benzyloxycarbonyle;

$R^2$ représente un atome d'hydrogène ou un radical alkyle en $C_1$-$C_{10}$, cycloalkyle en $C_4$-$C_7$, cycloalkyl($C_4$-$C_7$)-alkyle-($C_1$-$C_4$), aryle en $C_6$-$C_{14}$, aryl($C_6$-$C_{14}$)-alkyle($C_1$-$C_4$) ou (radical hétérocyclique)-alkyle($C_1$-$C_4$), l'hétérocycle présentant 4-7 chaînons formant le cycle, dont 1 ou 2 atomes de soufre et/ou d'oxygène; et

$R^4$ représente un radical alkyle en $C_1$-$C_8$, cycloalkyle en $C_3$-$C_8$, cycloalkyl($C_3$-$C_8$)-alkyle-($C_1$-$C_4$), qui peuvent être substitués chacun 1, 2 ou 3 fois par un ou des atomes d'halogène ou groupes amino, hydroxy, mercapto, alcoxy en $C_1$-$C_4$, mono- ou dialkyl-($C_1$-$C_4$)-amino, carboxy, guanidino, alcoxy($C_1$-$C_4$)-carbonyle ou par un reste $-CONR^5R^6$, dans lequel $R^5$ et $R^6$ sont tels que définis plus haut; un radical aryle en $C_6$-$C_{14}$, aryl($C_6$-$C_{14}$)-alkyle($C_1$-$C_4$), le reste aryle étant tel que décrit à propos de $R^1$ et la chaîne alkyle étant telle que décrite plus haut pour $R^4$; un radical Het ou Het-alkyle-($C_1$-$C_4$), Het étant défini comme décrit à propos de $R^1$, et la chaîne alkyle pouvant être substituée comme décrit plus haut pour $R^4$;

ainsi que de ses sels physiologiquement acceptables, caractérisé en ce que

a) on fait réagir un composé de formule générale II

$$R^1\text{-A-D-}\underset{}{\overset{R^3}{\overset{|}{C}}}H\text{-}\overset{O}{\overset{\|}{C}}\text{-B-NH-}\overset{R^2}{\overset{|}{C}}H\text{-CH=O} \qquad \text{(II)}$$

dans laquelle $R^1$, $R^2$, $R^3$, A, B et D ont la même signification que dans la formule I, avec un dérivé d'hydroxylamine de formule générale III

$HO\text{-}NH\text{-}R^4$ (III)

dans laquelle $R^4$ a la même signification que dans la formule I, dans un solvant organique, tel que l'éther diéthylique, l'éthanol, le benzène, le toluène ou dans l'eau sans aucun solvant, à une température comprise entre -20 °C et le point d'ébullition du solvant, de préférence entre -20 °C et +60 °C, en présence ou en absence d'un acide, par exemple l'acide chlorhydrique ou l'acide acétique, qui peut être introduit par exemple sous forme des sels des hydroxylamines de formule III, en présence ou en absence d'une base auxiliaire, telle que l'hydrogénocarbonate de sodium, l'acétate de sodium, le carbonate de sodium, l'éthanolate de sodium ou l'hydroxyde de potassium, ainsi qu'en présence ou en absence d'un agent déshydratant, tel que le chlorure de calcium ou un tamis moléculaire, et éventuellement on élimine un ou des groupes protecteurs introduits temporairement pour la protection d'autres groupes fonctionnels, ou

b) on assemble un fragment à groupe carboxy terminal, ou un dérivé réactif de celui-ci, avec un fragment correspondant à groupe amino libre, éventuellement on élimine un ou des groupes protecteurs introduits temporairement pour la protection d'autres groupes fonctionnels, et éventuellement on convertit le composé ainsi obtenu en un de ses sels physiologiquement acceptable.

**2.** Procédé selon la revendication 1, caractérisé en ce que

$R^1$ représente le radical méthyle, éthyle, isopropyle, tert-butyle, isobutyle, 2-hydroxyéthyle,

2-méthoxyéthyle, carboxyméthyle, 2-carboxyéthyle, méthoxycarbonylméthyle, 2-méthoxycarbonyléthyle, éthoxycarbonylméthyle, 2-éthoxycarbonyléthyle, carbamoylméthyle, 2-carbamoyléthyle, 2-aminoéthyle, 2-diméthylaminoéthyle, 2-morpholinoéthyle, aminoisobutyle, 2-pipéridinoéthyle, aminopropyle, diméthylaminopropyle, méthylaminopropyle, pipéridinopropyle, morpholinopropyle, méthylaminoisobutyle, diméthylaminoisobutyle, pipéridinoisobutyle, morpholinoisobutyle, cyclopropyle, cyclobutyle, cyclopentyle, ou cyclohexyle, phényle, 1- ou 2-naphtyle, o-, m- ou p-méthylphényle, o-, m- ou p-hydroxyphényle ou o-, m- ou p-aminophényle, benzyle, 2-phényléthyle ou α- ou β-naphtylméthyle, un radical hétéroaryle substitué ou non substitué, par exemple le radical 2- ou 3-pyrrolyle, 2-furyle, 2-thiényle, 2- ou 4-imidazolyle, 1-méthyl-2-, -4- ou -5-imidazolyle, 1,3-thiazol-2-yle, 2-, 3- ou 4-pyridyle, 1-oxydo-2-, -3- ou -4-pyridinio, 2-pyrazinyle, 2-, 4- ou 5-pyrimidinyle, 2-, 3- ou 4-quinolyle, 1-, 3- ou 4-isoquinolyle ou 2-benzoxazolyle, méthoxy, éthoxy ou n-butoxy, ou un radical $-NR^5R^6$, dans lequel

$R^5$ et $R^6$ sont identiques ou différents et représentent, indépendamment l'un de l'autre, un atome d'hydrogène ou le radical méthyle, éthyle, propyle, butyle, isobutyle, sec-butyle, tert-butyle, aminoéthyle, aminopropyle, méthylaminoéthyle, diméthylaminoéthyle, méthylaminopropyle, diméthylaminopropyle, hydroxyéthyle, méthoxyéthyle, cyclohexylméthyle, mercaptoéthyle, méthylthioéthyle, benzyle, 1-phénéthyle, 2-phénéthyle, 2-(3,4-diméthoxy)-phénéthyle, 2-pyridylméthyle, 3-pyridylméthyle, ou dans lequel

$R^5$ et $R^6$ forment, conjointement avec l'atome d'azote qui les porte, un cycle pyrrolidine, pipéridine, azépine, azocine, morpholine, pipérazine, 4-méthylpipérazine, 4-éthylpipérazine, homopipérazine ou thiomorpholine;

A représente S, SO, $SO_2$, O, CO ou CS;

D représente le groupe $CH_2$, le groupe NH ou le groupe $N(CH_3)$;

$R^3$ représente un radical phényle, 2-thiényle, 2-pyridyle, 1-naphtyle, phényl-alkyle($C_1$-$C_4$), 2-thiényl-alkyle($C_1$-$C_4$), 2-pyridyl-alkyle($C_1$-$C_4$), 1-naphtyl-alkyle($C_1$-$C_4$), qui sont éventuellement substitués chacun par 1, 2 ou 3 substituants choisis parmi les atomes de fluor et de chlore et les groupes méthyle, éthyle, isopropyle, tert-butyle, méthoxy, hydroxy et nitro, ou par un reste méthylènedioxy;

B représente un reste, lié à l'extrémité N-terminale par -NH- à $R^1$-A-D-$CHR^3$-CO- et, à l'extrémité C-terminale, par -CO- à

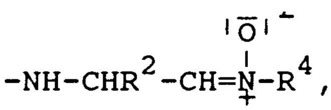

d'un aminoacide de la série phénylalanine, histidine, tyrosine, tryptophane, méthionine, leucine, isoleucine, asparagine, acide aspartique, β-2-thiénylalanine, β-3-thiénylalanine, β-2-furylalanine, β-3-furylalanine, lysine, ornithine, acide 2,4-diaminobutyrique, arginine, norvaline, 4-chlorophénylalanine, méthioninesulfone, méthioninesulfoxyde, 2-pyridylalanine, 3-pyridylalanine, cyclohexylalanine, cyclohexylglycine, im-méthylhistidine, O-méthyltyrosine, O-benzyltyrosine, O-tert-butyltyrosine, phénylglycine, 1-naphtylalanine, 2-naphtylalanine, 4-nitrophénylalanine, norleucine, valine, alanine, cystéine, S-méthylcystéine, N-méthylhistidine, benzodioxol-5-yl-alanine, acide 1,2,3,4-tétrahydroisoquinoléine-3-carboxylique, homophénylalanine, acide 2-amino-4-(3-thiényl)butyrique, (Z)-déhydrophénylalanine, (E)-déhydrophénylalanine, (1,3-dioxolanne-2-yl)alanine, (4-thiazolyl)-alanine, (2-, 4- ou 5-pyrimidyl)alanine, (1-, 3- ou 4-pyrazolyl)alanine, (2-, 3- ou 4-fluorophényl)alanine, cyclopentylglycine, tert-butylglycine et phénylsérine;

$R^2$ est tel que défini dans la revendication 1,

$R^4$ représente un radical méthyle, éthyle, propyle, isopropyle, butyle, isobutyle, sec-butyle, tert-butyle, pentyle, isopentyle, néopentyle, sec-pentyle, tert-pentyle, hexyle, cyclopentyle, cyclohexyle, cycloheptyle, cyclopentylméthyle, cyclohexylméthyle, cycloheptylméthyle, phényle, benzyle, phényléthyle, phénylpropyle, 2-, 3- ou 4-pyridyle, 2-, 4- ou 5-imidazolyle, (2-, 3- ou 4-pyridyl)méthyle, (2-, 4- ou 5-imidazolyl)méthyle, les radicaux alkyle et cycloalkyle cités pouvant être chacun 1 ou 2 fois substitués par un ou des atomes d'halogène ou groupes amino, mono- ou dialkyl($C_1$-$C_4$)-amino, carboxy, guanidino, alcoxy($C_1$-$C_4$)-carbonyle ou par un radical $-CONR^5R^6$, dans lequel $R^5$ et $R^6$ sont

tels que définis plus haut; les radicaux phényle cités pouvant être 1 ou 2 fois substitués par un ou des atomes de fluor ou de chlore ou groupes alkyle en $C_1$-$C_4$, méthoxy, hydroxy, amino, aminométhyle ou trifluorométhyle, et les radicaux hétéroaromatiques cités pouvant être substitués chacun par 1 ou 2 substituants choisis parmi les atomes de fluor, chlore et brome ou des groupes alkyle en $C_1$-$C_4$, méthoxy ou trifluorométhyle,

**3.** Procédé selon la revendication 1 ou 2, caractérisé en ce que

$R^1$ représente le radical méthyle, éthyle, isopropyle, tert-butyle, isobutyle, 2-hydroxyéthyle, 2-méthoxyéthyle, carboxyméthyle, 2-carboxyéthyle, méthoxycarbonylméthyle, 2-méthoxycarbonyléthyle, éthoxycarbonylméthyle, 2-éthoxycarbonyléthyle, carbamoylméthyle, 2-carbamoyléthyle, 2-aminoéthyle, 2-diméthylaminoéthyle, 2-morpholinoéthyle, aminopropyle, aminoisobutyle, méthylaminoisobutyle, diméthylaminoisobutyle, 2-pipéridinoéthyle, cyclopropyle, cyclobutyle, cyclopentyle ou cyclohexyle, phényle, 1- ou 2-naphtyle, o-, m- ou p-méthylphényle, o-, m- ou p-hydroxyphényle ou o-, m- ou p-aminophényle, benzyle, 2-phényléthyle ou $\alpha$- ou $\beta$-naphtylméthyle, un radical hétéroaryle substitué ou non substitué, par exemple 2- ou 3-pyrrolyle, 2-furyle, 2-thiényle, 2- ou 4-imidazolyle, 1-méthyl-2-, -4- ou -5-imidazolyle, 1,3-thiazol-2-yle, 2-, 3- ou 4-pyridyle, 1-oxydo-2-, -3- ou -4-pyridinio, 2-pyrazinyle, 2-, 4- ou 5-pyrimidinyle, 2-, 3- ou 4-quinolyle, 1-, 3- ou 4-isoquinolyle ou 2-benzoxazolyle, méthoxy, éthoxy ou n-butoxy, ou un radical $-NR^5R^6$, dans lequel

$R^5$ et $R^6$ sont identiques ou différents et représentent, indépendamment l'un de l'autre, un atome d'hydrogène ou le groupe méthyle, éthyle, propyle, butyle, isobutyle, sec-butyle, tert-butyle, hydroxyéthyle, méthoxyéthyle, aminoéthyle, aminopropyle, benzyle ou pyridylméthyle, ou dans lequel

$R^5$ et $R^6$ forment, conjointement avec l'atome d'azote qui les porte, un cycle pyrrolidine, pipéridine, morpholine ou pipérazine;

A représente S, SO, $SO_2$, O, CO ou CS;

D représente le groupe CH, le groupe NH ou le groupe $N(CH_3)$;

$R^3$ représente un radical aryle ou arylméthyle, le fragment aryle étant les radicaux phényle, 2-thiényl, 2-pyridyle, 1-naphtyle qui sont chacun éventuellement substitués par un atome de fluor ou de chlore ou un groupe hydroxy, dihydroxy, méthoxy, diméthoxy ou méthylènedioxy;

B représente un reste, lié à l'extrémité N-terminale par -NH- à $R^1$-A-D-$CHR^3$-CO- et, à l'extrémité C-terminale, par -CO- à

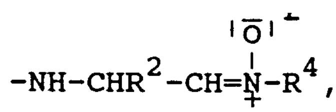

d'un aminoacide de la série phénylalanine, histidine, leucine, $\beta$-2-thiénylalanine, $\beta$-3-thiénylalanine, lysine, norvaline, 2-fluorophénylalanine, 3-fluorophénylalanine, 4-fluorophénylalanine, norleucine, S-méthylcystéine; (1,3-dioxolanne-2-yl)alanine, (1-, 3- ou 4-pyrazolyl)alanine, 4-thiazolylalanine, (2-, 4- ou 5-pyrimidyl)alanine;

$R^2$ représente le radical isobutyle, cyclohexylméthyle, benzyle ou (1,3-dithiolanne-2-yl)-méthyle; et

$R^4$ représente un radical méthyle, éthyle, propyle, isopropyle, butyle, isobutyle, sec-butyle, tert-butyle, pentyle, isopentyle, néopentyle, sec-pentyle, tert-pentyle, hexyle, cyclopentyle, cyclohexyle, cyclopentylméthyle, cyclohexylméthyle, phényle, benzyle, phényléthyle, 2-, 3- ou 4-pyridyle, 2-imidazolyle, (2-, 3- ou 4-pyridyl)méthyle, 2-imidazolylméthyle, les radicaux alkyle et cycloalkyle cités pouvant être chacun 1 ou 2 fois substitués par un ou des atomes de fluor ou de chlore ou groupes amino, méthylamino, éthylamino, diméthylamino, diéthylamino, carboxy, guanidino, méthoxycarbonyle, éthoxycarbonyle, tert-butyloxycarbonyle ou par un radical $-CONR^5R^6$, dans lequel $R^5$ et $R^6$ sont tels que définis plus haut, les radicaux phényle cités pouvant être 1 ou 2 fois substitués par un ou des atomes de fluor ou de chlore ou groupes méthyle, méthoxy, hydroxy, amino, aminométhyle ou trifluorométhyle, et les radicaux hétéroaromatiques cités pouvant être chacun substitué par 1 ou 2 atomes de fluor ou de chlore ou

radicaux méthyle, méthoxy ou trifluorométhyle.

4. Utilisation d'un composé selon l'une des revendications 1 à 3, en tant que médicament.

5. Utilisation d'un composé selon l'une des revendications 1 à 3, en tant que médicament dans le traitement de l'hypertension artérielle.

6. Utilisation d'un composé selon l'une des revendications 1 à 3, en tant que médicament dans le traitement de maladies virales.

7. Procédé pour la préparation d'une composition pharmaceutique contenant un composé de formule I, préparé selon l'un des procédés des revendications 1 à 3, caractérisé en ce que, conjointement avec un véhicule physiologiquement acceptable et éventuellement d'autres adjuvants et additifs, on le met sous une forme pharmaceutique appropriée.